# EUROPEAN PATENT APPLICATION

(11) **EP 4 506 455 A1**
(43) Date of publication of application: **12.02.2025**
(21) Application number: 23781071.8
(22) Date of filing: 31.03.2023
(51) Int. Cl.: C12N 15/09, A61K 35/12, A61K 48/00, C12N 5/10

(54) **CELL SUITABLE FOR GENE ENGINEERING, CELL ENGINEERING AND CELLULAR MEDICINE, AND METHOD FOR PRODUCING SAME**

(30) Priority: 01.04.2022 JP 2022061625; 27.01.2023 JP 2023011278
(71) Applicant: Logomix, Inc., Tokyo 104-0053 (JP)
(72) Inventor: AIZAWA, Yasunori, Tokyo 104-0053 (JP); OHNO, Tomoyuki, Tokyo 104-0053 (JP)
(74) Representative: Hoffmann Eitle
(86) International application number: PCT/JP2023/013574
(87) International publication number: WO 2023/191063

(57) **Abstract**

The present invention provides a cell suitable for gene engineering, cell engineering and cellular medicine, and a method for producing the same. The present invention provides a technique for removing repetitive sequences present in a specific gene region in two or more alleles, thereby facilitating gene targeting or sequencing in the region.

## Description

### Technical Field

The present invention relates to a cell suitable for gene engineering, cell engineering and cellular medicine, and a method for producing the same.

### Background Art

Since a CRISPR/Cas system was reported as a novel genome editing tool, various studies have been conducted using the CRISPR/Cas system (e.g., Patent Literature 1). In the genome editing using the CRISPR/Cas system, a target region targeted by guide RNA undergoes double-strand break by Cas9 nuclease. It is known that the DNA that thus has undergone double-strand break is repaired by homologous directed repair (HDR) or non-homologous end-joining repair (NHEJ). In HDR, an arbitrary sequence can be integrated into the target region by introducing a donor DNA having a sequence homologous to a neighboring region of the target region, together with the CRISPR/Cas system, to cells.

Using genome engineering techniques, a technique for efficiently engineering two or more alleles at the same time by HDR has been developed (e.g., Patent Literature 2). Patent Literature 2 discloses that large-scale deletions of several hundred kb could be efficiently induced at the same time with two or more alleles.

To address the problem of rejection of transplanted cells by a recipient due to differences in the major histocompatibility complex, techniques for producing cells deficient in MHC class I expression and function have been developed (e.g., Patent Literature 3). Patent Literature 3 proposes knocking out β2 microglobulin (B2M) to delete the MHC class I expression and function.

### Citation List

### Patent Literature

Patent Literature 1: WO 2014/093661
Patent Literature 2: WO 2021/206054
Patent Literature 3: WO 2012/145384

### Summary of Invention

MHC-deficient cells are being developed as an effective means of addressing the problem of immune rejection, and the techniques are roughly divided into the following two procedures: loss of cell surface expression of MHC by destruction of non-MHC molecules; and destruction of any of MHC molecules. However, the MHC locus is extremely rich in repetitive sequences, making it very difficult to decipher the locus with current sequencing technologies. Even if a specific part of the locus has been engineered, it is difficult to analyze whether other parts of the repetitive sequence have been maintained or have also been engineered. Hence, techniques of targeting a specific gene at the MHC locus cannot exclude the possibility that the genome has been unpredictably engineered and are difficult to employ in the production of cells used, for example, in regenerative medicine. In the procedure for causing loss of cell surface expression of MHC by destruction of non-MHC molecules, by contrast, the function of the molecules to be destroyed (e.g., β2 microglobulin) is unknown, and the risk of unpredictable problems due to destruction cannot be ruled out at present.

Accordingly, the present invention provides a cell in which a large region containing repetitive sequences that are difficult to decipher is deleted from a specific region of MHC or replaced with a region that can be deciphered, and at the same time, specific genes of MHC are deleted. The present invention also provides a method for producing the cell using a genome engineering method which can efficiently engineer two or more alleles and is capable of engineering a relatively large region. The resulting cells are suitable for transplantation applications or for further cell engineering applications.

As one example, the present invention includes the following aspects.
[1A] A cell (particularly, a human cell) comprising genomic DNA (particularly, human genomic DNA) with a deletion, wherein the deletion is of a region comprising a portion or the whole (preferably, of a region comprising the whole) of an HLA-similar sequence-cluster region located in each of one or more (preferably all) chromosomal regions selected from the group consisting of chromosomal regions defined in the following (i) to (iv):
   (i) a series of regions comprising HLA-F, HLA-G, and HLA-A (e.g., a chromosomal region corresponding to chr6:29,723,464-29,945,455, preferably chr6:29,723,464-30,010,618, for example, chr6:29,722,775-29,945,870 or chr6:29,711,000-30,020,000 chromosomal region of the hg38 genomic sequence),
   (ii) a series of regions comprising HLA-C and HLA-B (e.g., a chromosomal region corresponding to chr6:31,269,169-31,357,158 of the hg38 genomic sequence, preferably chr6:31,269,169-31,463,338, for example, chr6:31,268,749-31,357,179 or chr6:31,176,000-31,534,000 of the hg38 genomic sequence),
   (iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence), and
   (iv) a series of regions comprising HLA-DOA, HLA-DPA1, and HLA-DPB1 (e.g., a chromosomal region corresponding to chr6:33,006,838-33,086,238, preferably chr6:33,006,838-33,131,199, for example, chr6:32,934,636-33,089,696 or chr6:33,002,000-33,147,000 chromosomal region of the hg38 genomic sequence),
   wherein
   the HLA-similar sequence-cluster region comprises all of HLA-encoding genes and HLA-similar sequences contained in each of the chromosomal regions, and
   the portion comprises at least a stretch of region, the stretch of region comprising 50% or more of genes selected from the group consisting of the HLA-encoding genes and HLA-similar sequences contained in each of the chromosomal regions.
[2A] The cell according to [1A], wherein any or preferably all of (i) to (iv) with the deletion defined in claim 1 in the genomic DNA are free of or only one among endogenous HLA and HLA-similar sequences.
[3A] The cell according to [1A] or [2A], wherein any or preferably all of (i) to (iv) with the deletion defined in claim 1 in the genomic DNA are free of endogenous HLA and HLA-similar sequences.
[4A] The cell according to any of [1A] to [3A], wherein the cell comprises
   genomic DNA lacking the whole of the HLA-similar sequence-cluster region in (i) a series of regions comprising HLA-F, HLA-G, and HLA-A (e.g., a chromosomal region corresponding to chr6:29,723,464-29,945,455, preferably chr6:29,723,464-30,010,618, for example, chr6:29,722,775-29,945,870 or chr6:29,711,000-30,020,000 of the hg38 genomic sequence).
[5A] The cell according to any of [1A] to [4A], wherein the cell comprises
   genomic DNA lacking the whole of the HLA-similar sequence-cluster region in (ii) a series of regions comprising HLA-C and HLA-B (e.g., a chromosomal region corresponding to chr6:31,269,169-31,357,158 of the hg38 genomic sequence, preferably chr6:31,269,169-31,463,338, for example, chr6:31,268,749-31,357,179 or chr6:31,176,000-31,534,000 of the hg38 genomic sequence).
[6A] The cell according to any of [1A] to [5A], wherein the cell comprises
   genomic DNA lacking the whole of the HLA-similar sequence-cluster region in (iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence) or a chromosomal region corresponding to chr6:32,445,000-32,821,000.
[7A] The cell according to any of [1A] to [6A], wherein the cell comprises
   genomic DNA lacking the whole of the HLA-similar sequence-cluster region in (iv) a series of regions comprising HLA-DOA, HLA-DPA1, and HLA-DPB1 (e.g., a chromosomal region corresponding to chr6:33,006,838-33,086,238, preferably chr6:33,006,838-33,131,199, for example, chr6:32,934,636-33,089,696 or chr6:33,002,000-33,147,000 chromosomal region of the hg38 genomic sequence).
[8A] The cell according to any of [1A] to [7A], wherein the deletion is of a region having one end in a specific sequence (first sequence) in a chromosomal region corresponding to chr6:29,701,000-29,723,464 (e.g., Chr6:29,709,000-29,711,000) of the hg38 genomic sequence and the other end in a specific sequence (second sequence) in a chromosomal region corresponding to chr6:29,945,455-30,030,000 (e.g., Chr6:30,020,000-30,020,000 and chr6:30,021,500-30,022,800) of the hg38 genomic sequence.
[9A] The cell according to any of [1A] to [8A], wherein the genomic DNA comprises an insertion of an endogenous or exogenous desired gene operably linked to a control sequence, the insertion being located in any of the regions (i) to (iv) or in a region other than the regions (i) to (iv).
[10A] The cell according to any of [1A] to [9A], wherein the deletion is of a region having one end in a specific sequence (third sequence) in a chromosomal region corresponding to chr6:31,166,000-31,269,169 (e.g., Chr6:31,174,000-31,177,000) of the hg38 genomic sequence and the other end in a specific sequence (fourth sequence) in a chromosomal region corresponding to chr6:31,357,158-31,544,000 (e.g., Chr6:31,534,000-31,538,000) of the hg38 genomic sequence.
[11A] The cell according to any of [1A] to [10A], wherein the deletion is of a region having one end in a specific sequence (fifth sequence) in a chromosomal region corresponding to chr6:32,416,000-33,445,000(e.g., Chr6:32,440,000-32,448,500) of the hg38 genomic sequence and the other end in a specific sequence (sixth sequence) in a chromosomal region corresponding to chr6:32,439,951-32,831,000 (e.g., Chr6:32,820,500-32,822,500) of the hg38 genomic sequence.
[12A] The cell according to any of [1A] to [11A], wherein the deletion is of a region having one end in a specific sequence (seventh sequence) in a chromosomal region corresponding to chr6:32,924,000-33,006,838 (e.g., Chr6:32,999,500-33,002,500) of the hg38 genomic sequence and the other end in a specific sequence (eighth sequence) in a chromosomal region corresponding to chr6:33,086,238-33,165,000 (e.g., Chr6:33,147,500-33,150,500) of the hg38 genomic sequence.
[13A] The cell according to any of [1A] to [12A], wherein the regions (i) to (iv) with a deletion have no repetitive sequence within the chromosomal region.
[14A] The cell according to any of [1A] to [13A], wherein the cell comprises one or both of functional β2 microglobulin and functional CIITA.
[15A] The cell according to any of [1A] to [14A], wherein the cell does not substantially express HLA class I and/or HLA class II on a cell surface.
[16A] The cell according to any of [1A] to [15A], wherein the deletion is between 100 kb and 400 kb in size.
[17A] A composition comprising the cell according to any of [1A] to [16A].
[18A] A cell having a genome with a deletion of a region (e.g., a deletion of a region up to 1 Mb or up to 500 kb) in all alleles (two alleles in the case of a diploid cell) of the genome, wherein the deletion comprises a region comprising a portion, or preferably the whole, of an MHC-similar sequence-cluster region of a locus encoding an MHC molecule.
[19A] The cell according to [18A], wherein each MHC-similar sequence-cluster region comprises only a gene encoding no more than one MHC molecule.
[20A] The cell according to [18A], wherein each MHC-similar sequence-cluster region comprises only a gene encoding no more than four MHC molecules.
[21A] The cell according to [18A], wherein each MHC-similar sequence-cluster region does not comprise a gene encoding an MHC molecule.
[22A] The cell according to [18A], wherein the deletion comprises a region comprising all of the MHC-similar sequence-cluster region of a locus encoding an MHC molecule.
[23A] The cell according to [22A], further comprising a control sequence and a desired gene operably linked to the control sequence.
[24A] The cell according to [23A], wherein the control sequence and the desired gene operably linked to the control sequence have a non-naturally occurring sequence as a whole.
[25A] The cell according to [23A], wherein the control sequence and the desired gene operably linked to the control sequence have a naturally occurring (or endogenous) sequence.
[1B] A cell comprising genomic DNA with a deletion, wherein the deletion is of a region comprising a portion or the whole (preferably, of a region comprising the whole) of an HLA-similar sequence-cluster region located in each of one or more (preferably all) chromosomal regions selected from the group consisting of chromosomal regions defined in the following (i) to (iv):
   (i) a series of regions comprising HLA-F, HLA-G, and HLA-A (e.g., a chromosomal region corresponding to chr6:29,723,464-29,945,455, preferably chr6:29,723,464-30,010,618, for example, chr6:29,722,775-29,945,870 or chr6:29,711,000-30,020,000 chromosomal region of the hg38 genomic sequence),
   (ii) a series of regions comprising HLA-C and HLA-B (e.g., a chromosomal region corresponding to chr6:31,269,169-31,357,158 of the hg38 genomic sequence, preferably chr6:31,269,169-31,463,338, for example, chr6:31,268,749-31,357,179 or chr6:31,176,000-31,534,000 of the hg38 genomic sequence),
   (iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence), and
   (iv) a series of regions comprising HLA-DOA, HLA-DPA1, and HLA-DPB1 (e.g., a chromosomal region corresponding to chr6:33,006,838-33,086,238, preferably chr6:33,006,838-33,131,199, for example, chr6:32,934,636-33,089,696 or chr6:33,002,000-33,147,000 chromosomal region of the hg38 genomic sequence),
   wherein
   the HLA-similar sequence-cluster region comprises all of HLA-encoding genes and HLA-similar sequences contained in each of the chromosomal regions, and
   the portion comprises at least a stretch of region, the stretch of region comprising 50% or more of genes selected from the group consisting of the HLA-encoding genes and HLA-similar sequences contained in each of the chromosomal regions.
[2B] The cell according to [1B], wherein any or all of (i) to (iv) with the deletion defined in claim 1 in the genomic DNA are free of or only one of endogenous HLA and HLA-similar sequences.
[3B] The cell according to [1B] or [2B], wherein any or all of (i) to (iv) with the deletion defined in claim 1 in the genomic DNA are free of endogenous HLA and HLA-similar sequences.
[4B] The cell according to any of [1B] to [3B], wherein the cell comprises
   genomic DNA lacking the whole of the HLA-similar sequence-cluster region in (i) a series of regions comprising HLA-F, HLA-G, and HLA-A (e.g., a chromosomal region corresponding to chr6:29,723,464-29,945,455, preferably chr6:29,723,464-30,010,618, for example, chr6:29,722,775-29,945,870 or chr6:29,711,000-30,020,000 of the hg38 genomic sequence).
[5B] The cell according to any of [1B] to [4B], wherein the cell comprises
   genomic DNA lacking the whole of the HLA-similar sequence-cluster region in (ii) a series of regions comprising HLA-C and HLA-B (e.g., a chromosomal region corresponding to chr6:31,269,169-31,357,158 of the hg38 genomic sequence, preferably chr6:31,269,169-31,463,338, for example, chr6:31,268,749-31,357,179 or chr6:31,176,000-31,534,000 of the hg38 genomic sequence).
[6B] The cell according to any of [1B] to [5B], wherein the cell comprises
   genomic DNA lacking the whole of the HLA-similar sequence-cluster region in (iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence) or a chromosomal region corresponding to chr6:32,445,000-32,821,000.
[7B] The cell according to any of [1B] to [6B], wherein the cell comprises
   genomic DNA lacking the whole of the HLA-similar sequence-cluster region in (iv) a series of regions comprising HLA-DOA, HLA-DPA1, and HLA-DPB1 (e.g., a chromosomal region corresponding to chr6:33,006,838-33,086,238, preferably chr6:33,006,838-33,131,199, for example, chr6:32,934,636-33,089,696 or chr6:33,002,000-33,147,000 chromosomal region of the hg38 genomic sequence).
[8B] The cell according to any of [1B] to [7B], wherein the deletion is of a region having one end in a specific sequence (first sequence) in a chromosomal region corresponding to chr6:29,701,000-29,723,464 (e.g., Chr6:29,709,000-29,711,000) of the hg38 genomic sequence and the other end in a specific sequence (second sequence) in a chromosomal region corresponding to chr6:29,945,455-30,030,000 (e.g., Chr6:30,020,000-30,020,000 and chr6:30,021,500-30,022,800) of the hg38 genomic sequence.
[9B] The cell according to any of [1B] to [8B], wherein the genomic DNA comprises a control sequence and an insertion of an endogenous or exogenous desired gene operably linked to the control sequence, the insertion being located in any of the regions (i) to (iv) or in a region other than the regions (i) to (iv).
[10B] The cell according to any of [1B] to [9B], wherein the deletion is of a region having one end in a specific sequence (third sequence) in a chromosomal region corresponding to chr6:31,166,000-31,269,169 (e.g., Chr6:31,174,000-31,177,000) of the hg38 genomic sequence and the other end in a specific sequence (fourth sequence) in a chromosomal region corresponding to chr6:31,357,158-31,544,000 (e.g., Chr6:31,534,000-31,538,000) of the hg38 genomic sequence.
[11B] The cell according to any of [1B] to [10B], wherein the deletion is of a region having one end in a specific sequence (fifth sequence) in a chromosomal region corresponding to chr6:32,416,000-33,445,000(e.g., Chr6:32,440,000-32,448,500) of the hg38 genomic sequence and the other end in a specific sequence (sixth sequence) in a chromosomal region corresponding to chr6:32,439,951-32,831,000 (e.g., Chr6:32,820,500-32,822,500) of the hg38 genomic sequence.
[12B] The cell according to any of [1B] to [11B], wherein the deletion is of a region having one end in a specific sequence (seventh sequence) in a chromosomal region corresponding to chr6: 32,924,000 to 33,006,838 (e.g., Chr6:32,999,500-33,002,500) of the hg38 genomic sequence and the other end in a specific sequence (eighth sequence) in a chromosomal region corresponding to chr6:33,086,238-33,165,000 (e.g., Chr6:33,147,500-33,150,500) of the hg38 genomic sequence.
[13B] The cell according to any one of [1B] to [12B], wherein the regions (i) to (iv) with a deletion has no repetitive sequence within the chromosomal region.
[14B] The cell according to any of [1B] to [13B], comprising one or both of functional β2 microglobulin and functional CIITA.
[15B] The cell according to any of [1B] to [14B], wherein the cell does not substantially express HLA class I and/or HLA class II on a cell surface.
[16B] The cell according to any of [1B] to [15B], wherein the deletion is between 100 kb and 400 kb in size.
[17B] A composition comprising the cell according to any of [1B] to [16B].
[1C] A cell comprising genomic DNA with a deletion, wherein the deletion is of a region comprising the whole of an HLA-similar sequence-cluster region located in each of one or more (preferably all) chromosomal regions selected from the group consisting of chromosomal regions defined in the following (i) to (iv):
   (i) a series of regions comprising HLA-F, HLA-G, and HLA-A (e.g., a chromosomal region corresponding to chr6:29,723,464-29,945,455, preferably chr6:29,723,464-30,010,618, for example, chr6:29,722,775-29,945,870 or chr6:29,711,000-30,020,000 chromosomal region of the hg38 genomic sequence),
   (ii) a series of regions comprising HLA-C and HLA-B (e.g., a chromosomal region corresponding to chr6:31,269,169-31,357,158 of the hg38 genomic sequence, preferably chr6:31,269,169-31,463,338, for example, chr6:31,268,749-31,357,179 or chr6:31,176,000-31,534,000 of the hg38 genomic sequence),
   (iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence), and
   (iv) a series of regions comprising HLA-DOA, HLA-DPA1, and HLA-DPB1 (e.g., a chromosomal region corresponding to chr6:33,006,838-33,086,238, preferably chr6:33,006,838-33,131,199, for example, chr6:32,934,636-33,089,696 or chr6:33,002,000-33,147,000 chromosomal region of the hg38 genomic sequence),
   wherein
   the HLA-similar sequence-cluster region comprises all of HLA-encoding genes and HLA-similar sequences contained in each of the chromosomal regions,
   the genomic DNA may further comprise a deletion of a gene encoding HLA-E, and
   any of the regions with a deletion (e.g., a region with a deletion of the gene encoding HLA-E) has a first allele and a second allele; each of the first allele and the second allele has at the deletion site a cassette (referred to as a first cassette and a second cassette, respectively) containing a nucleotide sequence of one or more selective marker genes including a selective marker gene for negative selection (and preferably a marker gene for positive selection); and the selective marker gene for negative selection contained in the first cassette and one or more selective marker genes for negative selection contained in the second cassette are distinctively different from each other, whereby a cell having the engineered alleles in the first cassette or the second cassette can be selected by using non-expression of the selective marker gene for negative selection contained in the first cassette or the second cassette as an index after the first cassette or the second cassette is further engineered through removal or replacement with another sequence.
[2C] A cell comprising genomic DNA with a deletion, wherein the deletion is of a region comprising the whole of an HLA-similar sequence-cluster region located in each of one or more (preferably all) chromosomal regions selected from the group consisting of chromosomal regions defined in the following (i) to (iv):
   (i) a series of regions comprising HLA-F, HLA-G, and HLA-A (e.g., a chromosomal region corresponding to chr6:29,723,464-29,945,455, preferably chr6:29,723,464-30,010,618, for example, chr6:29,722,775-29,945,870 or chr6:29,711,000-30,020,000 chromosomal region of the hg38 genomic sequence),
   (ii) a series of regions comprising HLA-C and HLA-B (e.g., a chromosomal region corresponding to chr6:31,269,169-31,357,158 of the hg38 genomic sequence, preferably chr6:31,269,169-31,463,338, for example, chr6:31,268,749-31,357,179 or chr6:31,176,000-31,534,000 of the hg38 genomic sequence),
   (iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence), and
   (iv) a series of regions comprising HLA-DOA, HLA-DPA1, and HLA-DPB1 (e.g., a chromosomal region corresponding to chr6:33,006,838-33,086,238, preferably chr6:33,006,838-33,131,199, for example, chr6:32,934,636-33,089,696 or chr6:33,002,000-33,147,000 chromosomal region of the hg38 genomic sequence),
   wherein
   the HLA-similar sequence-cluster region comprises all of HLA-encoding genes and HLA-similar sequences contained in each of the chromosomal regions,
   the genomic DNA further comprises a deletion of a gene encoding HLA-E, and
   any of the regions with a deletion (e.g., a region with a deletion of the gene encoding HLA-E) has a first allele and a second allele; the first allele has a foreign gene of interest; and the second allele has at the deletion site a cassette (referred to as a second cassette) containing a nucleotide sequence of one or more selective marker genes including a selective marker gene for negative selection (and preferably a marker gene for positive selection), whereby a cell having the engineered alleles in the second cassette can be selected by using non-expression of the selective marker gene for negative selection contained in the second cassette as an index after the second cassette is further engineered through removal or replacement with another sequence.
[3C] A cell comprising genomic DNA with a deletion, wherein the deletion is of a region comprising the whole of an HLA-similar sequence-cluster region located in each of one or more (preferably all) chromosomal regions selected from the group consisting of chromosomal regions defined in the following (i) to (iv):
   (i) a series of regions comprising HLA-F, HLA-G, and HLA-A (e.g., a chromosomal region corresponding to chr6:29,723,464-29,945,455, preferably chr6:29,723,464-30,010,618, for example, chr6:29,722,775-29,945,870 or chr6:29,711,000-30,020,000 chromosomal region of the hg38 genomic sequence),
   (ii) a series of regions comprising HLA-C and HLA-B (e.g., a chromosomal region corresponding to chr6:31,269,169-31,357,158 of the hg38 genomic sequence, preferably chr6:31,269,169-31,463,338, for example, chr6:31,268,749-31,357,179 or chr6: 31,176,000 to 31,534,000 of the hg38 genomic sequence),
   (iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence), and
   (iv) a series of regions comprising HLA-DOA, HLA-DPA1, and HLA-DPB1 (e.g., a chromosomal region corresponding to chr6:33,006,838-33,086,238, preferably chr6: 33,006,838-33,131,199, for example, chr6:32,934,636-33,089,696 or chr6:33,002,000-33,147,000 chromosomal region of the hg38 genomic sequence),
   wherein
   the HLA-similar sequence-cluster region comprises all of HLA-encoding genes and HLA-similar sequences contained in each of the chromosomal regions;
   the genomic DNA further comprises a deletion of a gene encoding HLA-E;
   any of the regions with a deletion (e.g., a region with a deletion of the gene encoding HLA-E) has a first allele and a second allele, and
      (1) each of the first allele and the second allele has a foreign gene of interest at the deletion site, or
      (2) one of the first allele and the second allele has a foreign gene of interest at the deletion site, and the other has a structure in which both ends of the deletion site are linked directly or indirectly (e.g., indirectly via a spacer, etc.) to each other.
[4C] The cell according to [2C], wherein the first allele comprises a foreign gene encoding any one, two, three, or all selected from the group consisting of HLA-A, HLA-B, HLA-C, and HLA-E.
[5C] The cells according to [3C], wherein the foreign gene of interest comprises a foreign gene encoding any one, two, three, or all selected from the group consisting of HLA-A, HLA-B, HLA-C, and HLA-E, and each of foreign genes of interest is included in the first allele or the second allele.
[6C] The cell according to [2C] or [4C], wherein the foreign gene further comprises a sequence upstream of a transcription start point of a coding region of the gene, an intron, and a sequence downstream of a stop codon.
[7C] The cell according to [3C] or [5C], wherein the foreign gene further comprises a sequence upstream of a transcription start point of a coding region of the gene, an intron, and a sequence downstream of a stop codon.
[8C] The cell according to [6C], wherein the upstream sequence has a length of 1 kbp or more.
[9C] The cell according to [7C], wherein the upstream sequence has a length of 1 kbp or more.
[10C] A composition comprising the cell according to any of [1C] to [9C].
[11C] A composition for use in analyzing expression and/or function of the foreign gene, comprising the cell according to any of [2C] to [9C].

As one example, the present invention includes the following aspects.
[1] A genome engineering method for engineering two or more alleles in the chromosomal genome at the MHC locus (particularly, a method for engineering two or more alleles in the chromosomal genome to delete a specific gene group), comprising the steps of:
   (a) introducing the following (i) and (ii) to a cell comprising the chromosome:
      (i) a genome engineering system comprising a sequence-specific nucleic acid cleaving molecule targeting a target region in the chromosomal genome, or a polynucleotide encoding the sequence-specific nucleic acid cleaving molecule, and
      (ii) two or more donor DNAs for selective markers, each of which comprises a nucleotide sequence of a selective marker gene between an upstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering; and
   (b) after the step (a), selecting the cell on the basis of all the selective marker genes carried by the two or more donor DNAs for selective markers.
[2] The genome engineering method according to [1], wherein the selective marker gene is a positive selective marker gene, and the step (b) is the step of selecting a cell expressing the same number of the positive selective marker gene as the number of the alleles.
[3] The genome engineering method according to [2], wherein each of the donor DNAs for selective markers further has a negative selective marker gene between the upstream homology arm and the downstream homology arm.
[4] The genome engineering method according to [3], further comprising the steps of: (c) after the step (b), introducing, to the cell, a donor DNA for recombination comprising a desired nucleotide sequence between an upstream homology arm having a nucleotide sequence homologous to an upstream nucleotide sequence adjacent to the target region and a downstream homology arm having a nucleotide sequence homologous to a downstream nucleotide sequence adjacent to the target region; and (d) after the step (c), selecting a cell not expressing the negative selective marker gene.
[5] The genome engineering method according to [3] or [4], wherein the positive selective marker gene is a drug resistance gene, and the negative selective marker gene is a fluorescent protein gene.
[6] The genome engineering method according to any one of [1] to [5], wherein the sequence-specific nucleic acid cleaving molecule is sequence-specific endonuclease.
[7] The genome engineering method according to [6], wherein the genome engineering system comprises Cas protein, and guide RNA having a nucleotide sequence homologous to a nucleotide sequence within the target region.
[8] A genome engineering kit for engineering two or more alleles in the chromosomal genome, comprising the following (i) and (ii):
   (i) a genome engineering system comprising a sequence-specific nucleic acid cleaving molecule targeting a target region in the chromosomal genome, or a polynucleotide encoding the sequence-specific nucleic acid cleaving molecule, and
   (ii) two or more donor DNAs for selective markers, each of which comprises a nucleotide sequence of a selective marker gene between an upstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering.
[9] The genome engineering kit according to [8], wherein the selective marker gene is a positive selective marker gene.
[10] The genome engineering kit according to [9], wherein each of the donor DNAs for selective markers further has a negative selective marker gene between the upstream homology arm and the downstream homology arm.
[11] The genome engineering kit according to any one of [8] to [10], wherein the sequence-specific nucleic acid cleaving molecule is sequence-specific endonuclease.
[12] The genome engineering kit according to any one of [8] to [11], wherein the genome engineering system comprises Cas protein, and guide RNA having nucleotide sequence homologous to a nucleotide sequence within the target region.

As one example, the present invention includes the following aspects.
[1] A method for preparing a cell in which two or more alleles in the chromosomal genome are engineered, comprising the steps of:
   (a) introducing the following (i) and (ii) to a cell comprising two or more alleles to introduce a selective marker gene to each of the two or more alleles:
      (i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
      (ii) two or more donor DNAs for selective markers, each of which has an upstream homology arm having a nucleotide sequence homologously recombinable with an upstream nucleotide sequence of the target region and a downstream homology arm having a nucleotide sequence homologously recombinable with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and
   (b) after the step (a), respectively homologously recombining the two or more alleles with different types of donor DNAs for selective markers so that the distinguishably different unique selective marker genes are respectively introduced in the two or more alleles, and selecting a cell expressing all the distinguishably different selective marker genes thus introduced (step for positive selection).
[2] A method for engineering two or more alleles in the chromosomal genome, comprising the steps of:
   (a) introducing the following (i) and (ii) to a cell comprising two or more alleles to introduce a selective marker gene to each of the two or more alleles:
      (i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
      (ii) two or more donor DNAs for selective markers, each of which has an upstream homology arm having a nucleotide sequence homologously recombinable with an upstream nucleotide sequence of the target region and a downstream homology arm having a nucleotide sequence homologously recombinable with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and
   (b) after the step (a), respectively homologously recombining the two or more alleles with different types of donor DNAs for selective markers so that the distinguishably different unique selective marker genes are respectively introduced in the two or more alleles, and selecting a cell expressing all the distinguishably different selective marker genes thus introduced (step for positive selection).
[3] The method according to [1] or [2], wherein the target region has a length of 5 kbp or more.
[4] The method according to [3], wherein the target region has a length of 8 kbp or more.
[5] The method according to any of [1] to [4], wherein
   each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
   the method further comprising the steps of:
      (c) after the step (b), introducing the following (iii) and (iv) to the selected cell to introduce a donor DNA for recombination to each of the two or more alleles:
         (iii) a genome engineering system comprising a sequence-specific nucleic acid cleaving molecule capable of targeting the target sequence and cleaving the target sequence, or a polynucleotide encoding the sequence-specific nucleic acid cleaving molecule, and
         (iv) a donor DNA for recombination comprising a desired nucleotide sequence, the donor DNA for recombination having an upstream homology arm having a nucleotide sequence homologously recombinable with an upstream nucleotide sequence of the target region and a downstream homology arm having a nucleotide sequence homologously recombinable with a downstream nucleotide sequence of the target region; and
      (d) after the step (c), selecting a cell not expressing the marker gene for negative selection (step for negative selection).
[6] The method according to [3], wherein
   each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
   the method further comprising the steps of:
      (c) after the step (b), introducing the following (iii) and (iv) to the selected cell to introduce a donor DNA for recombination to each of the two or more alleles:
         (iii) a genome engineering system comprising a sequence-specific nucleic acid cleaving molecule capable of targeting the target sequence and cleaving the target sequence, or a polynucleotide encoding the sequence-specific nucleic acid cleaving molecule, and
         (iv) a donor DNA for recombination comprising a desired nucleotide sequence, the donor DNA for recombination having an upstream homology arm having a nucleotide sequence homologously recombinable with an upstream nucleotide sequence of the target region and a downstream homology arm having a nucleotide sequence homologously recombinable with a downstream nucleotide sequence of the target region; and
      (d) after the step (c), selecting a cell not expressing the marker gene for negative selection (step for negative selection).
[7] The method according to [4], wherein
   each of the two or more donor DNAs for selective markers has a selective marker gene for positive selection, a marker gene for negative selection, and a target sequence between the upstream homology arm and the downstream homology arm, wherein in the case of using the selective marker gene both for positive selection and for negative selection, another selective marker gene for negative selection is optionally absent,
   the method further comprising the steps of:
      (c) after the step (b), introducing the following (iii) and (iv) to the selected cell to introduce a donor DNA for recombination to each of the two or more alleles:
         (iii) a genome engineering system comprising a sequence-specific nucleic acid cleaving molecule capable of targeting the target sequence and cleaving the target sequence, or a polynucleotide encoding the sequence-specific nucleic acid cleaving molecule, and
         (iv) a donor DNA for recombination comprising a desired nucleotide sequence, the donor DNA for recombination having an upstream homology arm having a nucleotide sequence homologously recombinable with an upstream nucleotide sequence of the target region and a downstream homology arm having a nucleotide sequence homologously recombinable with a downstream nucleotide sequence of the target region; and
      (d) after the step (c), selecting a cell not expressing the marker gene for negative selection (step for negative selection).
[8] The method according to any of [5] to [7], wherein the region between the upstream homology arm and the downstream homology arm of the donor DNA for recombination has a length of 5 kbp or more.
[9] The method according to [6] or [7], wherein the region between the upstream homology arm and the downstream homology arm of the donor DNA for recombination has a length of 5 kbp or more.
[10] The method according to [7], wherein the region between the upstream homology arm and the downstream homology arm of the donor DNA for recombination has a length of 5 kbp or more.
[11] The method according to [8], wherein the region between the upstream homology arm and the downstream homology arm of the donor DNA for recombination has a length of 8 kbp or more.
[12] A genome engineering kit for engineering two or more alleles in the chromosomal genome, comprising the following (i) and (ii):
   (i) a genome engineering system comprising a sequence-specific nucleic acid cleaving molecule capable of targeting a target region in the chromosomal genome and cleaving the target region, or a polynucleotide encoding the sequence-specific nucleic acid cleaving molecule, and
   (ii) two or more donor DNAs for selective markers, each of which has an upstream homology arm having a nucleotide sequence homologously recombinable with an upstream nucleotide sequence of the target region and a downstream homology arm having a nucleotide sequence homologously recombinable with a downstream nucleotide sequence of the target region, and comprises a nucleotide sequence of a selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering.
[13] The kit according to [12], wherein the target region has a length of 5 kbp or more.
[14] The kit according to [13], wherein the target region has a length of 8 kbp or more.
[15] The kit according to any of [12] to [14], further comprising a donor DNA for recombination.
[16] The kit according to any of [12] to [15], wherein a region between an upstream homology arm and a downstream homology arm of the donor DNA for recombination has a length of 5 kbp or more.
[17] The kit according to any of [12] to [16], wherein a region between an upstream homology arm and a downstream homology arm of the donor DNA for recombination has a length of 8 kbp or more.
[18] The kit according to [12], wherein the target region has a length of 5 kbp or more, and a region between an upstream homology arm and a downstream homology arm of the donor DNA for recombination has a length of 5 kbp or more.
[19] The kit according to [18], wherein the target region has a length of 8 kbp or more, and a region between an upstream homology arm and a downstream homology arm of the donor DNA for recombination has a length of 8 kbp or more.
[20] The method according to [5], wherein the donor DNA for recombination has no nucleotide sequence in the region between the upstream homology arm and the downstream homology arm of the donor DNA for recombination, and the upstream and downstream sequences of the target region are seamlessly linked, without insertion, substitution and deletion of a base, in the thus-engineered two or more alleles in the chromosomal genome.
[21] The method according to [6] or [7], wherein the donor DNA for recombination has no nucleotide sequence in the region between the upstream homology arm and the downstream homology arm of the donor DNA for recombination, and the upstream and downstream sequences of the target region are seamlessly linked, without insertion, substitution and deletion of a base, in the thus-engineered two or more alleles in the chromosomal genome.
[22] The method according to [3], wherein a target sequence of site-specific recombinase is absent in the thus-engineered two or more alleles in the chromosomal genome.
[23] The method according to [4], wherein a target sequence of site-specific recombinase is absent in the thus-engineered two or more alleles in the chromosomal genome.
[24] The method according to [5], wherein a target sequence of site-specific recombinase is absent in the thus-engineered two or more alleles in the chromosomal genome.
[25] The method according to [6] or [7], wherein a target sequence of site-specific recombinase is absent in the thus-engineered two or more alleles in the chromosomal genome.
[26] The method according to any of [1] to [11] and [20] to [25], wherein in the step (b), single-cell cloning is not performed in a process up to the selection of the cell in which the two or more alleles are engineered.
[27] A cell having two or more alleles in the chromosomal genome in relation to a target region, wherein the respective target regions of the two or more alleles are deleted, and the upstream and downstream sequences of the target region are seamlessly linked without insertion, substitution and deletion of a base.
[28] The cell according to [27], wherein the target region has a length of 5 kbp or more.
[29] The cell according to [27] or [28], wherein the cell has no target sequence of site-specific recombinase in the genome.
[30] The method according to [6] or [7], wherein the region between the upstream homology arm and the downstream homology arm of the donor DNA for recombination has a length of 8 kbp or more.
[31] The kit according to [15], wherein the donor DNA for recombination has no nucleotide sequence in the region between the upstream homology arm and the downstream homology arm of the donor DNA for recombination.

As one example, the present invention includes the following aspects.
[1D] A cell (particularly, a human cell) comprising genomic DNA (particularly, human genomic DNA) with a deletion, wherein the deletion is of a region comprising a portion or the whole of an HLA-similar sequence-cluster region located in each of one or more chromosomal regions selected from the group consisting of chromosomal regions defined in the following (i) to (iv):
   (i) a chromosomal region corresponding to chr6:29,711,000-30,020,000 chromosomal region of the hg38 genomic sequence,
   (ii) a chromosomal region corresponding to chr6:31,176,000-31,534,000 chromosomal region of the hg38 genomic sequence,
   (iii) a chromosomal region corresponding to chr6:32,445,000-32,821,000 chromosomal region of the hg38 genomic sequence, and
   (iv) a chromosomal region corresponding to chr6:33,002,000-33,147,000 chromosomal region of the hg38 genomic sequence, wherein
      the HLA-similar sequence-cluster region comprises all of HLA-encoding genes and HLA-similar sequences contained in each of the chromosomal regions, and
      the portion comprises at least a stretch of region, the stretch of region comprising 50% or more of genes selected from the group consisting of the HLA-encoding genes and HLA-similar sequences contained in each of the chromosomal regions.
[2D] The cell according to [1D], wherein any or preferably all of (i) to (iv) with the deletion defined in claim 1 in the genomic DNA are free of or only one of endogenous HLA and HLA-similar sequences.
[3D] The cell according to [1D] or [2D], wherein any or preferably all of (i) to (iv) with the deletion defined in claim 1 in the genomic DNA are free of endogenous HLA and HLA-similar sequences.
[4D] The cell according to any of [1D] to [3D], wherein the cell comprises
   genomic DNA lacking the whole of the HLA-similar sequence-cluster region in (i) chr6:29,711,000-30,020,000 chromosomal region.
[5D] The cell according to any of [1D] to [4D], wherein the cell comprises
   genomic DNA lacking the whole of the HLA-similar sequence-cluster region in (ii) chr6:31,176,000-31,534,000 chromosomal region.
[6D] The cell according to any of [1D] to [5D], wherein the cell comprises
   genomic DNA lacking the whole of the HLA-similar sequence-cluster region in (iii) chr6:32,445,000-32,821,000 chromosomal region.
[7D] The cell according to any of [1D] to [6D], wherein the cell comprises
   genomic DNA lacking the whole of the HLA-similar sequence-cluster region in (iv) chr6:33,002,000-33,147,000 chromosomal region.
[8D] The cell according to any of [1D] to [7D], wherein the deletion is of a region having one end in a specific sequence (first sequence) in Chr6:29,709,000-29,711,000 chromosomal region and the other end in a specific sequence (second sequence) in Chr6:30,020,000-30,020,000 chromosomal region and Chr6:30,021,500-30,022,800 chromosomal region.
[9D] The cell according to any of [1D] to [8D], wherein the genomic DNA comprises an insertion of an endogenous or exogenous desired gene operably linked to a control sequence, the insertion being located in any of the regions (i) to (iv) or in a region other than the regions (i) to (iv).
[10D] The cell according to any of [1D] to [9D], wherein the deletion is of a region having one end in a specific sequence (third sequence) in Chr6:31,174,000-31,177,000 chromosomal region and the other end in a specific sequence (fourth sequence) in Chr6:31,534,000-31,538,000 chromosomal region.
[11D] The cell according to any of [1D] to [10D], wherein the deletion is of a region having one end in a specific sequence (fifth sequence) in Chr6:32,446,000-32,448,500 chromosomal region and the other end in a specific sequence (sixth sequence) in Chr6:32,820,500-32,822,500 chromosomal region.
[12D] The cell according to any of [1D] to [11D], wherein the deletion is of a region having one end in a specific sequence (seventh sequence) in Chr6:32,999,500-33,002,500 chromosomal region and the other end in a specific sequence (eighth sequence) in Chr6:33,147,500-33,150,500 chromosomal region.
[13D] The cell according to any of [1D] to [12D], wherein the regions (i) to (iv) with a deletion has no repetitive sequence within the chromosomal region.
[14D] The cell according to any of [1D] to [13D], wherein the cell comprises one or both of functional β2 microglobulin and functional CIITA.
[15D] The cell according to any of [1D] to [14D], wherein the cell does not substantially express HLA class I and/or HLA class II on a cell surface.
[16D] The cell according to any of [1D] to [15D], wherein the deletion is between 100 kb and 400 kb in size.
[17D] A composition comprising the cell according to any of [1D] to [16D].
[18D] A cell having a genome with a deletion of a region (e.g., a deletion of a region up to 1 Mb or up to 500 kb) in all alleles (two alleles in the case of a diploid cell) of the genome, wherein the deletion comprises a region comprising a portion, or preferably the whole, of an MHC-similar sequence-cluster region of a locus encoding an MHC molecule.
[19D] The cell according to [18D], wherein each MHC-similar sequence-cluster region comprises only a gene encoding no more than one MHC molecule.
[20D] The cell according to [18D], wherein each MHC-similar sequence-cluster region comprises only a gene encoding no more than four MHC molecules.
[21D] The cell according to [18D], wherein each MHC-similar sequence-cluster region does not comprise a gene encoding an MHC molecule.
[22D] The cell according to [18D], wherein the deletion comprises a region comprising all of the MHC-similar sequence-cluster region of a locus encoding an MHC molecule.
[23D] The cell according to [22D], further comprising a control sequence and a desired gene operably linked to the control sequence.
[24D] The cell according to [23D], wherein the control sequence and the desired gene operably linked to the control sequence have a non-naturally occurring sequence as a whole.
[25D] The cell according to [23D], wherein the control sequence and the desired gene operably linked to the control sequence have a naturally occurring (or endogenous) sequence.

### Advantageous Effects of Invention

According to the present invention, two or more alleles can be efficiently engineered, and a genome engineering method and a genome engineering kit which are capable of engineering a relatively large region (particularly, a large-scale deletion of the HLA class I region and the HLA class II region), and a cell having the engineered alleles can be provided.

### Brief Description of Drawings

[Figure 1] Figure 1 is a genetic map of the chromosome 6 region in which the HLA class I and HLA class II regions are located. Clusters in which HLA genes are present are highlighted with arrows.
[Figure 2] Figure 2 shows search results for similar sequences of the HLA-A gene in the chromosome 6 region in which the HLA class I and HLA class II regions are located.
[Figure 3] Figure 3 shows search results for similar sequences of the HLA-B gene in the chromosome 6 region in which the HLA class I and HLA class II regions are located.
[Figure 4] Figure 4 shows search results for similar sequences of the HLA-C gene in the chromosome 6 region in which the HLA class I and HLA class II regions are located.
[Figure 5] Figure 5 shows search results for similar sequences of the HLA-E gene in the chromosome 6 region in which the HLA class I and HLA class II regions are located.
[Figure 6] Figure 6 shows search results for similar sequences of the HLA-F gene in the chromosome 6 region in which the HLA class I and HLA class II regions are located.
[Figure 7] Figure 7 shows search results for similar sequences of the HLA-G gene in the chromosome 6 region in which the HLA class I and HLA class II regions are located.
[Figure 8] Figure 8 shows search results for similar sequences of the HLA-H gene in the chromosome 6 region in which the HLA class I and HLA class II regions are located.
[Figure 9] Figure 9 shows search results for similar sequences of the HLA-J gene in the chromosome 6 region in which the HLA class I and HLA class II regions are located.
[Figure 10] Figure 10 shows search results for similar sequences of the HLA-W gene in the chromosome 6 region in which the HLA class I and HLA class II regions are located.
[Figure 11] Figure 11 shows search results for similar sequences of the MICA gene in the chromosome 6 region in which the HLA class I and HLA class II regions are located.
[Figure 12] Figure 12 shows search results for similar sequences of the MICB gene in the chromosome 6 region in which the HLA class I and HLA class II regions are located.
[Figure 13] Figure 13 shows search results for similar sequences of the HLA-DRA gene in the chromosome 6 region in which the HLA class I and HLA class II regions are located.
[Figure 14] Figure 14 shows search results for similar sequences of the HLA-DRB1 gene in the chromosome 6 region in which the HLA class I and HLA class II regions are located.
[Figure 15] Figure 15 shows search results for similar sequences of the HLA-DRB5 gene in the chromosome 6 region in which the HLA class I and HLA class II regions are located.
[Figure 16] Figure 16 shows search results for similar sequences of the HLA-DRB6 gene in the chromosome 6 region in which the HLA class I and HLA class II regions are located.
[Figure 17] Figure 17 shows search results for similar sequences of the HLA-DRB9 gene in the chromosome 6 region in which the HLA class I and HLA class II regions are located.
[Figure 18] Figure 18 shows search results for similar sequences of the HLA-DQA1 gene in the chromosome 6 region in which the HLA class I and HLA class II regions are located.
[Figure 19] Figure 19 shows search results for similar sequences of the HLA-DQA2 gene in the chromosome 6 region in which the HLA class I and HLA class II regions are located.
[Figure 20] Figure 20 shows search results for similar sequences of the HLA-DQB1 gene in the chromosome 6 region in which the HLA class I and HLA class II regions are located.
[Figure 21] Figure 21 shows search results for similar sequences of the HLA-DPA1 gene in the chromosome 6 region in which the HLA class I and HLA class II regions are located.
[Figure 22] Figure 22 shows search results for similar sequences of the HLA-DPB1 gene in the chromosome 6 region in which the HLA class I and HLA class II regions are located.
[Figure 23] Figure 23 shows search results for similar sequences of the HLA-DMB gene in the chromosome 6 region in which the HLA class I and HLA class II regions are located.
[Figure 24] Figure 24 shows search results for similar sequences of the HLA-DOA gene in the chromosome 6 region in which the HLA class I and HLA class II regions are located.
[Figure 25] Figure 25 shows search results for similar sequences of the HLA-DOB gene in the chromosome 6 region in which the HLA class I and HLA class II regions are located.
[Figure 26] Figure 26 shows positions of four HLA-similar sequence (sequence)-cluster regions (i) to (iv) in the HLA class I and HLA class II regions.
[Figure 27] Figure 27 shows an example of Blat search results for identifying terminal regions on the telomere side of the HLA-similar sequence-cluster region (i).
[Figure 28] Figure 28 shows an example of Blat search results for identifying terminal regions on the centromere side of the HLA-similar sequence-cluster region (i).
[Figure 29] Figure 29 shows an example of Blat search results for identifying terminal regions on the telomere side of the HLA-similar sequence-cluster region (ii).
[Figure 30] Figure 30 shows an example of Blat search results for identifying terminal regions on the centromere side of the HLA-similar sequence-cluster region (ii).
[Figure 31] Figure 31 shows an example of Blat search results for identifying terminal regions on the telomere side of the HLA-similar sequence-cluster region (iii).
[Figure 32] Figure 32 shows an example of Blat search results for identifying terminal regions on the centromere side of the HLA-similar sequence-cluster region (iii).
[Figure 33] Figure 33 shows an example of Blat search results for identifying terminal regions on the telomere side of the HLA-similar sequence-cluster region (iv).
[Figure 34] Figure 34 shows an example of Blat search results for identifying terminal regions on the centromere side of the HLA-similar sequence-cluster region (iv).

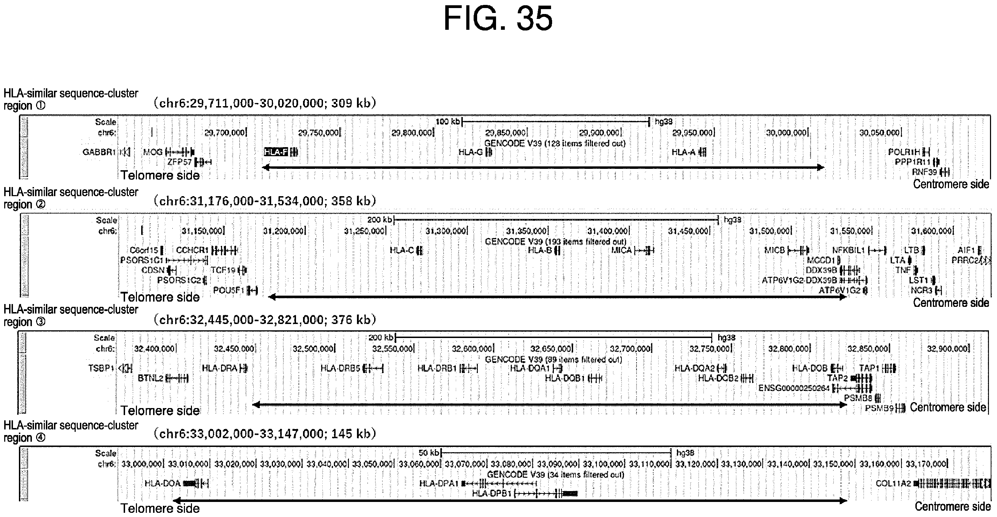

[Figure 35] Figure 35 shows a summary of the results of Figures 27 to 34.
[Figure 36] Figure 36 shows a scheme of a method for removing the whole series of regions comprising HLA-F, HLA-G, and HLA-A by the method of the present disclosure and results thereof.
[Figure 37] Figure 37 shows a scheme of a method for removing the whole series of regions comprising HLA-C and HLA-B by the method of the present disclosure and results thereof. The resulting cells lack HLA-F, HLA-G, and HLA-A, as well as HLA-C and HLA-B.

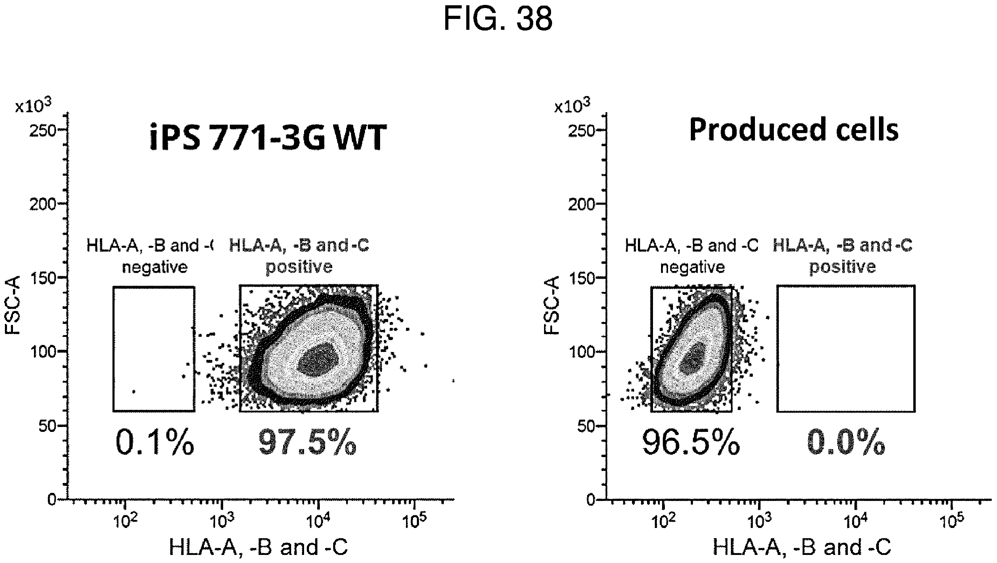

[Figure 38] Figure 38 is a cytogram showing that the resulting cells are negative for all of HLA-A, HLA-B, and HLA-C.
[Figure 39] Figure 39 shows that the resulting cells exhibit expression of pluripotent stem cell markers comparable to unengineered iPS cells.
[Figure 40] Figure 40 shows a scheme of a method for further deleting the HLA-E region and results thereof.
[Figure 41] Figure 41 shows a scheme of a method for further deleting the HLA-E region and results thereof.
[Figure 42] Figure 42 shows a scheme for introducing HLA-E with a signal sequence added in place of HLA-E and results thereof.
[Figure 43] Figure 43 shows a scheme of a method for further removing the whole series of regions comprising HLA-DRAs, HLA-DRBs, HLA-DQAs, HLA-DQB1, and HLA-DOBs by the method of the present disclosure and results thereof. The resulting cells lack HLA-F, HLA-G, and HLA-A; HLA-C and HLA-B; and HLA-DRAs, HLA-DRBs, HLA-DQAs, HLA-DQB1, and HLA-DOBs.
[Figure 44] Figure 44 shows a scheme of a method for further removing the whole series of regions comprising HLA-DRAs, HLA-DRBs, HLA-DQAs, HLA-DQB1, and HLA-DOBs by the method of the present disclosure and results thereof. The resulting cells lack HLA-F, HLA-G, and HLA-A; HLA-C and HLA-B; and HLA-DRAs, HLA-DRBs, HLA-DQAs, HLA-DQB1, and HLA-DOB.
[Figure 45] Figure 45 shows a scheme for introducing an arbitrary gene of interest into a region with a deletion of HLA-E.
[Figure 46] Figure 46 shows a scheme for introducing a foreign gene (e.g., HLA-A, HLA-B, HLA-C, and HLA-E) into a defective site of HLA-E.
[Figure 47] Figure 47 shows a scheme for integrating HLA-A and -B into a cell obtained by replacing HLA-E with a UKiS marker in a cell in which a series of regions comprising HLA-F, HLA-G, and HLA-A and a series of regions comprising HLA-C and HLA-B have been deleted, and results thereof.
[Figure 48] Figure 48 shows a scheme for integrating HLA-C and -E into a cell obtained by replacing HLA-E with a UKiS marker in a cell in which a series of regions comprising HLA-F, HLA-G, and HLA-A and a series of regions comprising HLA-C and HLA-B have been deleted (see Figure 37), and results thereof.
[Figure 49] Figure 49 shows cell surface expression of HLA in a cell re-harboring HLA-A and -B (see Figure 47) and a cell re-harboring HLA-C and E- (see Figure 48).
[Figure 50] Figure 50 shows that HLA-deficient cells evade T cell cytotoxicity.

### Description of Embodiments

### [Definition]

The terms "polynucleotide" and "nucleic acid" are used interchangeably with each other and each refer to a nucleotide polymer in which nucleotides are linked through phosphodiester bonds. The "polynucleotide" or the "nucleic acid" may be DNA, may be RNA, or may be constituted by a combination of DNA and RNA. The "polynucleotide" or the "nucleic acid" may be a polymer of natural nucleotides, may be a polymer of natural nucleotides and non-natural nucleotides (analogs of natural nucleotides, nucleotides modified at one of their base moiety, sugar moiety and phosphate moiety (e.g., phosphorothioate skeletons), etc.), or may be a polymer of non-natural nucleotides.

The nucleotide sequence of the "polynucleotide" or the "nucleic acid" is described by generally accepted single-letter codes unless otherwise specified. Each nucleotide sequence is described from the 5' side toward the 3' side unless otherwise specified. The nucleotide residues constituting the "polynucleotide" or the "nucleic acid" may be simply described by adenine, thymine, cytosine, guanine, or uracil, etc., or their single-letter codes.

The term "gene" refers to a polynucleotide containing at least one open reading frame encoding a particular protein. The gene can contain both an exon and an intron.

The terms "polypeptide", "peptide" and "protein" are used interchangeably with each other and each refer to a polymer of amino acids linked through amide bonds. The "polypeptide", the "peptide" or the "protein" may be a polymer of natural amino acids, may be a polymer of natural amino acids and non-natural amino acids (chemical analogs, modified derivatives, etc. of natural amino acids), or may be a polymer of non-natural amino acids. Each amino acid sequence is described from the N-terminal side toward the C-terminal side unless otherwise specified.

The term "alleles" refer to a set of nucleotide sequences present at the same locus on the chromosomal genome. In an aspect, a diploid cell has two alleles at the same locus, and a triploid cell has three alleles at the same locus. In an aspect, an additional allele may be formed by an abnormal copy of the chromosome or an abnormal additional copy of the locus.

The terms "genome engineering" and "genome editing" are used interchangeably with each other and each refer to mutagenesis at a desired position (target region) in the genome. The genome engineering can involve using a sequence-specific nucleic acid cleaving molecule designed so as to cleave DNA of the target region. In a preferred embodiment, the genome engineering can involve using nuclease manipulated so as to cleave DNA of the target region. In a preferred embodiment, the genome engineering can involve using nuclease (e.g., TALEN or ZFN) manipulated so as to cleave a target sequence having a particular nucleotide sequence in the target region. In a preferred embodiment, the genome engineering may employ sequence-specific endonuclease such as a restriction enzyme (e.g., meganuclease) having only one cleavage site in the genome (e.g., a restriction enzyme having 16-base sequence specificity (theoretically, which is present at a ratio of 1 out of 4¹⁶ bases), a restriction enzyme having 17-base sequence specificity (theoretically, which is present at a ratio of 1 out of 4¹⁷ bases), and a restriction enzyme having 18-base sequence specificity (theoretically, which is present at a ratio of 1 out of 4¹⁸ bases)) so as to cleave a target sequence having a particular nucleotide sequence in the target region. Typically, use of site-specific nuclease induces double-strand break (DSB) in DNA of the target region, followed by the repair of the genome by an endogenous process of cells, such as homologous directed repair (HDR) and non-homologous end-joining repair (NHEJ). NHEJ is a repair method of linking ends that have undergone double-strand break, without the use of a donor DNA, and induces insertion and/or deletion (indel) with high frequency during the repair. HDR is a repair mechanism using a donor DNA and is also capable of introducing a desired mutation to a target region. Examples of the genome engineering technique preferably include a CRISPR/Cas system. The meganuclease that can be used is, for example, meganuclease selected from the group consisting of I-SceI, I-SceII, I-SceIII, I-SceIV, I-SceV, I-SceVI, I-SceVII, I-CeuI, I-CeuAIIP, I-CreI, I-CrepsbIP, I-CrepsbIIP, I-CrepsbIIIP, I-CrepsbIVP, I-TliI, I-PpoI, PI-PspI, F-SceI, F-SceII, F-SuvI, F-TevI, F-TevII, I-AmaI, I-AniI, I-ChuI, I-CmoeI, I-CpaI, I-CpaII, I-CsmI, I-CvuI, I-CvuAIP, I-DdiI, I-DdiII, I-DirI, I-DmoI, I-HmuI, I-HmuII, I-HsNIP, I-LlaI, I-MsoI, I-NaaI, I-NanI, I-NclIP, I-NgrIP, I-NitI, I-NjaI, I-Nsp236IP, I-PakI, I-PboIP, I-PcuIP, I-PcuAI, I-PcuVI, I-PgrIP, I-PobIP, I-PorI, I-PorIIP, I-PbpIP, I-SpBetaIP, I-ScaI, I-SexIP, I-SneIP, I-SpomI, I-SpomCP, I-SpomIP, I-SpomIIP, I-SquIP, I-Ssp68031, I-SthPhiJP, I-SthPhiST3P, I-SthPhiSTe3bP, I-TdeIP, I-TevI, I-TevII, I-TevIII, I-UarAP, I-UarHGPAIP, I-UarHGPA13P, I-VinIP, I-ZbiIP, PI-MtuI, PI-MtuHIP PI-MtuHIIP, PI-PfuI, PI-PfuII, PI-PkoI, PI-PkoII, PI-Rma43812IP, PI-SpBetaIP, PI-SceI, PI-TfuI, PI-TfuII, PI-ThyI, PI-TliI, and PI-TliII and their functional derivative restriction enzymes, or a cleavage site (or a recognition site) thereof, preferably meganuclease which is a restriction enzyme having 18-base or more sequence specificity, or a cleavage site (or a recognition site) thereof, particularly, meganuclease that does not cleave one location or two or more locations of the genome in a cell, or a cleavage site thereof.

The term "target region" refers to a genomic region that is subject to genome engineering. The term "deletion" includes a deletion of one or more bases and a deletion of one or more genes relative to a reference genome. The deletion can be a deletion of 100 bp or more, a deletion of 200 bp or more, a deletion of 300 bp or more, a deletion of 400 bp or more, a deletion of 500 bp or more, a deletion of 600 bp or more, a deletion of 700 bp or more, a deletion of 800 bp or more, a deletion of 900 bp or more, a deletion of 1 kbp or more, a deletion of 10 kbp or more, a deletion of 50 kbp or more, a deletion of 100 kbp or more, a deletion of 200 kbp or more, a deletion of 300 kbp or more, a deletion of 400 kbp or more, a deletion of 500 kbp or more, or a deletion of 1 Mbp or more, or a smaller deletion. The deletion can be a deletion of 1 Mbp or less. The deletion can be a deletion of 700 kbp or less. The deletion can be a deletion of 600 kbp or less. The deletion can be a deletion of 500 kbp or less. The deletion can be a deletion of 10 kbp or more and 600 kbp or less. The deletion can be a deletion of 100 kbp or more and 600 kbp or less. The deletion can be a deletion of 100 kbp or more and 500 kbp or less.

The term "donor DNA" is DNA for use in the repair of double-strand break of DNA and refers to DNA homologously recombinable with neighboring DNA of a target region. The donor DNA comprises, as homology arms, a nucleotide sequence upstream and a nucleotide sequence downstream of a target region (e.g., nucleotide sequences adjacent to a target region). In the present specification, the homology arm consisting of an upstream nucleotide sequence of (e.g., an upstream nucleotide sequence adjacent to) the target region is also referred to as an "upstream homology arm", and the homology arm consisting of a downstream nucleotide sequence of (e.g., a downstream nucleotide sequence adjacent to) the target sequence is also referred to as a "downstream homology arm". The donor DNA can comprise a desired nucleotide sequence between the upstream homology arm and the downstream homology arm. The length of each homology arm is preferably 300 bp or more and is usually on the order of 500 to 3000 bp. The upstream homology arm and the downstream homology arm may have the same lengths or may have different lengths. When a target region successfully induces homologous recombination with the donor DNA after sequence-dependent cleavage, the sequence between the nucleotide sequence upstream and the nucleotide sequence downstream of the target region is replaced with the sequence of the donor DNA.

The term "upstream" of the target region means a DNA region positioned on the 5' side of a reference nucleotide strand in the double-stranded DNA of the target region. The term "downstream" of the target region means a DNA region positioned on the 3' side of the reference nucleotide strand. When the target region comprises a protein coding sequence, the reference nucleotide strand is usually a sense strand. In general, a promoter is positioned upstream of the protein coding sequence. A terminator is positioned downstream of the protein coding sequence.

The term "sequence-specific nucleic acid cleaving molecule" refers to a molecule that can recognize a particular nucleic acid sequence and cleave a nucleic acid at the particular nucleic acid sequence. The sequence-specific nucleic acid cleaving molecule is a molecule having activity of cleaving a nucleic acid in a sequence-specific manner (sequence-specific nucleic acid cleaving activity).

The term "target sequence" refers to a DNA sequence, in the genome, to be cleaved by the sequence-specific nucleic acid cleaving molecule. When the sequence-specific nucleic acid cleaving molecule is Cas protein, the target sequence refers to a DNA sequence, in the genome, to be cleaved by the Cas protein. In the case of using Cas9 protein as the Cas protein, the target sequence needs to be a sequence adjacent to the 5' side of a protospacer adjacent motif (PAM). The target sequence is usually selected as a sequence of 17 to 30 bases (preferably 18 to 25 bases, more preferably 19 to 22 bases, further preferably 20 bases) immediately adjacent to the 5' side of PAM. The target sequence can be designed using a design tool known in the art such as CRISPR DESIGN (crispr.mit.edu/).

The term "Cas protein" refers to CRISPR-associated protein. In a preferred aspect, the Cas protein forms a complex with guide RNA and exhibits endonuclease activity or nickase activity. Examples of the Cas protein include, but are not particularly limited to, Cas9 protein, Cpf1 protein, C2c1 protein, C2c2 protein, and C2c3 protein. The Cas protein encompasses wild-type Cas protein and its homologs (paralogs and orthologs), and their mutants as long as they exhibit endonuclease activity or nickase activity in cooperation with guide RNA.

In a preferred aspect, the Cas protein is involved in a class 2 CRISPR/Cas system and more preferably involved in a type II CRISPR/Cas system. Preferred examples of the Cas protein include Cas9 protein. Preferred examples of the Cas protein include Cas3 protein.

The term "Cas9 protein" refers to Cas protein that is involved in a type II CRISPR/Cas system. The Cas9 protein forms a complex with guide RNA and exhibits activity of cleaving DNA of a target region in cooperation with the guide RNA. The Cas9 protein encompasses wild-type Cas9 protein and its homologs (paralogs and orthologs), and their mutants as long as they exhibit the activity described above. The wild-type Cas9 protein has a RuvC domain and a HNH domain as nuclease domains. In the present specification, any one of the RuvC domain and the HNH domain in the Cas9 protein may be inactivated. Cas9 in which any one of the RuvC domain and the HNH domain is inactivated introduces single-strand cleavage (nick) in double-stranded DNA. Hence, in the case of using Cas9 in which any one of the RuvC domain and the HNH domain is inactivated in the cleavage of double-stranded DNA, an engineering system can be configured such that a target sequence of Cas9 is set in each of the sense strand and the antisense strand and nick for the sense strand and nick for the antisense strand occur at sufficiently close positions, thereby inducing double-strand break.

Examples of the organism species from which the Cas9 protein is derived preferably include, but are not particularly limited to, bacteria belonging to the genus *Streptococcus,* the genus *Staphylococcus,* the genus *Neisseria,* or the genus *Treponema.* More specifically, examples thereof preferably include Cas9 protein derived from *S. pyogenes, S. thermophilus, S. aureus, N. meningitidis,* or *T. denticola.* In a preferred aspect, the Cas9 protein is *S. pyogenes*-derived Cas9 protein.

Information on the amino acid sequence of each Cas protein, and its coding sequence can be obtained from various databases such as GenBank, UniProt, and Addgene. For example, a sequence registered under plasmid No. 42230 in Addgene can be used as the amino acid sequence of *S. pyogenes* Cas9 protein. One example of the amino acid sequence of *S. pyogenes* Cas9 protein is shown in SEQ ID NO: 1.

The terms "guide RNA" and "gRNA" are used interchangeably with each other and each refer to RNA that can form a complex with Cas protein and lead the Cas protein to a target region. In a preferred aspect, the guide RNA comprises CRISPR RNA (crRNA) and transactivating CRISPR RNA (tracrRNA). crRNA is involved in binding to a target region in the genome, and tracrRNA is involved in binding to the Cas protein. In a preferred aspect, crRNA comprises a spacer sequence and a repeat sequence, and the spacer sequence binds to a complementary strand of a target sequence in the target region. In a preferred aspect, tracrRNA comprises an anti-repeat sequence and a 3' tail sequence. The anti-repeat sequence has a sequence complementary to the repeat sequence of crRNA and forms base pairs with the repeat sequence. The 3' tail sequence usually forms three stem loops.

The guide RNA may be single-guide RNA (sgRNA) in which the 5' end of tracrRNA is linked to the 3' end of crRNA, or may be formed by the base pairing of the repeat sequence and the anti-repeat sequence of crRNA and tracrRNA prepared as separate RNA molecules. In a preferred aspect, the guide RNA is sgRNA.

The repeat sequence of crRNA and the sequence of tracrRNA can be appropriately selected according to the type of the Cas protein, and sequences derived from the same bacterial species as that for the Cas protein can be used.

In the case of using, for example, *S. pyogenes-*derived Cas9 protein, the length of sgRNA can be on the order of 50 to 220 nucleotides (nt) and is preferably on the order of 60 to 180 nt, more preferably on the order of 80 to 120 nt. The length of crRNA can be a length of approximately 25 to 70 bases including a spacer sequence, and is preferably on the order of 25 to 50 nt. The length of tracrRNA can be on the order of 10 to 130 nt and is preferably on the order of 30 to 80 nt.

The repeat sequence of crRNA may be the same as that in the bacterial species from which the Cas protein is derived, or may be 3'-terminally truncated. tracrRNA may have the same sequence as that of mature tracrRNA in the bacterial species from which the Cas protein is derived, or may be a terminally truncated form of the mature tracrRNA obtained by the cleavage of the 5' end and/or the 3' end. For example, tracrRNA can be a terminally truncated form of the mature tracrRNA obtained by the removal of approximately 1 to 40 nucleotide residues from the 3' end. tracrRNA can be a terminally truncated from of the mature tracrRNA obtained by the removal of approximately 1 to 80 nucleotide residues from the 5' end. tracrRNA can be, for example, a terminally truncated from of the mature tracrRNA obtained by the removal of approximately 1 to 20 nucleotide residues from the 5' end and the removal of approximately 1 to 40 nucleotide residues from the 3' end.

Various crRNA repeat sequences and tracrRNA sequences for sgRNA design have been proposed. Those skilled in the art can design sgRNA on the basis of a technique known in the art (e.g., Jinek et al., (2012) Science, 337, 816-21; Mali et al., (2013) Science, 339: 6121, 823-6; Cong et al., (2013) Science, 339: 6121, 819-23; Hwang et al., (2013) Nat. Biotechnol. 31: 3, 227-9; and Jinek et al., (2013) eLife, 2, e00471).

The terms "protospacer adjacent motif" and "PAM" are used interchangeably with each other and each refer to a sequence that is recognized by Cas protein upon DNA cleavage by the Cas protein. The sequence and position of PAM differ depending on the type of the Cas protein. In the case of, for example, Cas9 protein, PAM needs to be a downstream sequence immediately adjacent to the 3' side of the target sequence. The sequence of PAM compatible with the Cas9 protein differs depending on the bacterial species from which the Cas9 protein is derived. For example, PAM compatible with *S. pyogenes* Cas9 protein is "NGG". PAM compatible with *S. thermophilus* Cas9 protein is "NNAGAA". PAM compatible with *S. aureus* Cas9 protein is "NNGRRT" or "NNGRR(N)". PAM compatible with *N. meningitidis* Cas9 protein is "NNNNGATT". PAM compatible with *T. denticola* Cas9 protein is "NAAAAC" ("R" is A or G; "N" is A, T, G or C).

The terms "spacer sequence" and "guide sequence" are used interchangeably with each other and each refer to a sequence that is contained in guide RNA and is capable of binding to a complementary strand of a target sequence. Usually, the spacer sequence is a sequence identical to the target sequence (except that T in the target sequence corresponds to U in the spacer sequence). In an embodiment of the present invention, the spacer sequence can contain a 1-base mismatch or mismatches of two or more bases with respect to the target sequence. In the case of containing mismatches of two or more bases, the mismatches may be present at adjacent positions or may be present at distant positions. In a preferred aspect, the spacer sequence can contain 1- to 5-base mismatches with respect to the target sequence. In a particularly preferred aspect, the spacer sequence may contain a 1-base mismatch with respect to the target sequence.

In the guide RNA, the spacer sequence is placed on the 5' side of crRNA.

The term "functionally linked" used in relation to a polynucleotide means that a first nucleotide sequence is placed sufficiently close to a second nucleotide sequence so that the first nucleotide sequence is capable of influencing the second nucleotide sequence or a region controlled by the second nucleotide sequence. For example, the phrase "polynucleotide is functionally linked to a promoter" means that the polynucleotide is linked so as to be expressed under the control of the promoter.

The term "expressible state" refers to a state in which a polynucleotide can be transcribed in a cell harboring the polynucleotide.

The term "expression vector" is a vector containing a subject polynucleotide and refers to a vector having a system that puts the subject polynucleotide in an expressible state in a cell harboring the vector. For example, the "Cas protein expression vector" means a vector that permits expression of the Cas protein in a cell harboring the vector. For example, the "guide RNA expression vector" means a vector that permits expression of the guide RNA in a cell harboring the vector.

The term "HLA-similar sequence" means a stretch of nucleotide sequence having an identity value of 80% or more with at least one of HLA genes, as derived from a BLAT search included in the UCSC Genome Browser. The HLA locus includes a region in which HLA and HLA-similar sequences accumulate, and the region is referred to as an HLA-similar sequence-cluster region.

The term "MHC-similar sequence" means a stretch of nucleotide sequence having an identity value of 80% or more with at least one of MHC genes, as derived from a BLAT search included in the UCSC Genome Browser. The MHC locus includes a region in which MHC and MHC-similar sequences accumulate, and the region is referred to as an MHC-similar sequence-cluster region.

The term "unique sequence" means a specific sequence having no other similar sequences, and specifically means a sequence that has an identity value of 80% or more according to the BLAT search for the full length thereof and exists only at one position in the genome having the unique sequence. The unique sequence is more preferably a sequence that has an identity value of 75% or more according to the BLAT search and exists only at one position in the genome having the unique sequence, further preferably a sequence that has an identity value of 70% or more according to the BLAT search and exists only at one position in the genome having the unique sequence, still more preferably a sequence that has an identity value of 65% or more according to the BLAT search and exists only at one position in the genome having the unique sequence, and even more preferably a sequence that has an identity value of 60% or more according to the BLAT search and exists only at one position in the genome having the unique sequence. The "unique sequence" can be used interchangeably with the "specific sequence".

HLA class I (e.g., HLA-A to C) forms a complex in which an endogenous peptide having about nine amino acid residues is embedded in a groove and presented to killer T cells. When non-self peptides are presented to the complex, for example, in infected cells and cancer cells, the killer T cells destroy the entire cells presenting the non-self peptides. HLA class II (e.g., HLA-DR, DQ, and DP) embeds a phagocytosed foreign peptide having about 15 amino acid residues in a groove and presents the peptide to helper T cells. Immune checkpoint molecules are factors involved in immune checkpoints that suppress immunity. Examples of the immune checkpoint molecules include PD-L1, PD-L2, B7 (CD80/CD86), CD275, CD276, VISTA, galectin-9, HVEM, and HLA class II molecules.

The cells of the present disclosure can be iPS cells. The cells of the present disclosure can be human cell-derived iPS cells (human iPS cells). The cells of the present disclosure can be used, for example, after being differentiated into immune cells. Thus, the cells of the present disclosure can be immune cells (particularly, iPS cell-derived immune cells). The immune cells can be, for examples, one or more immune cells selected from the group consisting of T cells (e.g., CD4 single positive T cells and CD8 single positive T cells), natural killer T cells (NKT cells), natural killer cells (NK cells), regulatory T cells (Treg), αβT cells, γδT cells, and macrophages. The immune cells may have an antigen-specific T cell receptor (TCR). The immune cells may not express an antigen-specific T cell receptor (e.g., may have a TCRα-chain deletion). The immune cells may express a chimeric antigen receptor (CAR). For example, the immune cells may be T cells, NKT cells, NK cells, or yδ T cells that express a CAR but may not express an antigen-specific TCR. The cell can be, for example, a primary cell (e.g., a primary immune cell). The cell can be, for example, a cell line (e.g., an immune cell line). The cell can be a non-cancer cell. The cell expressing a CAR may, for example, express one or both of endogenous or foreign interleukin-7 (IL-7) and endogenous or foreign CCL19. The endogenous or foreign IL-7 and endogenous or foreign CCL19 are each operably linked to a control sequences, and the cell expressing a CAR has any or preferably both of the IL-7 and CCL19. The cell expressing a CAR may also express endogenous or foreign endo β-D-glucuronidase (HPSE). Such immune cells may have an autologous or allogeneic relationship with the subject to which the cells are administered. The immune cells may also express endogenous or foreign CD3. The cells may or may not express one or more endogenous or exogenous factors selected from the group consisting of HLA-E, HLA-G, HACD16, 41BBL, CD3, CD4, CD8, CD47, CD137, CD80, PDL1, A2AR, CAR, and TCR. In an aspect, the cells have nucleic acids encoding one or more endogenous or exogenous factors selected from the group consisting of HLA-E, HLA-G, CD16, 41BBL, CD3, CD4, CD8, CD47, CD137, CD80, PDL1, A2AR, CAR, and TCR, wherein the nucleic acids are operably linked to control sequences. In an aspect, the cell expressing a CAR may be used in combination with antibody therapy. In this combination, a target molecule (tumor antigen) of the antibody may be knocked out in the cell expressing a CAR. In an aspect, examples of such tumor antigens include CD38, and CD52. Examples of anti-CD38 antibodies include daratumumab and isatuximab (the disease to be treated can be, for example, multiple myeloma). Examples of anti-CD52 antibodies include alemtuzumab (the disease to be treated may be, for example, leukemia, lymphoid leukemia, or chronic lymphocytic leukemia). In an aspect of the cells (e.g., immune cells or non-immune cells), NLRC5 may also be knocked out, thereby suppressing, for example, the development of graft-versus-host disease (GVHD). In an aspect, the cells (e.g., immune cells or non-immune cells) may express a CAR or TCR with a factor selected from the group consisting of PD1, CD52, CTLA4, dCK, GGH, HPRT, and β2 microglobulin. In a preferred aspect, the cells of the present disclosure have functional β2 microglobulin and/or functional CIITA. The cells of the present disclosure may, for example, express an IL-15:IL15Rα fusion protein.

As used herein, the "chimeric antigen receptor" (CAR) is a chimeric molecule having an antigen-binding fragment of an antibody (particularly scFv) and an activation domain for an immune cell. The CAR is generally a molecule consisting of a scFv, an extracellular hinge domain, a transmembrane domain (e.g., CD8α or CD28), and an activation signaling domain (e.g., CD3ζ) linked together. The CAR can be introduced into a cell and expressed on the cell surface. The cell expressing a CAR can be targeted against a specific antigen. For example, the CAR is introduced into immune cells such as T cells and NK cells to target the immune cells such as T cells and NK cells to cancer. While in the first generation CAR, a scFv, an extracellular hinge domain, a transmembrane domain (e.g., CD8α or CD28), and an activation signaling domain (e.g., CD3ζ) were linked together, the second generation CAR additionally contains a co-stimulatory molecule signaling domain for CAR-introduced immune cell activation. As the co-stimulatory molecule signaling domain, co-stimulatory factors such as CD28, 4-1BB, OX40, CD27, and ICOS have been used. In the third generation CAR, a plurality of co-stimulatory factors are integrated. Thus, modifications have been made to the CAR in order to sustainably expand the CAR-introduced immune cells *in vivo.* Preferably, any domain other than the scFv moiety is derived from a human protein.

In one embodiment, the endogenous or exogenous gene comprises any one or more of endogenous or exogenous HLA genes. Examples of the HLA gene include, but are not particularly limited to, HLA-A, HLA-B, HLA-C, HLA-E, HLA-F, HLA-G, HLA-H, HLA-J, HLA-W, MICA, MICB, HLA-DRA, HLA-DRB1, HLA-DRB5, HLA-DRB6, HLA-DRB9, HLA-DQA1, HLA-DQA2, HLA-DQB1, HLA-DPA1, HLA-DPB1, HLA-DMB, HLA-DOA, and HLA-DOB. In one embodiment, the HLA gene can be one or more selected from the group consisting of HLA-A, HLA-B, HLA-C, HLA-DR, HLA-DQ, and HLA-DP. In one embodiment, the HLA gene can be one or more selected from the group consisting of HLA-A, HLA-B, and HLA-C. In one embodiment, the HLA gene can be one or more selected from the group consisting of HLA-DR, HLA-DQ, and HLA-DP.

In one embodiment, the HLA gene can be, for example, HLA-A. The HLA-A may be HLA-A of any allele. Examples of the HLA-A include, but are not particularly limited to, A*01:01, A*02:01, A*02:03, A*02:05, A*02:06, A*02:07, A*02:10, A*02:11, A*02:15N, A*02:18, A*02:28, A*02:42, A*02:53N, A*02:59, A*02:72, A*03:01, A*03:02, A*11:01, A*11:02, A*11:13, A*23:01, A*24:02, A*24:03, A*24:04, A*24:05, A*24:07, A*24:08, A*24:10, A*24:20, A*24:25, A*24:28, A*24:46, A*25:01, A*26:01, A*26:02, A*26:03, A*26:04, A*26:05, A*26:06, A*29:01, A*29:02, A*30:01, A*30:02, A*30:04, A*31:01, A*31:11, A*32:01, A*33:01, A*33:03, A*33:08, A*34:01, and A*68:01. The HLA gene can also be, for example, HLA-B. The HLA-B may be HLA-B of any allele. Examples of the HLA-B include, but are not particularly limited to, B*07:02, B*07:05, B*08:01, B*13:01, B*13:02, B*14:01, B*14:02, B*15:01, B*15:02, B*15:03, B*15:05, B*15:07, B*15:11, B*15:12, B*15:13, B*15:17, B*15:18, B*15:21, B*15:25, B*15:26N, B*15:27, B*15:28, B*15:35, B*15:38, B*15:46, B*18:01, B*18:02, B*27:04, B*27:05, B*27:06, B*27:11, B*35:01, B*35:02, B*35:03, B*35:05, B*35:08, B*35:11, B*35:51, B*35:64, B*37:01, B*38:01, B*38:02, B*39:01, B*39:02, B*39:04, B*39:05, B*39:23, B*40:01, B*40:02, B*40:03, B*40:06, B*40:07, B*40:11, B*40:50, B*40:52, B*41:01, B*44:02, B*44:03, B*45:01, B*46:01, B*48:01, B*48:03, B*49:01, B*50:01, B*51:01, B*51:02, B*51:03, B*51:06, B*52:01, B*53:01, B*54:01, B*54:21, B*55:01, B*55:02, B*55:04, B*55:10, B*55:12, B*56:01, B*56:03, B*56:04, B*56:05, B*57:01, B*58:01, B*59:01, and B*67:01.

In one embodiment, the HLA gene can be HLA-C. The HLA-C may be HLA-C of any allele. Examples of the HLA-C include, but are not particularly limited to, C*01:02, C*01:03, C*01:55, C*02:02, C*03:02, C*03:03, C*03:04, C*03:23N, C*03:28, C*03:29, C*03:43, C*04:01, C*04:03, C*05:01, C*06:02, C*07:01, C*07:02, C*07:02N, C*07:04, C*08:01, C*08:02, C*08:03, C*08:39, C*12:02, C*12:03, C*12:04, C*14:02, C*14:03, C*15:02, C*15:05, C*15:10, C*16:01, C*16:02, and C*17:01.

In one embodiment, the HLA gene can be HLA-DR. The HLA-DR may be HLA-DR of any allele. Examples of the HLA-DR include, but are not particularly limited to, DRB1*01:01, DRB1*01:02, DRB1*01:03, DRB1*03:01, DRB1*04:01, DRB1*04:02, DRB1*04:03, DRB1*04:04, DRB1*04:05, DRB1*04:06, DRB1*04:07, DRB1*04:08, DRB1*04:09, DRB1*04:10, DRB1*04:11, DRB1*07:01, DRB1*08:01, DRB1*08:02, DRB1*08:03, DRB1*08:09, DRB1*08:23, DRB1*09:01, DRB1*10:01, DRB1*11:01, DRB1*11:04, DRB1*11:06, DRB1*11:08, DRB1*11:19, DRB1*11:23, DRB1*12:01, DRB1*12:02, DRB1*12:05, DRB1*13:01, DRB1*13:02, DRB1*13:03, DRB1*13:07, DRB1*13:12, DRB1*14:02, DRB1*14:03, DRB1*14:04, DRB1*14:05, DRB1*14:06, DRB1*14:07, DRB1*14:12, DRB1*14:29, DRB1*14:45, DRB1*14:54, DRB1*15:01, DRB1*15:02, DRB1*15:04, DRB1*16:01, DRB1*16:02, DRB3*01:01, DRB3*02:02, DRB3*03:01, DRB4*01:01, DRB4*01:02, DRB4*01:03, DRB5*01:01, DRB5*01:02, and DRB5*02:02.

In one embodiment, the HLA gene can be HLA-DQ. The HLA-DQ may be HLA-DQ of any allele. Examples of the HLA-DQ include, but are not particularly limited to, DQB1*02:01, DQB1*02:02, DQB1*03:01, DQB1*03:02, DQB1*03:03, DQB1*04:01, DQB1*04:02, DQB1*05:01, DQB1*05:02, DQB1*05:03, DQB1*06:01, DQB1*06:02, DQB1*06:03, DQB1*06:04, DQB1*06:09, DQA1*01:01, DQA1*01:02, DQA1*01:03, DQA1*01:04, DQA1*01:05, DQA1*02:01, DQA1*03:01, DQA1*03:02, DQA1*03:03, DQA1*04:01, DQA1*05:01, DQA1*05:03, DQA1*05:05, DQA1*05:06, DQA1*05:08, and DQA1*06:01.

In one embodiment, the HLA gene can be HLA-DP. The HLA-DP may be HLA-DP of any allele. Examples of the HLA-DP include, but are not particularly limited to, DPB1*02:01, DPB1*02:02, DPB1*03:01, DPB1*04:01, DPB1*04:02, DPB1*05:01, DPB1*06:01, DPB1*09:01, DPB1*13:01, DPB1*14:01, DPB1*17:01, DPB1*19:01, DPB1*36:01, DPB1*38:01, DPB1*41:01, DPA1*01:03, DPA1*02:01, DPA1*02:02, and DPA1*04:01.

In one embodiment, the insertion comprises an endogenous or exogenous gene and an upstream sequence of the gene comprising the control sequence or a presumed control sequence. Specifically, the insertion in this embodiment comprises an endogenous or exogenous gene and the native control sequence of the gene or a candidate thereof upstream of the gene. In one embodiment, the insertion comprises an endogenous or exogenous gene, wherein the gene comprises one or more introns or all of the introns. In one embodiment, the insertion comprises an upstream sequence of the gene comprising a control sequence or a presumed control sequence and the gene comprising one or more introns or all of the introns. In one embodiment, the insertion has a sequence of a stretch of region in the genome, wherein the region comprises the gene, an upstream sequence of the gene comprising a control sequence or a presumed control sequence and all of introns. In this way, the effects of the control sequence on gene expression, for example, can be analyzed, and the effects of the intron on gene expression, for example, can be analyzed. In a preferred aspect, the gene can be an HLA gene.

In a preferred aspect, the gene in the genome corresponding to the HLA gene included in the insertion is deleted. In a preferred aspect, when the insertion comprises an HLA gene, all functional HLA in the genome is deleted. The HLA gene included in the insertion may be a null allele.

In one embodiment, the inserted gene can be, for example, a chimeric antigen receptor (CAR). The chimeric antigen receptor can include, for example, a single chain antibody comprising heavy and light chains (e.g., scFv), an extracellular hinge domain (e.g., CD8), a transmembrane domain (e.g., CD8α and CD28), a co-stimulatory molecular signaling domain (4-1BB, CD28, and CD137), and an activation signaling domains (CD3ζ). The chimeric antigen receptor thereby generates an activating signal to the cell upon binding to the target. The chimeric antigen receptor can bind to an antigen expressed on a cancer cell. Such an antigen can be, for example, one or more selected from the group consisting of CD16, CD19, CD20, CD22, CD123, CD171, epidermal growth factor receptor (EGFR), particularly EGFRvIII, type 3 EGFR, de2-7 EGFR and HER2, carcinoembryonic antigen (CEA), prostate stem cell antigen (PSCA), B-cell maturation antigen (BCMA), CS1, NKG2D, NKp30, B7H6, MUC-16 (CA125), receptor tyrosine kinase-like orphan receptor 1 (ROR-1), GD3, GM2, glypican-3 (GPC3), mesothelin, IL13R, c-KIT, c-MET, NY-ESO-1, WT1, MAGE-A3, MAGE-A4, MAGE-A10, HPV E6, HPV E7, CMV, AFP, PRAME, SSX2, KRAS, HER2, and PD-L1. The chimeric antigen receptor may also have binding to, for example, a proteinaceous tag. The antigen of the scFv can be, for example, a fluorescent protein such as fluorescein isothiocyanate (FITC) since cancer cells can also be killed by binding an antibody labeled with a proteinaceous tag to a cancer antigen on the surface of the cancer cells, followed by further administration of cells expressing the chimeric antigen receptor targeting the tag.

In one embodiment, the inserted gene can be, for example, a T cell receptor (TCR). The TCR may have antigen specificity. The antigen-specific TCR can bind to, for example, a cancer antigen. The antigen-specific TCR can bind to, for example, one or more selected from the group consisting of CD16, CD19, CD20, CD22, CD123, CD171, epidermal growth factor receptor (EGFR), particularly EGFRvIII, type 3 EGFR, de2-7 EGFR and HER2, carcinoembryonic antigen (CEA), prostate stem cell antigen (PSCA), B-cell maturation antigen (BCMA), CS1, NKG2D, NKp30, B7H6, MUC-16 (CA125), receptor tyrosine kinase-like orphan receptor 1 (ROR-1), GD3, GM2, glypican-3 (GPC3), mesothelin, IL13R, c-KIT, c-MET, NY-ESO-1, WT1, MAGE-A3, MAGE-A4, MAGE-A10, HPV E6, HPV E7, CMV, AFP, PRAME, SSX2, KRAS, HER2, and PD-L1. The TCR may be selected to be compatible with HLA.

In one embodiment, the inserted gene includes a gene encoding immunosuppressive factors, for example, one or more selected from the group consisting of CD47, CD24, CD200, PD-L1, IDO1, CTLA4-Ig, C1-inhibitor, IL-10, IL-35, FASL, Serpmb9, CC121, and Mfge8.

In one embodiment, the inserted gene can be a therapeutic gene. The therapeutic gene can be a gene that, when expressed in the body, produces a therapeutic benefit. The therapeutic gene can be a proinflammatory protein, for example, a cytokine. The therapeutic gene can be an anti-inflammatory protein, for example, a cytokine.

In one embodiment, the inserted gene can be a suicide gene. Examples of the suicide gene include a thymidine kinase gene, particularly herpes virus-derived thymidine kinase gene (HSVtk), a cytotoxic signal receptor (e.g., diphtheria toxin receptor), and iCas9. The thymidine kinase gene phosphorylates ganciclovir to generate cytotoxic ganciclovir triphosphate. In addition, iCas9 (inducible caspase-9), a protein whose CARD is replaced by FKBP12, induces cell death in an iCas9-expressing cell in the presence of a tacrolimus derivative (e.g., AP1903). Such a suicide gene is beneficial when the body needs to remove cells that evade the immune system, such as universal donor cells (UDCs).

In the present specification, the sequence identity (or homology) between nucleotide sequences or amino acid sequences is determined as the ratio of identical bases or amino acids to the whole nucleotide sequences or the whole amino acid sequences, except for gaps, in alignments obtained by juxtaposing two nucleotide sequences or amino acid sequences so as to attain the highest identity of the corresponding bases or amino acids while placing the gaps in moieties corresponding to insertion and deletion. The sequence identity between nucleotide sequences or amino acid sequences can be determined using various homology search software known in the art. The value of sequence identity (identity value) between nucleotide sequences can be obtained, for example, but not particularly limited to, by a BLAT search included in homology search software UCSC Genome Browser known in the art.

### [Genome engineering method]

In one embodiment, the present invention provides a genome engineering method for engineering two or more alleles in the chromosomal genome. The genome engineering method comprises the following steps (a) and (b) :
(a) introducing the following (i) and (ii) to a cell comprising the chromosome:
   (i) a genome engineering system comprising a sequence-specific nucleic acid cleaving molecule targeting a target region in the chromosomal genome, or a polynucleotide encoding the sequence-specific nucleic acid cleaving molecule, and
   (ii) two or more donor DNAs for selective markers, each of which comprises a nucleotide sequence of a selective marker gene between an upstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering; and
(b) after the step (a), selecting the cell on the basis of all the selective marker genes carried by the two or more donor DNAs for selective markers. In this aspect, the selective marker gene can be unique to each type of donor DNA for the selective marker. In this aspect, the step (b) can be the step of, after the step (a), respectively homologously recombining the two or more alleles with different types of donor DNAs for selective markers so that the distinguishably different unique selective marker genes are respectively introduced in the two or more alleles, and selecting a cell expressing all the distinguishably different selective marker genes thus introduced (step for positive selection). The method may be a method for preparing a cell in which two or more alleles in the chromosomal genome are engineered.

In the present specification, when referring to the position of the human genome for convenience, the position in the hg38 genomic sequence is used as a reference genome. The hg38 is a reference genome released in December 2013 by the University of California, Santa Cruz (UCSC). The reference genome is a genome for reference created by combining various genomes, which does not mean that a human with this genome exist. However, the fragmented sequence information decoded from the genomic DNA of the human individual is compared with the reference genome, and the decoded fragmented sequence information is linked to construct a stretch of sequence on a computer, whereby the sequence of the genomic DNA of the human individual can be inferred. In this way, the genomic DNA of an individual such as a human individual is usually decoded by associating the sequence of the genomic DNA of the human individual with the reference genome. Then, the position or region corresponding to a specific position or specific region of the hg38 genomic sequence means a position or region that is linked to the specific position or specific region in the genome of another individual with a different specific sequence. Specifically, the position or region having a sequence characteristic of the position or region based on the identity of the sequence is a position or region corresponding to a specific position or specific region of the hg38 genomic sequence. The compatible position can be determined by aligning partial sequences of two genomic DNAs. Even when there is a difference in the specific sequence, the correspondence between the two genomic DNAs can be determined by aligning the sequences with the ortholog relationship or the sequence identity. In a region in which paralogs generated by gene duplication are abundant, determination of the sequence correspondence based on simple individual sequences may not be enough to determine the true correspondence between the two genomes. This increases the difficulty of sequencing the region in which similar sequences have accumulated. In the determination of compatible sequences, a correspondence between two genomes can be revealed by determining a high degree of sequence identity. In addition, when a specific region is a large region containing a plurality of genes, synteny can be considered. Synteny refers to preservation of the physical positional relationship of orthologs in the genome. Individuals and organisms can have synteny. Therefore, specific regions can be determined in consideration of synteny.

In one embodiment, the present invention provides a cell having a genome with a deletion of a region (e.g., a deletion of a region up to 1 Mb or up to 500 kb) in all alleles (two alleles in the case of a diploid cell) of the genome, wherein the deletion comprises a region comprising a portion, or preferably the whole, of an MHC-similar sequence-cluster region of a locus encoding an MHC molecule. In this aspect, repetitive sequences due to similar sequences are truncated, thereby improving the readability of the genome and/or enhancing the targeting efficiency of genes (e.g., MHC genes) of the genome. The cell with a deletion may have an insertion of an endogenous or exogenous gene. Preferably, the inserted gene is operably linked to a control sequence. In one embodiment, the cell is a human cell, and the deletion comprises a region of a portion, or preferably the whole, of the MHC-similar sequence-cluster region of the locus encoding the HLA molecule.

In one embodiment, the present invention provides cells lacking one, two, three, or four regions selected from the group consisting of a series of regions from HLA-F to HLA-A (first HLA-similar sequence-cluster region), a series of regions from HLA-C to HLA-B (second HLA-similar sequence-cluster region), a series of regions from HLA-DRA to HLA-DOB (third HLA-similar sequence-cluster region), and a series of regions from HLA-DMB to HLADPB2 (fourth HLA-similar sequence-cluster region), and portions thereof. In one embodiment, the cells may lack the region comprising HLA-E.

The first to fourth HLA-similar sequence-cluster regions can be targeted to their respective unique sequences. In this way, unexpected modification such as off-target cleavage can be prevented. Those skilled in the art may understand how targeting can be performed to prevent off-target cleavage and perform the targeting and engineering as appropriate. Although individual HLA engineering may be difficult due to sequence similarity to other HLAs, a large-scale deletion according to the present disclosure allows for the creation of the desired large-scale deletion because the region in which the unique sequence exists can be freely configured. In one embodiment, the first to fourth HLA-similar sequence-cluster regions are each targeted to sequences outside the first to fourth HLA-similar sequence-cluster regions.

In one embodiment, the first HLA-similar sequence-cluster region can be present at chr6:29,706,837-29,956,199 of the hg38 genomic sequence. In one embodiment, the first HLA-similar sequence-cluster region can be present at chr6:29,711,000-30,020,000 of the hg38 genomic sequence. In one embodiment, the second HLA-similar sequence-cluster region can be present at chr6:31,225,738-31,376,781 of the hg38 genomic sequence. In one embodiment, the second HLA-similar sequence-cluster region can be present at chr6:31,176,000-31,534,000 of the hg38 genomic sequence. In one embodiment, the third HLA-similar sequence-cluster region can be present at chr6:32,445,000-32,821,000 of the hg38 genomic sequence. In one embodiment, the third HLA-similar sequence-cluster region can be present at chr6:32,445,000-32,821,000 of the hg38 genomic sequence. In one embodiment, the fourth HLA-similar sequence-cluster region can be present at chr6:32,934,298-33,161,621 of the hg38 genomic sequence. In one embodiment, the fourth HLA-similar sequence-cluster region can be present at chr6:33,002,000-33,147,000 of the hg38 genomic sequence.

In one embodiment, the first HLA-similar sequence-cluster region can be present at chr6:29,722,775-29,945,870 of the hg38 genomic sequence. In one embodiment, the second HLA-similar sequence-cluster region can be present at chr6:31,268,749-31,357,179 of the hg38 genomic sequence. In one embodiment, the third HLA-similar sequence-cluster region can be present at chr6:32,439,887-32,817,002 of the hg38 genomic sequence. In one embodiment, the fourth HLA-similar sequence-cluster region can be present at chr6:32,934,636-33,089,696 of the hg38 genomic sequence.

In each of the first to fourth HLA-similar sequence-cluster regions, the smallest region containing the HLA to be deleted is identified, and then the regions with unique sequences in the genome on both sides of the region are determined. The determination can be appropriately made with reference to a genome database, for example. Such regions with unique sequences can be used for genome editing and the design of homologous arms for homologous recombination. The homologous arms are typically designed to be capable of homologous recombination (e.g., designed to have the same sequence) as the region with the unique sequence. In a preferred embodiment, the region comprising all functional HLA-encoding genes is deleted in each of the first to fourth HLA-similar sequence-cluster regions. In a preferred embodiment, the region comprising all functional HLA-encoding genes is deleted, provided that the deletion is performed in such a way that another gene present outside of the region is included as few as possible. For example, the region is deleted so as not to include 5 or more, 4 or more, 3 or more, 2 or more, or 1 or more other genes present outside the region. The deleted regions depend on the existence of a region with a unique sequence in the genome in which the homologous recombination arm is capable of homologous recombination. It is desired to conduct a study specific to humans in the case of humans and a study specific to each non-human species in the case of non-humans.

In one embodiment, the present invention provides a cell comprising genomic DNA with a deletion, wherein the deletion is of a region comprising a portion or the whole of an HLA-similar sequence-cluster region located in each of one or more chromosomal regions selected from the group consisting of chromosomal regions defined in the following (i) to (iv):
(i) a chromosomal region corresponding to chr6:29,723,464-29,945,455 chromosomal region of the hg38 genomic sequence,
(ii) a chromosomal region corresponding to chr6:31,269,169-31,357,158 chromosomal region of the hg38 genomic sequence,
(iii) a chromosomal region corresponding to chr6:32,439,951-32,816,951 chromosomal region of the hg38 genomic sequence, and
(iv) a chromosomal region corresponding to chr6:33,006,838-33,086,238 chromosomal region of the hg38 genomic sequence,
wherein
the HLA-similar sequence-cluster region comprises all of HLA-encoding genes and HLA-similar sequences contained in each of the chromosomal regions, and
the portion comprises at least a stretch of region, the stretch of region comprising 50% or more, 60% or more, 70% or more, 80% or more, 90% or more, or 100% of genes selected from the group consisting of the HLA-encoding genes and HLA-similar sequences contained in each of the chromosomal regions. Throughout the present specification, the deletion is a deletion in both the alleles. In an aspect, the cells of the present disclosure may further lack, in addition to the above regions, regions each independently within 20 kbp, 15 kbp, 10 kbp, 9 kbp, 8 kbp, 7 kbp, 6 kbp, 5 kbp, 4 kbp, 3 kbp, 2 kbp, or 1 kbp outwards from both ends thereof. In an aspect, the cells of the present disclosure may not lack one or more (preferably one) HLA genes located at both ends within the above regions.

In one embodiment, the present invention provides a cell comprising genomic DNA with a deletion, wherein the deletion is of a region comprising a portion or the whole of an HLA-similar sequence-cluster region located in each of one or more chromosomal regions selected from the group consisting of chromosomal regions defined in the following (i) to (iv):
(i) a chromosomal region corresponding to chr6:29,723,464-30,010,618) chromosomal region of the hg38 genomic sequence,
(ii) a chromosomal region corresponding to chr6:31,269,169-31,463,338 chromosomal region of the hg38 genomic sequence,
(iii) a chromosomal region corresponding to chr6:32,439,887-32,817,002 chromosomal region of the hg38 genomic sequence, and
(iv) a chromosomal region corresponding to chr6:33,006,838-33,131,199 chromosomal region of the hg38 genomic sequence,
wherein
the HLA-similar sequence-cluster region comprises all of HLA-encoding genes and HLA-similar sequences contained in each of the chromosomal regions, and
the portion comprises at least a stretch of region, the stretch of region comprising 50% or more, 60% or more, 70% or more, 80% or more, 90% or more, or 100% of genes selected from the group consisting of the HLA-encoding genes and HLA-similar sequences contained in each of the chromosomal regions. Throughout the present specification, the deletion is a deletion in both the alleles. In an aspect, the cells of the present disclosure may further lack, in addition to the above regions, regions each independently within 20 kbp, 15 kbp, 10 kbp, 9 kbp, 8 kbp, 7 kbp, 6 kbp, 5 kbp, 4 kbp, 3 kbp, 2 kbp, or 1 kbp outwards from both ends thereof. In an aspect, the cells of the present disclosure may not lack one or more (preferably one) HLA genes located at both ends within the above regions.

In one embodiment, the present invention provides a cell comprising genomic DNA with a deletion, wherein the deletion is of a region comprising a portion or the whole of an HLA-similar sequence-cluster region located in each of one or more chromosomal regions selected from the group consisting of chromosomal regions defined in the following (i) to (iv):
(i) a chromosomal region corresponding to chr6:29,711,000-30,020,000 chromosomal region of the hg38 genomic sequence,
(ii) a chromosomal region corresponding to chr6:31,176,000-31,534,000 chromosomal region of the hg38 genomic sequence,
(iii) a chromosomal region corresponding to chr6:32,445,000-32,821,000 chromosomal region of the hg38 genomic sequence, and
(iv) a chromosomal region corresponding to chr6:33,002,000-33,147,000 chromosomal region of the hg38 genomic sequence,
wherein
the HLA-similar sequence-cluster region comprises all of HLA-encoding genes and HLA-similar sequences contained in each of the chromosomal regions, and
the portion comprises at least a stretch of region, the stretch of region comprising 50% or more, 60% or more, 70% or more, 80% or more, 90% or more, or 100% of genes selected from the group consisting of the HLA-encoding genes and HLA-similar sequences contained in each of the chromosomal regions. Throughout the present specification, the deletion is a deletion in both the alleles. In an aspect, the cells of the present disclosure may further lack, in addition to the above regions, regions each independently within 20 kbp, 15 kbp, 10 kbp, 9 kbp, 8 kbp, 7 kbp, 6 kbp, 5 kbp, 4 kbp, 3 kbp, 2 kbp, or 1 kbp outwards from both ends thereof. In an aspect, the cells of the present disclosure may not lack one or more (preferably one) HLA genes located at both ends within the above regions.

In one embodiment, the present invention provides a cell comprising genomic DNA with a deletion, wherein the deletion is of a region comprising a portion or the whole of an HLA-similar sequence-cluster region located in each of one or more chromosomal regions selected from the group consisting of chromosomal regions defined in the following (i) to (iv):
(i) a chromosomal region corresponding to chr6:29,706,837-29,956,199 chromosomal region of the hg38 genomic sequence,
(ii) a chromosomal region corresponding to chr6:31,268,749-31,534,000 chromosomal region of the hg38 genomic sequence,
(iii) a chromosomal region corresponding to chr6:32,439,887-32,817,002 chromosomal region of the hg38 genomic sequence, and
(iv) a chromosomal region corresponding to chr6:32,934,636-33,089,696 chromosomal region of the hg38 genomic sequence,
wherein
the HLA-similar sequence-cluster region comprises all of HLA-encoding genes and HLA-similar sequences contained in each of the chromosomal regions, and
the portion comprises at least a stretch of region, the stretch of region comprising 50% or more, 60% or more, 70% or more, 80% or more, 90% or more, or 100% of genes selected from the group consisting of the HLA-encoding genes and HLA-similar sequences contained in each of the chromosomal regions. Throughout the present specification, the deletion is a deletion in both the alleles. In an aspect, the cells of the present disclosure may further lack, in addition to the above regions, regions each independently within 20 kbp, 15 kbp, 10 kbp, 9 kbp, 8 kbp, 7 kbp, 6 kbp, 5 kbp, 4 kbp, 3 kbp, 2 kbp, or 1 kbp outwards from both ends thereof. In an aspect, the cells of the present disclosure may not lack one or more (preferably one) HLA genes located at both ends within the above regions.

The chromosomal region defined in (i) above comprises an HLA-similar sequence-cluster region comprising HLA-F, HLA-G, and HLA-A. In an aspect, the deletion is of a portion, or preferably the whole, of the HLA-similar sequence-cluster region, where the portion is a region comprising at least one HLA and HLA-similar sequence. For example, in an aspect, the deletion is of a region comprising one or more, preferably all, selected from the group consisting of HLA-F, HLA-G, and HLA-A. In an aspect, the deletion is of a region comprising the whole HLA-similar sequence-cluster region and comprising HLA-F, HLA-G, and HLA-A.

In an aspect, the cells lacking a portion or the whole of the HLA may have, for example, endogenous or exogenous desired genes that are operably linked to additionally introduced control sequences. The endogenous or exogenous desired genes that are operably linked to additionally introduced control sequences in an aspect have naturally occurring sequences, but in another aspect, are not naturally occurring sequences or are artificially designed sequences. The additionally introduced desired genes can, for example, confer additional functions to the cells. The additionally introduced desired genes can encode any one or more selected from the group consisting of, for example, differentiation enhancing factors, differentiation suppressing factors, reprogramming factors (including factors required for reprogramming to pluripotent cells including Oct4), factors activating specific functions of cells, factors suppressing specific functions of cells, signaling factors, signaling suppressing factors, receptors, membrane proteins, enzymes (e.g., metabolic enzymes, phosphorylation enzymes, nucleic acid-synthesizing enzymes, and telomerase), growth factors, growth-suppressing factors, immunostimulatory molecules or immunosuppressive molecules, degradative enzymes (e.g., proteolytic enzymes and nucleolytic enzymes), secreted factors (e.g., cytokines and hormones), nuclear proteins, cytoplasmic proteins, mitochondrial proteins, antisense oligonucleotides, siRNAs, shRNAs, crRNAs, and trcrRNAs, single-stranded gRNAs, marker genes (e.g., drug resistance genes and visible marker genes), inducible suicide genes (e.g., toxins operably linked to inducible promoters, such as diphtheria toxin), and microRNAs. In an aspect, the control sequence may be a constitutive promoter or an inducible promoter.

In an aspect, the cells lacking a portion of the HLA in (i) above may have (may retain), for example, one to several (e.g., one to four, preferably three, more preferably two, further preferably one) HLA in (i). In an aspect, the cells lacking a portion of the HLA in (ii) above may have (may retain), for example, one to several (e.g., one to four, preferably three, more preferably two, further preferably one) HLA in (ii). In an aspect, the cells lacking a portion of the HLA in (iii) above may have (may retain), for example, one to several (e.g., one to four, preferably three, more preferably two, further preferably one) HLA in (iii). In an aspect, the cells lacking a portion of the HLA in (iv) above may have (may retain), for example, one to several (e.g., one to four, preferably three, more preferably two, further preferably one) HLA in (iv).

In an aspect, the deletion is of a region comprising a portion, or preferably the whole, of the HLA-similar sequence-cluster region. In this aspect, the desired gene is preferably introduced to the chromosomal region defined in (i) above. As a result, in this aspect, the chromosomal region defined in (i) lacks the whole of the HLA-similar sequence-cluster region but has the desired gene (endogenous or preferably exogenous gene) or an insertion thereof operably linked to a control sequence. In an aspect, the deletion is of a region comprising a portion, or preferably the whole, of the HLA-similar sequence-cluster region. In this aspect, the desired gene is preferably reintroduced to a region other than the chromosomal region defined in (i). As a result, the genomic DNA has a deletion of a portion, or preferably the whole, of the HLA-similar sequence-cluster region in the chromosomal region defined in (i) above but has an endogenous or preferably exogenous desired gene operably linked to a control sequence in a region other than the chromosomal region defined in (i). The term "endogenous" means having the same nucleotide sequence as the endogenous gene that the cell with a deletion has or had, and the term "exogenous" means having a different nucleotide sequence than the endogenous gene that the cell with a deletion has or had. The desired gene is a functional gene. The functional gene means that the gene has an intrinsic function. The deletion in (i) comprises at least either HLA-A or HLA-F, or both.

In all aspects, the desired gene operably linked to the control sequence is one in which the control sequence is exogenous or endogenous, and the desired gene is exogenous or endogenous. In an aspect, the control sequence is endogenous, and the desired gene is exogenous. In an aspect, the control sequence is endogenous, and the desired gene is endogenous. In an aspect, the control sequence is exogenous, and the desired gene is endogenous. In an aspect, the control sequence is exogenous, and the desired gene is exogenous. In an aspect, the control sequence is endogenous, and the desired gene is endogenous, but a series of sequence comprising the control sequence and the desired gene is endogenous or exogenous as a whole. Examples of the control sequence include a variety of known control sequences such as an enhancer and a promoter.

The chromosomal region defined in (ii) above comprises an HLA-similar sequence-cluster region comprising HLA-C, HLA-B, MICA, and MICB. In an aspect, the deletion is of a portion, or preferably the whole, of the HLA-similar sequence-cluster region, where the portion is a region comprising at least one HLA and HLA-similar sequence. For example, in an aspect, the deletion is of a region comprising one or more, preferably all, selected from the group consisting of HLA-C, HLA-B, MICA, and MICB.

The chromosomal region defined in (iii) above comprises an HLA-similar sequence-cluster region comprising HLA-DRB5, HLA-DRB1, HLA-DQA1, HLA-DQB1, HLA-DQA2, HLA-DQB2, and HLA-DOB. In an aspect, the deletion is of a portion, or preferably the whole, of the HLA-similar sequence-cluster region, where the portion is a region comprising at least one HLA and HLA-similar sequence. For example, in an aspect, the deletion is of a region comprising one or more, preferably all, selected from the group consisting of HLA-DRB5, HLA-DRB1, HLA-DQA1, HLA-DQB1, HLA-DQA2, HLA-DQB2, and HLA-DOB.

The chromosomal region defined in (iv) above comprises an HLA-similar sequence-cluster region comprising HLA-DOA, HLA-DPA1, and HLA-DPB1. In an aspect, the deletion is of a portion, or preferably the whole, of the HLA-similar sequence-cluster region, where the portion is a region comprising at least one HLA and HLA-similar sequence. For example, in an aspect, the deletion is of a region comprising one or more, preferably all, selected from the group consisting of HLA-DOA, HLA-DPA1, and HLA-DPB1.

In view of the gist of the present invention, the deletion of the region defined in (i) above can be a deletion of all other regions except the region comprising only one of HLA and HLA-similar sequences in the HLA-similar sequence-cluster region.

In an aspect, the deletion of the region defined in (ii) above can be a deletion of all other regions except the region comprising only one of HLA and HLA-similar sequences in the HLA-similar sequence-cluster region.

In an aspect, the deletion of the region defined in (iii) above can be a deletion of all other regions except the region comprising only one of HLA and HLA-similar sequences in the HLA-similar sequence-cluster region.

In an aspect, the deletion of the region defined in (iv) above can be a deletion of all other regions except the region comprising only one of HLA and HLA-similar sequences in the HLA-similar sequence-cluster region.

In an aspect, the deletion is of the region defined in (i) and no other region of (i) to (iv) lacks a portion or the whole of the HLA-similar sequence-cluster region. In an aspect, the deletion is of the region defined in (ii) and no other region of (i) to (iv) lacks a portion or the whole of the HLA-similar sequence-cluster region. In an aspect, the deletion is of the region defined in (iii) and no other region of (i) to (iv) lacks a portion or the whole of the HLA-similar sequence-cluster region. In an aspect, the deletion is of the region defined in (iv) and no other region of (i) to (iv) lacks a portion or the whole of the HLA-similar sequence-cluster region.

In an aspect, the deletion is of the region defined in each of (i) to (iv).

In an aspect, one or more genes may be inserted (knocked in) in the region with a deletion. The inserted gene can be a gene of interest with a selective marker (e.g., a drug resistance marker and a visible marker) and physiological function. The inserted gene is preferably operably linked to a control sequence. In a preferred aspect, the position at which the gene is inserted is any of (i) to (iv) above. In a preferred aspect, the position at which the gene is inserted is none of (i) to (iv) above. In a preferred aspect, the position at which the gene is inserted is within the region of (ii) above. In a preferred region, the region of (ii) above has a deletion (preferably the deletion as defined above) and an insertion of one or more genes above. In an aspect, the inserted gene has no insulator either upstream or downstream or both. The insulator is, for example, a cis-regulatory element having an effect of preventing an enhancer at a distant position from acting on a promoter or preventing silencing of euchromatin due to expansion of adjacent heterochromatin. Examples of the insulator include a CTCF insulator, a gypsy insulator, and a β-globin insulator. The insulator has a length of about 200 to 1000 bp. The insulator can inhibit, for example, PCR amplification across insulators. In an aspect, the region of (ii) above has a deletion (preferably the deletion as defined above) and an insertion of one or more genes above, and the one or more genes have no insulator on either the centromere side or the telomere side (or upstream and downstream) or both sides. In an aspect, the region with a deletion has no insertion of a new sequence. In an aspect, the region with a deletion has no recognition sequence of site-specific recombinase such as loxP or FRT (flippase recognition target) and variants thereof.

In an aspect, the deletion, particularly the deletion of the region defined in (i) above can be a deletion of a portion, or preferably the whole, of a series of regions comprising HLA-F, HLA-G, and HLA-A (e.g., a chromosomal region corresponding to chr6:29,723,464-29,945,455, preferably chr6:29,723,464-30,010,618 of the hg38 genomic sequence). In an aspect, the deletion, particularly the deletion of the region defined in (i) above, is of a region having one end in a specific sequence (first sequence) in chr6:29,701,000-29,723,464 (e.g., Chr6:29,709,000-29,711,000) chromosomal region and the other end in a specific sequence (second sequence) in chr6:29,945,455-30,030,000 (e.g., Chr6:30,020,000-30,020,000 chromosomal region and chr6:30,021,500-30,022,800) chromosomal region. Since the first sequence and the second sequence comprise a unique sequence in the genome, the upstream homology arm (e.g., telomeric homology arm) and the downstream homology arm (e.g., centromeric homology arm) in genome engineering by homologous recombination using donor DNA described below can be targeted to selectively bind to the unique sequence to achieve the genome engineering as intended. The deletion is a deletion of about 300 kb, and the following method can be preferably used as a technique for efficiently generating such a giant deletion.

In an aspect, the deletion, particularly the deletion of the region defined in (ii) above is of a portion, or preferably the whole, of a series of regions comprising HLA-C and HLA-B (e.g., a chromosomal region corresponding to chr6:31,269,169-31,357,158, preferably chr6:31,269,169-31,463,338 chromosomal region of the hg38 genomic sequence). In an aspect, the deletion, particularly the deletion of the region defined in (ii) above, is of a region having one end in a specific sequence (third sequence) in chr6:31,166,000-31,269,169 (e.g., Chr6:31,174,000-31,177,000) chromosomal region and the other end in a specific sequence (fourth sequence) in chr6:31,357,158-31,544,000 (e.g., Chr6:31,534,000-31,538,000) chromosomal region. Since the third sequence and the fourth sequence comprise a unique sequence (in other words, a specific sequence having no other similar sequence) in the genome, the upstream homology arm (e.g., telomeric homology arm) and the downstream homology arm (e.g., centromeric homology arm) in genome engineering by homologous recombination using donor DNA described below can be targeted to selectively bind to the unique sequence to achieve the genome engineering as intended. The deletion is a deletion of about 360 kb, and the following method can be preferably used as a technique for efficiently generating such a giant deletion.

In an aspect, the deletion, particularly the deletion of the region defined in (iii) above is of a portion, or preferably the whole, of a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence). In an aspect, the deletion, particularly the deletion of the region defined in (iii) above, is of a region having one end in a specific sequence (fifth sequence) in chr6:32,416,000-33,445,000, for example, chr6:32,420,000-33,445,000, for example, chr6:32,429,000-33,445,000 (e.g., Chr6:32,446,000-32,448,500) chromosomal region and the other end in a specific sequence (sixth sequence) in chr6:32,439,951-32,831,000 (e.g., Chr6:32,820,500-32,822,500) chromosomal region. Since the fifth sequence and the sixth sequence comprise a unique sequence (in other words, a specific sequence having no other similar sequence) in the genome, the upstream homology arm (e.g., telomeric homology arm) and the downstream homology arm (e.g., centromeric homology arm) in genome engineering by homologous recombination using donor DNA described below can be targeted to selectively bind to the unique sequence to achieve the genome engineering as intended. The deletion is a deletion of about 380 kb, and the following method can be preferably used as a technique for efficiently generating such a giant deletion.

In an aspect, the deletion, particularly the deletion of the region defined in (iv) above can be a deletion of a portion or the whole of a series of regions comprising HLA-DOA, HLA-DPA1, and HLA-DPB1 (e.g., a chromosomal region corresponding to chr6:33,006,838-33,086,238, preferably chr6:33,006,838-33,131,199 chromosomal region of the hg38 genomic sequence). In an aspect, the deletion, particularly the deletion of the region defined in (iv) above, is of a region having one end in a specific sequence (seventh sequence) in chr6: 32,924,000 to 33,006,838 (e.g., Chr6:32,999,500-33,002,500) chromosomal region and the other end in a specific sequence (eighth sequence) in chr6:33,086,238-33,165,000 (e.g., Chr6:33,147,500- 33,150,500) chromosomal region. Since the seventh sequence and the eighth sequence comprise a unique sequence (in other words, a specific sequence having no other similar sequence) in the genome, the upstream homology arm (e.g., telomeric homology arm) and the downstream homology arm (e.g., centromeric homology arm) in genome engineering by homologous recombination using donor DNA described below can be targeted to selectively bind to the unique sequence to achieve the genome engineering as intended. The deletion is a deletion of about 150 kb, and the following method can be preferably used as a technique for efficiently generating such a giant deletion.

In an aspect, the cell with a deletion comprises genomic DNA with a deletion, wherein the deletion is of a region comprising a portion or the whole of an HLA-similar sequence-cluster region located in each of all chromosomal regions selected from the group consisting of chromosomal regions defined in the following (i) to (iv):
(i) a series of regions comprising HLA-F, HLA-G, and HLA-A (e.g., a chromosomal region corresponding to chr6:29,723,464-29,945,455, preferably chr6:29,723,464-30,010,618 of the hg38 genomic sequence),
(ii) a series of regions comprising HLA-C and HLA-B (e.g., a chromosomal region corresponding to chr6:31,269,169-31,357,158, preferably chr6:31,269,169-31,463,338 chromosomal region of the hg38 genomic sequence),
(iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence), and
(iv) a series of regions comprising HLA-DOA, HLA-DPA1, and HLA-DPB1 (e.g., a chromosomal region corresponding to chr6:33,006,838-33,086,238, preferably chr6:33,006,838-33,131,199 chromosomal region of the hg38 genomic sequence),
wherein
the HLA-similar sequence-cluster region comprises all of HLA-encoding genes and HLA-similar sequences contained in each of the chromosomal regions, and
the portion comprises at least a stretch of region, the stretch of region comprising 50% or more, 60% or more, 70% or more, 80% or more, 90% or more, or 100% of genes selected from the group consisting of the HLA-encoding genes and HLA-similar sequences contained in each of the chromosomal regions.

In an aspect, the cell with a deletion comprises genomic DNA with a deletion, wherein the deletion is of all chromosomal regions selected from the group consisting of chromosomal regions defined in the following (i) to (iv):
(i) a series of regions comprising HLA-F, HLA-G, and HLA-A (e.g., a chromosomal region corresponding to chr6:29,723,464-29,945,455, preferably chr6:29,723,464-30,010,618 of the hg38 genomic sequence),
(ii) a series of regions comprising HLA-C and HLA-B (e.g., a chromosomal region corresponding to chr6:31,269,169-31,357,158, preferably chr6:31,269,169-31,463,338 chromosomal region of the hg38 genomic sequence),
(iii) a series of regions comprising HLA-DRA, HLA-DRB5, and HLA-DRB1 (e.g., a chromosomal region corresponding to chr6:32,445,000-32,816,951, preferably chr6:32,439,951-32,817,002 chromosomal region of the hg38 genomic sequence), and
(iv) a series of regions comprising HLA-DOA, HLA-DPA1, and HLA-DPB1 (e.g., a chromosomal region corresponding to chr6:33,006,838-33,086,238, preferably chr6:33,006,838-33,131,199 chromosomal region of the hg38 genomic sequence),
preferably a further deletion of a series of regions comprising HLA-E, and further deletions of a series of regions comprising HLA-DMB, a series of regions comprising MICA and MICB, and a series of regions comprising HLA-DMA.

In an aspect, when the region (i) has a deletion, the region (i) with a deletion has no repetitive sequence within the chromosomal region. In an aspect, when the region (ii) has a deletion, the region (ii) with a deletion has no repetitive sequence within the chromosomal region. In an aspect, when the region (iii) has a deletion, the region (iii) with a deletion has no repetitive sequence within the chromosomal region. In an aspect, when the region (iv) has a deletion, the region (iv) with a deletion has no repetitive sequence within the chromosomal region. The repetitive sequence is, for example, a stretch of sequence that makes sequencing difficult. The repetitive sequence is, for example, a stretch of sequence that induces an off-target to a homology arm of the donor DNA. The repetitive sequence comprises, for example, repetition of a sequence of 0.5 kb to 2 kb.

In an aspect, the human cells with a deletion have a gene encoding functional β2 microglobulin (B2M). In an aspect, the human cells with a deletion have a gene encoding functional class II major histocompatibility complex transactivator (CIITA). In an aspect, the human cells with a deletion have a gene encoding functional B2M and a gene encoding functional CIITA. As a result, the deletion of B2M and CIITA can prevent unexpected functional engineering that can occur in the cells. Nevertheless, in an aspect, the human cell with a deletion does not substantially express HLA class I and/or HLA class II on the cell surface.

In an aspect, the deletion can be 100 kb or more, 150 kb or more, 200 kb or more, 250 kb or more, 300 kb or more, 350 kb or more, or 400 kb or more in size. In an aspect, the deletion can be 400 kb or less, 350 kb or less, 300 kb or less, 250 kb or less, 200 kb or less, 150 kb or less, or 100 kb or less in size. In an aspect, the deletion can be 100 kb to 400 kb in size.

In an aspect, the cell can be a complete somatic cell but may also be a precursor cell for further introduction of engineering.

Examples of cells that can be used as useful cells themselves but are also useful as precursor cells for further introduction of engineering will be given below as Cell Examples 1 to 4. Cell Examples 1 to 4 can eliminate the problem that the sequencing of the genome or the targeting of the gene is hindered by the presence of repetitive sequences because at least one of the HLA-similar sequence-cluster regions is deleted. As a result, engineering is facilitated, and sequencing after engineering is facilitated, thereby enabling more reliable application of gene engineering.

### Cell Example 1

In an aspect, the cell has a deletion in (i) above. In a preferred aspect, the deletion can be a deletion of the whole HLA-similar sequence-cluster region. In an aspect, the deletion in (i) above can be a deletion due to replacement by DNA containing one or more desired genes or insertions thereof. In an aspect, the desired genes can be selective marker genes. The selective marker genes preferably include positive selective marker genes, more preferably both positive and negative selective marker genes, or marker genes that can be used for both positive and negative selection. In an aspect, the cell has a deletion in (i) above and no deletion in (ii) to (iv) above. Cell Example 1, which lacks HLA-A and F and is itself a useful cell, can be used, for example, to produce Cell Examples 2 to 4.

### Cell Example 2

In an aspect, the cell has a deletion in (i) above and the desired gene or insertion thereof operably linked to a control sequence. In a preferred aspect, the deletion in (i) above is a deletion comprising the whole HLA-similar sequence-cluster region. In an aspect, the deletion in (i) above can be a deletion due to replacement by DNA containing one or more desired genes or insertions thereof. In an aspect, the desired genes can be molecules that suppress immunity against the cell with a deletion, for example, genes encoding immunosuppressive molecules. In an aspect, the region with a deletion comprises selective marker genes. The selective marker genes preferably include positive selective marker genes, more preferably both positive and negative selective marker genes, or marker genes that can be used for both positive and negative selection. In an aspect, the cell with a deletion has a desired gene in a region other than (i) above. Cell Example 2, which is itself a useful cell, can be used, for example, to produce Cell Examples 3 and 4.

### Cell Example 3

In an aspect, the cell has a deletion in (i) above, and (iii) and/or (iv) above, and the desired gene or insertion thereof operably linked to a control sequence. In a preferred aspect, the cell has a deletion in (i), (iii) and (iv) above and the desired gene or insertion thereof operably linked to a control sequence. In a preferred aspect, the deletion in each of (i), (iii), and (iv) above is a deletion comprising the whole HLA-similar sequence-cluster region within that region. In an aspect, the deletion in (i) above can be a deletion due to replacement by DNA containing one or more desired genes or insertions thereof. In an aspect, the desired genes can be molecules that suppress immunity against the cell with a deletion, for example, genes encoding immunosuppressive molecules. In an aspect, the cell has a deletion in (i) above, and (iii) and/or (iv) above, and any one or more or all of the deleted regions comprise selective marker genes. The selective marker genes preferably include positive selective marker genes, more preferably both positive and negative selective marker genes, or marker genes that can be used for both positive and negative selection. In a preferred aspect, none of (i), (iii) and (iv) above has the insertion of a desired gene, such as a marker gene or a new gene. In an aspect, the cell has no deletion in (ii) above. Cell Example 3, which is itself a useful cell, can be used, for example, to produce Cell Example 4.

### Cell Example 4

In an aspect, the cell has a deletion in all of (i) to (iv) above and the desired gene or insertion thereof operably linked to a control sequence. In a preferred aspect, the deletion in (i) above can be a deletion due to replacement by DNA containing one or more desired genes or insertions thereof. In an aspect, the desired genes can be molecules that suppress immunity against the cell with a deletion, for example, genes encoding immunosuppressive molecules. In a preferred aspect, the deletion in (ii) above can be a deletion due to replacement by DNA containing one or more desired genes. In a preferred aspect, the desired genes can be selective marker genes. The selective marker genes preferably include positive selective marker genes, more preferably both positive and negative selective marker genes, or marker genes that can be used for both positive and negative selection. In a preferred aspect, neither (iii) nor (iv) above has the insertion of a desired gene, such as a marker gene or a new gene. Cell Example 4, which is itself a useful cell, can be used, for example, to further replace a DNA portion containing one or more desired genes or insertions thereof in (ii) above with other DNA. As a result, for example, the marker gene can be removed, or another desired gene can be introduced after the marker gene has been removed. From the genome analysis, it can be expected that the region of (ii) above is a transcriptionally activated region, and the desired gene can be favorably expressed by introducing the desired gene. In a preferred aspect, the desired gene is inserted to the region of (ii) above in the absence of an insulator on one or both of the centromere side and the telomere side of the gene.

In an aspect, the cell with a deletion does not have at least HLA-A. In an aspect, the cell with a deletion does not have at least HLA-A and F. On the other hand, the cell with a deletion may have one or more or all selected from the group consisting of HLA-B, C, E, G, H, J, and W; MICA and MICB; and HLA-DRA, DRB1, DRB5, DRB6, DRB9, DQA1, DQA2, DQB1, DPA1, DPB1, DMB, DOA, and DOB.

Cell Examples 1 to 4 are described above. Thus, in a cell with an HLA deletion, the HLA region may be replaced with a marker gene or a desired gene (gene of interest). The marker gene and the gene of interest are operably linked to a control sequence.

In all aspects, in the cells with a deletion, the deletion occurs in two alleles, thereby deleting expression of the deleted gene.

In an aspect, the cell with a deletion can be an isolated cell or cell line.

In an aspect, the cell with a deletion can evade cytotoxicity by T cell natural killer cells (NK cells). In an aspect, the cell with a deletion can be administered to an allogeneic individual without inducing immune rejection against the cell. As a result, cells which can be preferably used for hematopoietic stem cell transplantation, organ transplantation, cellular transplantation, or tissue transplantation, for example, are provided.

In an aspect, the cell with a deletion can be frozen in a cell cryoprotectant. In an aspect, the cell cryoprotectant containing the cells with a deletion can be provided in a non-frozen state or, preferably, in a frozen state. The cell cryoprotectant containing the cells with a deletion in a frozen state (also referred to as a "freeze stock") can be used as a research cell bank (RCB), master cell bank (MCB), or working cell bank (WCB). Therefore, in the present invention, a research cell bank (RCB), master cell bank (MCB), or working cell bank (WCB) containing the freeze stock is provided. The genomic DNA with a deletion can be subjected to whole genome sequencing, and in an aspect, reference genome data is generated from the whole genome sequence. When the repetitive sequences in (i) to (iv) are removed in the above, the generation of this reference genome data becomes easier. After further genome engineering of the cells having the genomic DNA with a deletion, the engineering status of the engineered site can be confirmed based on the reference genome. As a result, the cells with a deletion can facilitate or ensure further genome engineering and the sequence of the resulting engineered genome can be easily determined.

Examples of the cells include pluripotent cells (e.g., pluripotent stem cells such as embryonic stem cells (ES cells) and induced pluripotent stem cells (iPS cells)), hematopoietic stem cells, hematopoietic progenitor cells, bone marrow cells, spleen cells, common myeloid progenitor cells, immune cells (e.g., T cells, B cells, NK cells, NKT cells, macrophages, monocytes, neutrophils, eosinophils, and basophils), erythrocytes, megakaryocytes, cardiac cells, cardiomyocytes, cardiac fibroblasts, pancreatic β cells, corneal cells (e.g., corneal epithelial cells and corneal endothelial cells), epidermal cells, dermal cells, adipocytes, chondrocytes, bone cells, osteoclasts, osteoblasts, mesenchymal stem cells (e.g., adipose-derived, bone marrow-derived, placenta-derived, and umbilical cord-derived), dental pulp cells, tendon cells, ligament cells, neural cells (e.g., pyramidal cells, astrocytes, and granule cells), glial cells, Purkinje cells, retinal ganglion cells, retinal cells, optic nerve cells, and neural stem cells. In a preferred aspect, the cell can be a primary cell. In a preferred aspect, the cell can be an immortalized cell or cell line.

The cells with a deletion may form a cell aggregate, such as an organoid. The cell with a deletion may form a cell sheet. The cell with a deletion may form the whole or a partial structure of an organ or tissue.

In an aspect, a composition (e.g., a pharmaceutical composition or a cellular preparation) comprising the cell with a deletion is provided. The composition can be used for medical applications. The composition, in addition to the cell, may further comprise pharmaceutically acceptable carriers and/or additives. Examples of the pharmaceutically acceptable carriers include water and saline. Examples of the pharmaceutically acceptable additives include salts, pH buffers, and isotonic agents. The composition can be serum free in a preferred aspect.

The method described below (e.g., UKiS; see International Publication No. WO 2021/206054) is beneficial as a method for efficiently introducing the same engineering to two or more alleles of a chromosomal genome at the same time, is suitable for, for example, creating a deletion of about 100 kb to 500 kb in size in a target chromosomal region in a sequence-specific manner, and can be preferably used for the production of the cells described above. This method can also be applied to haploid cell engineering. The method can also be applied to cells having only one allele of the HLA gene region on the genomic DNA.

In one embodiment, the present invention can be a method for preparing a cell in which two or more alleles in the chromosomal genome are engineered, comprising the steps of:
(a) introducing the following (i) and (ii) to a cell comprising two or more alleles to introduce a selective marker gene to each of the two or more alleles:
   (i) a genome engineering system comprising a sequence-specific nucleic acid cleaving molecule capable of targeting a target region in the two or more alleles in the chromosomal genome and cleaving the target region, or a polynucleotide encoding the sequence-specific nucleic acid cleaving molecule, and
   (ii) two or more donor DNAs for selective markers, each of which has an upstream homology arm having a nucleotide sequence homologously recombinable with an upstream nucleotide sequence of the target region and a downstream homology arm having a nucleotide sequence homologously recombinable with a downstream nucleotide sequence of the target region, and comprises a nucleotide sequence of the selective marker gene between the upstream homology arm and the downstream homology arm, the two or more donor DNAs for selective markers respectively having distinguishably different selective marker genes, wherein the selective marker gene is unique to each type of donor DNA for the selective marker, and the number of types of the donor DNAs for selective markers is equal to or more than the number of the alleles that are subject to genome engineering; and
(b) after the step (a), respectively homologously recombining the two or more alleles with different types of donor DNAs for selective markers so that the distinguishably different unique selective marker genes are respectively introduced in the two or more alleles, and selecting a cell expressing all the distinguishably different selective marker genes thus introduced (step for positive selection).

### (Step (a))

In the step (a), (i) and (ii) described above are introduced to a cell comprising the chromosome.

The cell for use in the genome engineering method of the present embodiment is not particularly limited and can be a cell having a monoploid or diploid or higher chromosomal genome. The cell may be a diploid, may be a triploid, or may be a tetraploid or higher. Examples of the cell include, but are not particularly limited to, cells of eukaryotes. The cell may be a plant cell, may be an animal cell, or may be a fungal cell. The animal cell is not particularly limited and may be any cell of humans, non-human mammals (e.g., non-human primates such as monkeys, and non-human mammals such as dogs, cats, cattle, horses, sheep, goats, llamas, and rodents), bird, reptiles, amphibians, fish, and other vertebrate animals.

The target region that is subject to genome engineering can be an arbitrary region, in the genome, having one or more alleles. The size of the target region is not particularly limited. The genome engineering method of the present embodiment can engineer a region having a size larger than ever. The target region may be, for example, 10 kbp or more. The target region may be, for example, 100 bp or more, 200 bp or more, 400 bp or more, 800 bp or more, 1 kbp or more, 2 kbp or more, 3 kbp or more, 4 kbp or more, 5 kbp or more, 8 kbp or more, 10 kbp or more, 20 kbp or more, 40 kbp or more, 80 kbp or more, 100 kbp or more, 200 kbp or more, 300 kbp, 400 kbp or more, 500 kbp or more, 600 kbp or more, 700 kbp or more, 800 kbp or more, 900 kbp or more, or 1 Mbp or more, or equal to or less than any one of the values described above. In an aspect, the engineered cell lacks the target region.

### <(i) Genome engineering system>

The "genome engineering system" means a molecular mechanism capable of engineering a desired target region. The genome engineering system comprises a sequence-specific nucleic acid cleaving molecule targeting a target region in the chromosomal genome, or a polynucleotide encoding the sequence-specific nucleic acid cleaving molecule.

The sequence-specific nucleic acid cleaving molecule is not particularly limited as long as the molecule has sequence-specific nucleic acid cleaving activity. The sequence-specific nucleic acid cleaving molecule may be a synthetic organic compound or may be a biopolymer compound such as a protein. Examples of the synthetic organic compound having sequence-specific nucleic acid cleaving activity include pyrrole-imidazole-polyamide. Examples of the protein having sequence-specific site cleaving activity include sequence-specific endonuclease.

The sequence-specific endonuclease is an enzyme that can cleave a nucleic acid at a predetermined sequence. The sequence-specific endonuclease can cleave doublestranded DNA at a predetermined sequence. The sequence-specific endonuclease is not particularly limited. Examples thereof include, but are not limited to, zinc finger nuclease (ZFN)), TALEN (transcription activator-like effector nuclease), and Cas protein.

ZFN is artificial nuclease containing a nucleic acid cleavage domain conjugated with a binding domain containing a zinc finger array. Examples of the cleavage domain include the cleavage domain of type II restriction enzyme FokI. Zinc finger nuclease capable of cleaving a target sequence can be designed by a method known in the art.

TALEN is artificial nuclease containing a DNA cleavage domain (e.g., a FokI domain) as well as the DNA binding domain of a transcription activator-like (TAL) effector. A TALE construct capable of cleaving a target sequence can be designed by a method known in the art (e.g., Zhang, Feng et. al. (2011) Nature Biotechnology 29 (2)).

In the case of using Cas protein as the sequence-specific nucleic acid cleaving molecule, the genome engineering system comprises a CRISPR/Cas system. Specifically, the genome engineering system preferably comprises Cas protein, and guide RNA having a nucleotide sequence homologous to a nucleotide sequence within the target region. The guide RNA can comprise, as a spacer sequence, a sequence homologous to a sequence (target sequence) within the target region. The guide RNA can bind to DNA within the target region and does not have to have a sequence completely identical to the target sequence. This binding can be formed under physiological conditions in the cell nucleus. The guide RNA can contain, for example, 0- to 3-base mismatches with respect to the target sequence. The number of the mismatches is preferably 0 to 2 bases, more preferably 0 to 1 bases, further preferably zero mismatch. The guide RNA can be designed on the basis of a method known in the art. The genome engineering system is preferably a CRISPR/Cas system and preferably comprises Cas protein and guide RNA. The Cas protein is preferably Cas9 protein.

The sequence-specific endonuclease may be introduced as a protein to the cell, or may be introduced as a polynucleotide encoding the sequence-specific endonuclease to the cell. For example, mRNA of the sequence-specific endonuclease may be introduced, or an expression vector of the sequence-specific endonuclease may be introduced. In the expression vector, a coding sequence (sequence-specific endonuclease gene) of the sequence-specific endonuclease is functionally linked to a promoter. The promoter is not particularly limited, and, for example, various pol II promoters can be used. Examples of the pol II promoter include, but are not particularly limited to, CMV promoter, EF1 promoter (EF1α promoter), SV40 promoter, MSCV promoter, hTERT promoter, β actin promoter, CAG promoter, and CBh promoter.

The promoter may be an inducible promoter. The inducible promoter is a promoter that can induce the expression of a polynucleotide functionally linked to this promoter in the presence of an inducer that drives the promoter. Examples of the inducible promoter include promoters, such as heat shock promoter, which induce gene expression by heating. Other examples of the inducible promoter include promoters for which the inducer that drives the promoter is a drug. Examples of such a drug-inducible promoter include Cumate operator sequences, λ operator sequences (e.g., 12 × λOp), and tetracycline-inducible promoter. Examples of the tetracycline-inducible promoter include promoters that drive gene expression in the presence of tetracycline or a derivative thereof (e.g., doxycycline), or reverse tetracycline-controlled transactivator (rtTA). Examples of the tetracycline-inducible promoter include TRE3G promoter.

An expression vector known in the art can be used without particular limitations. Examples of the expression vector include plasmid vectors and virus vectors. When the sequence-specific endonuclease is Cas protein, the expression vector may contain a coding sequence (Cas protein gene) of the Cas protein as well as a guide RNA coding sequence (guide RNA gene). In this case, it is preferred that the guide RNA coding sequence (guide RNA gene) should be functionally linked to pol III promoter. Examples of the pol III promoter include mouse and human U6-snRNA promoters, human H1-RNase P RNA promoter, and human valine-tRNA promoter.

### <(ii) Donor DNAs for selective markers>

The donor DNAs for selective markers are donor DNAs for knocking-in selective markers to target regions. Each of the donor DNAs for selective markers comprises the nucleotide sequences of one or more selective marker genes between an upstream homology arm having a nucleotide sequence homologous to an upstream nucleotide sequence adjacent to the target region and a downstream homology arm having a nucleotide sequence homologous to a downstream nucleotide sequence adjacent to the target region.

The donor DNAs for selective markers can have a length of, for example, but not particularly limited to, 1 kb or more, 2 kb or more, 3 kb or more, 4 kb or more, 5 kb or more, 6 kb or more, 7 kb or more, 8 kb or more, 9 kb or more, 9.5 kb or more, or 10 kb or more. The donor DNAs for selective markers can have a length of, for example, but not particularly limited to, 50 kb or less, 45 kb or less, 40 kb or less, 35 kb or less, 30 kb or less, 25 kb or less, 20 kb or less, 15 kb or less, 14 kb or less, 13 kb or less, 12 kb or less, 11 kb or less, 10 kb or less, 9 kb or less, 8 kb or less, 7 kb or less, 6 kb or less, 5 kb or less, or 4 kb or less.

The "selective marker" means a protein that permits cells to be selected on the basis of the presence or absence of its expression. The selective marker gene is a gene encoding the selective marker. In the case of selecting selective marker-expressing cells from a cell population in which the selective marker-expressing cells coexist with selective marker non-expressing cells, the selective marker is referred to as a "positive selective marker" or a "selective marker for positive selection". In the case of selecting selective marker non-expressing cells from a cell population in which selective marker-expressing cells coexist with the selective marker non-expressing cells, the selective marker is referred to as a "negative selective marker" or a "selective marker for negative selection". Different selective markers mean that the selective markers can be distinguished from each other (e.g., distinguishably different), and means that, for example, the selective markers can be distinguished from each other at least in terms of physiological properties such as drug resistance property or other physicochemical properties imparted to cells harboring the selective markers. Specifically, different selective markers mean that one of a plurality of different selective markers can be detected distinguishably from the other selective marker(s), or permits drug selection distinguishably from the other selective marker(s). The phrase "selective marker gene is unique to each type of donor DNA for the selective marker" means that the selective marker gene carried by one of the donor DNAs for selective markers is not contained in the other type(s) of donor DNA(s) for selective marker(s), or the selective marker gene, if contained in plural types of donor DNAs, is configured such that this selective marker gene is not expressed from two or more types of donor DNAs at the same time. In this respect, the two or more types of donor DNAs may be the same with each other except for their respective selective markers, or may differ in sequence and/or configuration other than the selective markers.

The positive selective marker is not particularly limited as long as a cell expressing the positive selective marker can be selected. Examples of the positive selective marker gene include drug resistance genes, fluorescent protein genes, luminescent enzyme genes, and chromogenic enzyme genes.

The negative selective marker is not particularly limited as long as a cell not expressing the negative selective marker can be selected. Examples of the negative selective marker gene include suicide genes (thymidine kinase, etc.), fluorescent protein genes, luminescent enzyme genes, and chromogenic enzyme genes. When the negative selective marker gene is a gene that has negative influence on the survival of cells (e.g., a suicide gene), the negative selective marker gene can be functionally linked to an inducible promoter. The negative selective marker gene thus functionally linked to the inducible promoter can be expressed only when the removal of cells having the negative selective marker gene is desired. The negative selective marker gene, for example, an optically detectable (e.g., fluorescent, luminescent, and chromogenic) marker gene (visible marker gene), may be constitutively expressed because of having little negative influence on the survival of cells.

Examples of the drug resistance gene include, but are not limited to, puromycin resistance gene, blasticidin resistance gene, geneticin resistance gene, neomycin resistance gene, tetracycline resistance gene, kanamycin resistance gene, zeocin resistance gene, hygromycin resistance gene, and chloramphenicol resistance gene.

Examples of the fluorescent protein gene include, but are not limited to, green fluorescent protein (GFP) gene, yellow fluorescent protein (YFP) gene, and red fluorescent protein (RFP) gene.

Examples of the luminescent enzyme gene include, but are not limited to, luciferase gene.

Examples of the chromogenic enzyme gene include, but are not limited to, β galactosidase gene, β glucuronidase gene, and alkaline phosphatase gene.

Examples of the suicide gene include, but are not limited to, herpes simplex virus thymidine kinase (HSV-TK) and inducible caspase 9.

The selective marker genes carried by the donor DNAs for selective markers are preferably positive selective marker genes. Specifically, cells expressing the selective markers can be selected as cells in which the selective marker genes are knocked in.

The upstream homology arm has a nucleotide sequence homologously recombinable with an upstream nucleotide sequence of the target region in the genome to be engineered, and has, for example, a nucleotide sequence homologous to an upstream nucleotide sequence adjacent to the target sequence. The downstream homology arm has a nucleotide sequence homologously recombinable with an upstream nucleotide sequence of the target region in the genome to be engineered, and has, for example, a nucleotide sequence homologous to a downstream nucleotide sequence adjacent to the target sequence. The upstream homology arm and the downstream homology arm are not particularly limited by their lengths and sequences as long as these homology arms are homologously recombinable with the neighboring regions of the target region. The upstream homology arm and the downstream homology arm are not necessarily required to be identical to the upstream sequence and the downstream sequence, respectively, of the target region as long as these homology arms are homologously recombinable therewith. For example, the upstream homology arm can be a sequence having 90% or more sequence identity (homology) to the upstream nucleotide sequence adjacent to the target region and preferably has 92% or more, 93% or more, 94% or more, 95% or more, 96% or more, 97% or more, 98% or more, or 99% or more sequence identity thereto. For example, the downstream homology arm can be a sequence having 90% or more sequence identity (homology) to the downstream nucleotide sequence adjacent to the target region and preferably has 92% or more, 93% or more, 94% or more, 95% or more, 96% or more, 97% or more, 98% or more, or 99% or more sequence identity thereto. The engineering efficiency of the alleles can be enhanced provided that at least any one of the upstream homology arm and the downstream homology arm is located closer to a cleavage location in the target region. In this context, the term "close" can mean that the distance between two sequences is 100 bp or less, 50 bp or less, 40 bp or less, 30 bp or less, 20 bp or less, or 10 bp or less.

In the donor DNAs for selective markers, the selective marker gene is positioned between the upstream homology arm and the downstream homology arm. As a result, in the case of introducing the donor DNAs for selective markers, together with the genome engineering system (i), to the cell, the selective marker gene is introduced to the target region by HDR (this is referred to gene knock-out when the gene is disrupted, and referred to as gene knock-in when the desired gene is introduced; a gene may be knocked out while another gene can be knocked in).

It is preferred that the selective marker gene should be functionally linked to a promoter so as to be expressed under the control of an appropriate promoter. The promoter can be appropriately selected according to the type of the cell to which the donor DNAs are to be introduced. Examples of the promoter include SRα promoter, SV40 early promoter, retrovirus LTR, CMV (cytomegalovirus) promoter, RSV (Rous sarcoma virus) promoter, HSV-TK (herpes simplex virus thymidine kinase) promoter, EF1α promoter, metallothionein promoter, and heat shock promoter. Each of the donor DNAs for selective markers may have, for example, an arbitrary control sequence such as an enhancer, a poly-A addition signal, or a terminator.

Each of the donor DNAs for selective markers may have an insulator sequence. The "insulator" refers to a sequence that ensures or enhances the independence of transcriptional regulation of DNA flanked by its regions by blocking or mitigating the influence of adjacent chromosomal environments. The insulator is defined by an enhancer blocking effect (effect of blocking the influence of an enhancer on promoter activity by the insulator inserted between the enhancer and the promoter), and a suppressive effect on a position effect (effect of preventing the expression of a transgene from being influenced by the position of the insert in the genome, by the insulators flanking both sides of the transgene). Each of the donor DNAs for selective markers may have an insulator sequence between the upstream arm and the selective marker gene (or between the upstream arm and a promoter that controls the selective marker gene). Each of the donor DNAs for selective markers may have an insulator sequence between the downstream arm and the selective marker gene.

The donor DNAs for selective markers may be linear or may be cyclic and are preferably cyclic. Preferably, the donor DNAs for selective markers are plasmids. Each of the donor DNAs for selective markers may comprise an arbitrary sequence in addition to the sequences described above. For example, a spacer sequence may be contained wholly or partially between the respective sequences of the upstream homology arm, the insulator, the selective marker gene, and the downstream homology arm.

In the step (a), the donor DNAs for selective markers, the number of types of which is equal to or more than the number of the alleles that are subject to genome engineering, are introduced to the cell. Such different types of donor DNAs for selective markers respectively have different (distinguishable) types of selective marker genes. In an aspect, the different types of donor DNAs for selective markers do not have completely identical selective marker genes or sets thereof. In short, the first type of donor DNA for a selective marker has the first type of selective marker gene, and the second type of donor DNA for a selective marker has the second type of selective marker gene. The third type of donor DNA for a selective marker has the third type of selective marker gene. The same holds true for subsequent types of donor DNAs for selective markers. When the number of the alleles that are subject to genome engineering is 2, the types of the donor DNAs for selective markers are two or more types. When the number of the alleles that are subject to genome engineering is 3, the types of the donor DNAs for selective markers are three or more types. In an aspect, one donor DNA for selective markers may have two or more types of different (distinguishable) selective markers (in this case as well, the different types of donor DNAs for selective markers must respectively have different (distinguishable) types of (e.g., unique) selective marker genes). In an aspect, the donor DNAs for selective markers do not have a recombination sequence of site-specific recombinase (e.g., a loxP sequence and its variant which are recombined by Cre recombinase). In an aspect, the method of the present invention employs neither site-specific recombinase nor its recombination sequence (e.g., a loxP sequence and its variant which are recombined by Cre recombinase). When site-specific recombinase is used, one recombination sequence of the site-specific recombinase usually remains in the genome after editing. By contrast, in an aspect, the engineered genome of the cell obtained by the method of the present invention does not have a recombination sequence (which is a foreign sequence) of site-specific recombinase.

The number of types of the donor DNAs for selective markers can be equal to or more than the number of the alleles that are subject to genome engineering, and the upper limit is not particularly limited. Use of the donor DNAs for selective markers, the number of types of which is equal to or more than the number of the alleles that are subject to genome engineering, enables the two or more alleles to be stably engineered. The number of types of the donor DNAs for selective markers is preferably equal to or more by approximately 1 or 2 than the number of the alleles that are subject to genome engineering, more preferably equal to the number of the alleles that are subject to genome engineering, from the viewpoint of a selection operation in the step (b) mentioned later.

A method for introducing (i) and (ii) described above to the cell is not particularly limited, and a method known in the art can be used without particular limitations. Examples of the method for introducing (i) and (ii) to the cell include, but are not limited to, viral infection method, lipofection method, microinjection method, calcium phosphate method, DEAE-dextran method, electroporation method, and particle gun method. As a result of introducing (i) and (ii) described above to the cell, DNA of the target region is cleaved by the sequence-specific nucleic acid cleaving molecule of (i), followed by the knock-in of each of the selective markers of the donor DNAs (ii) for selective markers in the target region by HDR. In this respect, when the two or more donor DNAs for selective markers have the same upstream homology arms and downstream homology arms, the donor DNAs can be randomly knocked in the two or more alleles of the target regions. However, the two or more donor DNAs for selective markers do not have to have completely identical nucleotide sequences of the homology arms because the donor DNAs can respectively engineer the two or more alleles as long as the homology arms have nucleotide sequences homologously recombinable with upstream sequences and downstream sequences of the respective target regions of the two or more alleles. In an aspect, the nucleotide sequences of the upstream and downstream homology arms in the two or more donor DNAs for selective markers may have nucleotide sequences having higher identity to the upstream sequences and downstream sequences of the target regions of their respective alleles (e.g., the nucleotide sequences may be optimized in that way).

In an aspect, each of the donor DNAs for selective markers has an upstream homology arm and a downstream homology arm and has a selective marker gene between the upstream homology arm and the downstream homology arm, and preferably, may further have a target sequence of endonuclease (nucleotide sequence-specific nucleic acid cleaving molecule), such as a cleavage site of meganuclease. In a preferred aspect of this aspect, the selective markers include selective marker genes for positive selections and a marker gene for negative selection. In another preferred aspect, the selective markers include selective markers for positive selections but may not include a negative selective marker gene aside therefrom. In a preferred aspect, the selective marker gene for positive selection may also be used for negative selection. Examples of such a marker gene include visible marker genes.

A set of two or more donor DNAs for selective markers is a combination of the donor DNAs for selective markers described above, and these donor DNAs respectively have selective marker genes for positive selections distinguishable from each other. In the set, each of the donor DNAs may further have a target sequence of endonuclease (nucleotide sequence-specific nucleic acid cleaving molecule), such as a cleavage site of meganuclease. Their respective target sequences may be different from each other and are preferably identical (or can be cleaved by the same nucleotide sequence-specific nucleic acid cleaving molecule). The length of the donor DNAs for selective markers is as described above and can be, for example, 5 kbp or more, 8 kbp or more, or 10 kbp or more.

### (Step (b))

After the step (a), the step (b) is performed. In the step (b), a cell in which distinguishably different selective marker genes or a combination thereof are respectively introduced in the two or more alleles is selected on the basis of the expression of the distinguishably different selective marker genes. More specifically, in the step (b), the two or more alleles are respectively homologously recombined with different types of donor DNAs for selective markers so that the distinguishably different unique selective marker genes are respectively introduced in the two or more alleles, and a cell expressing all the distinguishably different selective marker genes thus introduced is selected. In an aspect, in the step (b), a cell in which these alleles are engineered by the introduction of the donor DNAs for different selective markers is selected on the basis of the expression of all the selective marker genes, integrated in the chromosomal genome, as the selective marker genes carried by the two or more donor DNAs for selective markers. In an aspect, in the step (b), a cell is selected on the basis of all the selective marker genes carried by the two or more donor DNAs for selective markers. In an aspect, in the step (b), a cell in which these alleles are engineered by the introduction of the donor DNAs for distinguishable selective markers is selected on the basis of the expression of all the selective marker genes (marker genes for positive selections), integrated in the chromosomal genome, as the selective marker genes carried by the two or more donor DNAs for selective markers. In an aspect, the alleles in the cell obtained in the step (b) respectively have different marker genes for positive selections. In an aspect, the alleles in the cell obtained in the step (b) may respectively common marker genes for positive selections. In an aspect, in the step (b), single-cell cloning is not performed {however, single-cell cloning to be performed after selection of the cell in which the two or more alleles are engineered in the step (b) may or may not be included in the present invention}. In an aspect, in the step (b), the selection of the cell is performed on the basis of the expression of a plurality of distinguishable marker genes for positive selections respectively introduced in the alleles. In an aspect, the step (b) is not performed by a method of estimating the number of engineered alleles on the basis of the expression strength of a single selective marker gene (e.g., the expression strength or fluorescence intensity of a fluorescent protein). This is because, in the case of selecting a cell by a method of estimating the number of engineered alleles on the basis of the expression strength of a single selective marker gene, a gene expression level varies among cells so that the cell in which the two or more alleles are engineered is difficult to completely separate from cells in which one allele is engineered; thus the step (b) requires single-cell cloning.

In the step (b), the selection of the cell can be appropriately performed according to the types of the selective marker genes used in the step (a). In this respect, the cell is selected on the basis of the expression of all the selective marker genes used in the step (a).

When the selective marker genes are, for example, positive selective marker genes, a cell expressing all the selective marker genes to be integrated (or integrated) in the chromosomal genome to be engineered can be selected. For example, a cell expressing the same number of positive selective markers as the number of the alleles to be engineered can be selected. When the positive selective marker genes are drug resistance genes, a cell expressing the positive selective markers can be selected by cell culture in a medium containing the drugs. When the positive selective marker genes are fluorescent protein genes, luminescent enzyme genes, or chromogenic enzyme genes, a cell expressing the positive selective markers can be selected by selecting a cell emitting fluorescences, luminescences, or colors ascribable to the fluorescent proteins, the luminescent enzymes, or the chromogenic enzymes. In this step, when the same number of donor DNAs for selective markers as the number of the alleles to be engineered is incorporated in the genome, this number of the alleles is engineered. In a n-ploid cell, the number of alleles to be engineered is n or less. When the donor DNAs for selective markers, the number of types of which is equal to or more than this number and equal to or less than n, are incorporated in the genome, at least the alleles to be engineered (which are the two or more alleles) are engineered. In an aspect, the number of the alleles to be engineered is n, and this number of types of the donor DNAs for selective markers is incorporated in the chromosomal genome; thus, all the alleles are engineered. In an aspect, since the donor DNAs for selective markers, the number of types of which is equal to or more than the number of the alleles to be engineered, are used in this step, the number of positive selective markers expressed by the cell means that this number of alleles is reliably engineered. In the step (b), the number of the alleles to be engineered is preferably equal to the number of types of the donor DNAs for selective markers from the viewpoint of enhancing the selection efficiency of the cell.

As described above, in the genome engineering method of the present embodiment, HDR can be induced using n types of donor DNAs for selective markers for engineering n alleles in a n-ploid cell, to efficiently obtain a cell in which all the alleles carried by the cell are engineered. Since such a cell in which all the alleles are engineered can be reliably obtained, a cell having an engineered target region can be efficiently obtained even if the target region has a large size (e.g., 10 kbp or more). Hence, large-scale genome engineering is also achieved.

In an aspect, in the step (b), an engineered cell can be selected, without cloning cells, from a pool containing cells obtained by the step (a). The omission of the cloning step can shorten a time required for the process. In an aspect, the pool may contain 10⁵ or more, 10⁶ or more, 10⁷ or more, or 10⁸ or more cells.

### (Optional steps)

The genome engineering method of the present embodiment may comprise optional steps in addition to the step (a) and the step (b). Examples of the optional steps include the following steps (c) and (d):
(c) after the step (b), introducing, to the cell, a donor DNA for recombination comprising a desired nucleotide sequence between an upstream homology arm having a nucleotide sequence homologous to an upstream nucleotide sequence adjacent to the target region and a downstream homology arm having a nucleotide sequence homologous to a downstream nucleotide sequence adjacent to the target region; and
(d) after the step (c), selecting a cell not expressing the negative selective marker.

In an aspect, the genome engineering method of the present embodiment may comprise optional steps in addition to the step (a) and the step (b). In an aspect, in the genome engineering method or the method for obtaining a cell having an engineered genome according to the present embodiment, each of the two or more donor DNAs for selective markers has a selective marker gene for positive selection, a marker gene for negative selection other therethan, and a target sequence between the upstream homology arm and the downstream homology arm, wherein in the case of using the selective marker gene both for positive selection and for negative selection, the selective marker gene for negative selection is optionally absent, and the method may further comprise the following steps (c) and (d):
(c) after the step (b), introducing the following (iii) and (iv) to the selected cell to introduce a donor DNA for recombination to each of the two or more alleles:
   (iii) a genome engineering system comprising a sequence-specific nucleic acid cleaving molecule capable of targeting the additional target sequence and cleaving the additional target sequence, or a polynucleotide encoding the sequence-specific nucleic acid cleaving molecule, and
   (iv) a donor DNA for recombination comprising a desired nucleotide sequence, the donor DNA for recombination having an upstream homology arm having a nucleotide sequence homologously recombinable with an upstream nucleotide sequence of the target region and a downstream homology arm having a nucleotide sequence homologously recombinable with a downstream nucleotide sequence of the target region {the donor DNA for recombination may comprise a desired nucleotide sequence between the upstream homology arm and the downstream homology arm or may not comprise any nucleotide sequence therebetween}; and
(d) after the step (c), selecting a cell not expressing the marker gene for negative selection (step for negative selection).

### <Step (c)>

After the step (b), the step (c) may be performed. In an aspect, in the step (c), a donor DNA for recombination comprising or not comprising a desired nucleotide sequence between an upstream homology arm and a downstream homology arm is introduced to the cell selected in the step (b). In an aspect, in the step (c), a donor DNA for recombination comprising a desired nucleotide sequence between an upstream homology arm having a nucleotide sequence homologous to an upstream nucleotide sequence adjacent to the target region and a downstream homology arm having a nucleotide sequence homologous to a downstream nucleotide sequence adjacent to the target region is introduced to the cell selected in the step (b).

### <<Donor DNA for recombination>>

The donor DNA for recombination may comprise a desired nucleotide sequence to be knocked in. The desired nucleotide sequence is not particularly limited. For example, in the case of performing genome engineering for the purpose of knocking out the function of a gene contained in the target region, a nucleotide sequence lacking a portion or the whole of the nucleotide sequence of the target region can be used as the desired nucleotide sequence. In the case of integrating a foreign gene into the target region, a nucleotide sequence including the gene can be used as the desired nucleotide sequence. The size of the desired nucleotide sequence is not particularly limited and can be an arbitrary size. The desired nucleotide sequence can be, for example, 10 bp or more, 20 bp or more, 40 bp or more, 80 bp or more, 200 bp or more, 400 bp or more, 800 bp or more, 1 kbp or more, 2 kbp or more, 3 kbp or more, 4 kbp or more, 5 kbp or more, 6 kbp or more, 7 kbp or more, 8 kbp or more, 9 kbp or more, 10 kbp or more, 15 kbp or more, 20 kbp or more, 40 kbp or more, 80 kbp or more, 100 kbp or more, or 200 kbp or more. In the method of the present embodiment, a cell in which the desired nucleotide sequence is knocked in, in the two or more alleles can be efficiently selected. Hence, for example, DNA having a size as large as 5 kbp or more, 8 kbp or more, or 10 kbp or more can be knocked in. The donor DNA for recombination may be shorter than, for example, the donor DNAs for selective markers in terms of length.

The upstream homology arm and the downstream homology arm of the donor DNA for recombination may be the same as or different from those of the donor DNAs for selective markers. For the sake of convenience, the upstream homology arm and the downstream homology arm contained in each of the donor DNAs for selective markers are also referred to as a "first upstream homology arm" and a "first downstream homology arm", and the upstream homology arm and the downstream homology arm contained in the donor DNA for recombination are also referred to as a "second upstream homology arm" and a "second downstream homology arm". The second upstream homology arm and the second downstream homology arm are not particularly limited by their lengths and sequences as long as these homology arms are homologously recombinable with the first upstream homology arm or a region upstream therefrom and are homologously recombinable with the first downstream homology arm or a region downstream therefrom, for example (in an aspect, the second upstream and downstream homology arms are not particularly limited by their lengths and sequences as long as these homology arms are homologously recombinable with neighboring regions of the target region). After recombination with the donor DNA for recombination, it is accepted that the nucleotide sequences of the donor DNAs for selective markers partially remain in the genome. Preferably, the nucleotide sequences of the donor DNAs for selective markers are completely removed from the genome by recombination with the donor DNA for recombination. Various genes loaded in the donor DNAs for selective markers are removed by recombination with the donor DNA for recombination. As a result, in an aspect, each of the two or more alleles in the cell can be replaced with the donor DNA for recombination. In an aspect, the donor DNA for recombination may have a desired nucleotide sequence. As a result, the cell in which the two or more alleles are engineered has the desired nucleotide sequence in the engineered alleles.

In the donor DNA for recombination, the desired nucleotide sequence is positioned between the second upstream homology arm and the second downstream homology arm. When the donor DNA for recombination comprises a foreign gene, it is preferred that the foreign gene should be functionally linked to a promoter. The donor DNA for recombination may have, for example, an arbitrary control sequence such as an enhancer, a poly-A addition signal, or a terminator. When the donor DNA for recombination comprises a foreign gene, the donor DNA for recombination may have insulator sequences upstream and downstream of the foreign gene. In an aspect, the donor DNA for recombination comprises a spacer sequence between the second upstream homology arm and the second downstream homology arm. In an aspect, the donor DNA for recombination does not permit selection of a cell that has undergone homologous recombination with the donor DNA for recombination, if at the time of removal of a cell having the negative selective marker gene carried by the donor DNAs for selective markers, a gene that is the same as (or cannot be distinguished from) the negative selective marker gene is expressed under conditions in which its toxicity is exerted. Thus, the donor DNA for recombination is configured such that at the time of removal of a cell having the negative selective marker gene carried by the donor DNAs for selective markers, a gene that is the same as (or cannot be distinguished from) the negative selective marker gene is not expressed under conditions in which its toxicity is exerted. For example, in an aspect, the donor DNA for recombination has neither a negative selective marker gene nor a second target sequence between the second upstream homology arm and the second downstream homology arm.

The donor DNA for recombination is preferably introduced, together with (i) described above, to the cell. As a result of introducing the donor DNA for recombination, together with (i) described above, to the cell, DNA of the target region is cleaved by the sequence-specific nucleic acid cleaving molecule of (i), followed by the knock-in of the desired nucleotide sequence of the donor DNA for recombination in the target region by HDR. Since the cell to which the donor DNA for recombination is to be introduced in this step is the cell selected in the step (b), each of the nucleotide sequences of the donor DNAs for selective markers is knocked in, in the target region. Hence, a target sequence of the genome engineering system (i) is a nucleotide sequence contained in the target region after knock-in of the donor DNAs for selective markers. For the sake of convenience, the target sequence of the genome engineering system in the step (a) is also referred to as a "first target sequence", and the target sequence of the genome engineering system in the step (c) is also referred to as a "second target sequence". An arbitrary sequence contained in the target region in the cell after the step (b) can be used as the second target sequence. In an aspect, the second target sequence in each of the donor DNAs for selective markers can be a sequence that is absent in the genome of the cell. In an aspect, the second target sequence in each of the donor DNAs for selective markers is a sequence that is absent in the genome of the cell and is a sequence different from the other sequences in the genome to an extent that the other sequences are not cleaved through off-target. In an aspect, the second target sequence in each of the donor DNAs for selective markers can be a cleavage site of meganuclease that is absent in the genome. In an aspect, the second target sequence is a region other than the negative selective marker gene in the step (d). As a matter of course, the donor DNA for recombination is configured such that homologous recombination with the donor DNA for recombination is not markedly inhibited. When the first target sequence remains in the target region in the cell after the step (b) or when the first target sequence is reintroduced by the donor DNAs for selective markers, the second target sequence may be the same as or different from the first target sequence.

The donor DNA for recombination may not comprise a nucleotide sequence between the upstream homology arm and the downstream homology arm and may comprise a nucleotide sequence of 10 bp or less, 20 bp or less, 30 bp or less, 40 bp or less, 50 bp or less, 60 bp or less, 70 bp or less, 80 bp or less, 90 bp or less, 100 bp or less, 200 bp or less, 300 bp or less, 400 bp or less, 500 bp or less, 600 bp or less, 700 bp or less, 800 bp or less, 900 bp or less, or 1 kbp or less between the upstream homology arm and the downstream homology arm. The donor DNA for recombination may comprise a nucleotide sequence of 1 kbp or more, 2 kbp or more, 3 kbp or more, 4 kbp or more, 5 kbp or more, 6 kbp or more, 7 kbp or more, 8 kbp or more, 9 kbp or more, or 10 kbp or more between the upstream homology arm and the downstream homology arm.

The donor DNA for recombination comprise one or more or all selected from the group consisting of a selective marker gene, a target sequence of site-specific recombinase, a gene encoding a factor having physiological activity, a gene encoding a factor having cytotoxicity, and a promoter sequence between the upstream homology arm and the downstream homology arm, or does not comprise one or more or all selected from this group therebetween.

In the step (c), the donor DNA for recombination is introduced to the cell selected in the step (b). In the cell selected in the step (b), each of the selective marker genes is knocked in, in the target region. The step (c) can be regarded as the step of removing the knocked-in selective marker gene in the target region or replacing the knocked-in selective marker gene with a desired nucleotide sequence.

### (Step (d))

After the step (c), the step (d) may be performed. In the step (d), a cell not expressing the negative selective marker is selected.

In the case of performing the step (d), donor DNAs for selective markers each comprising a positive selective marker gene and a negative selective marker gene can be used in the step (a). Specifically, each of the donor DNAs for selective markers for use in the step (a) can comprise a positive selective marker gene and a negative selective marker gene between the upstream homology arm and the downstream homology arm. The positional relationship between the positive selective marker gene and the negative selective marker gene is not particularly limited, and the positive selective marker gene may be located upstream of the negative selective marker gene, or vice versa. When each of the donor DNAs for selective markers has a positive selective marker gene and a negative selective marker gene, for example, a nucleotide sequence encoding a self-cleaving peptide, or an IRES (internal ribozyme entry site) sequence may intervene between the positive selective marker gene and the negative selective marker gene. The intervention of such a sequence allows the positive selective marker gene and the negative selective marker gene to be independently expressed from one promoter. Examples of 2A peptide include foot-and-mouth disease virus (FMDV)-derived 2A peptide (F2A), equine rhinitis A virus (ERAV)-derived 2A peptide (E2A), porcine teschovirus (PTV-1)-derived 2A peptide (P2A) and *Thosea asigna* virus (TaV)-derived 2A peptide (T2A).

Alternatively, the same selective marker gene may be used as a positive selective marker in the step (a) and used as a negative selective marker in the step (d). For example, when the selective marker genes are marker genes involved in color development (e.g., fluorescence or dyes) (visible marker genes) such as fluorescent protein genes, luminescent enzyme genes, or chromogenic enzyme genes, a cell emitting fluorescences, luminescences, or colors ascribable to the expression of the fluorescent proteins, the luminescent enzymes, or the chromogenic enzymes may be selected in the step (a) and a cell in which these fluorescences, luminescences, or colors have disappeared can be selected in the step (c). The case where the same selective marker gene serves both as a positive selective marker and as a negative selective marker is also encompassed by the case where each of the donor DNAs for selective markers has a positive selective marker as well as a negative selective marker.

The negative selective marker gene may be different or the same between or among the types of the donor DNAs for selective markers. Use of a common negative selective marker gene simplifies a cell selection operation in the step (d).

In the step (d), the selection of the cell can be appropriately performed according to the type of the negative selective marker gene used in the step (a). In this respect, a cell expressing none of the negative selective marker gene(s) used in the step (a) is selected.

For example, when the negative selective marker gene is a visible marker gene such as a fluorescent protein gene, a luminescent enzyme gene, or a chromogenic enzyme gene, a cell in which the visible marker such as fluorescence, luminescence or color has disappeared can be selected. When the negative selective marker gene is a suicide gene, a cell not expressing the negative selective marker can be selected by cell culture in a medium containing a drug that exerts toxicity by the expression of the suicide gene. In the case of using, for example, thymidine kinase gene, as the suicide gene, the cell can be cultured in a medium containing ganciclovir. The disappearance of the expression of the negative selective marker gene means that the negative selective marker gene integrated into the target region in the step (a) is replaced with the polynucleotide comprising the desired nucleotide sequence of the donor DNA for recombination. In this respect, the replacement with the polynucleotide is considered to occur for the whole nucleotide sequences knocked in, in the step (a). Hence, a cell in which each of the nucleotide sequences knocked in, in the step (a) is replaced with the desired nucleotide sequence of the donor DNA for recombination can be efficiently selected by selecting a cell in which the expression of the negative selective marker gene has disappeared. The negative selective marker gene such as a suicide gene may be functionally linked to an inducible promoter. A cell not expressing the negative selective marker can be selected by cell culture in the presence of a drug that drives the inducible promoter such that the negative selective marker gene is expressed under conditions in which its toxicity is exerted. In this case, the negative selective marker gene may be a gene encoding a cytotoxin (e.g., ricin and diphtheria toxin) which causes toxicity to cells by only its expression.

In an aspect, in the step (d), the cell in which the two or more alleles are engineered (cell in which the negative selective marker gene is absent) can be selected, without cloning cells, from a pool containing cells obtained by the step (c). In an aspect, the pool may contain 10⁵ or more, 10⁶ or more, 10⁷ or more, or 10⁸ or more cells.

As described above, the step (c) and the step (d) can be performed to efficiently obtain a cell in which all the alleles carried by the cell are engineered into a desired sequence. Since such a cell in which all the alleles are engineered can be reliably obtained, a cell having a knocked-in desired nucleotide sequence in the target region can be efficiently obtained even if the desired nucleotide sequence has a large size (e.g., 10 kbp or more). In an aspect, the step (c) and the step (d) are performed so that the target region is deleted in all the alleles carried by the cell and sequences upstream and downstream thereof (i.e., sequences that undergo homologous recombination with the upstream homology arm and the downstream homology arm, respectively) are seamlessly linked without one or more selected from the group consisting of base insertion, substitution, and deletion (e.g., without base insertion, substitution and deletion). In an aspect, in the resulting cell, the upstream and downstream nucleotide sequences flanking the deleted region are seamlessly linked.

The number of live cells may be small or no live cell may be obtained by the step (b). This indicates that a gene that influences cell proliferation or survival is contained in the target region removed from the genome by homologous recombination with the upstream homology arm and the downstream homology arm. Thus, whether or not a gene that influences cell proliferation or survival is contained in the target region can be examined. In this case, the gene that influences cell proliferation or survival can be identified by changing the design positions of the upstream homology arm and the downstream homology arm and thereby changing a gene to be eliminated from the genome by homologous recombination. Thus, in the present invention, a step (e) can be performed after the step (b). Specifically, the step (e) comprises, when the number of live cells is small or no live cell is obtained by the step (b), identifying a gene that influences cell proliferation or survival by narrowing the target region and decreasing the number of genes to be eliminated from the genome. Provided that the target region contains only one gene, this gene is found to be the gene that influences cell proliferation or survival. After identification of the gene that influences cell proliferation or survival, a step (f) can be performed. The step (f) comprises knocking in the identified gene that influences cell proliferation or survival to another region (e.g., a safe harbor region) of the genome to be engineered {a donor DNA for recombination may be used in the knock-in}. As a result, a region to be deleted by the method of the present invention can be expanded (the target region can be extended upstream and/or downstream). The number of live cells that is small can be confirmed by comparison with the number of cells obtained by carrying out the steps (a) and (b) for a region that does not influence cell survival or proliferation. In an aspect, a region that eliminates cell proliferation or survival may not be the target region in the step (a). The present invention includes, for example, knocking in a desired gene to the chromosomal region of (i) or a region other than (i) (e.g., a safe harbor region) in the genomic DNA of a cell with a deletion in the chromosomal region of (i). Such a cell has a deletion in the chromosomal region of (i) and a genome with genes encoding the desired gene in the chromosomal region of (i) or in a region other than (i) (e.g., the region of (ii) with a deletion and the safe harbor region).

### [Genome engineering kit]

In one embodiment, the present invention provides a genome engineering kit for engineering two or more alleles in the chromosomal genome. The genome engineering kit comprises the following (i) and (ii):
(i) a genome engineering system comprising a sequence-specific nucleic acid cleaving molecule targeting a target region in the chromosomal genome, or a polynucleotide encoding the sequence-specific nucleic acid cleaving molecule, and
(ii) two or more donor DNAs for selective markers, each of which comprises a nucleotide sequence of a selective marker gene between a downstream homology arm having a nucleotide sequence homologous to an upstream nucleotide sequence adjacent to the target region and a downstream homology arm having a nucleotide sequence homologous to a downstream nucleotide sequence adjacent to the target region, the two or more donor DNAs for selective markers respectively having different selective marker genes, wherein the number of types of the donor DNAs for selective markers is equal to or more than the number of the alleles that are subject to genome engineering.

In one embodiment, the present invention provides a genome engineering kit for engineering two or more alleles in the chromosomal genome, comprising the following (i) and (ii):
(i) a genome engineering system comprising a sequence-specific nucleic acid cleaving molecule capable of targeting a target region in the chromosomal genome and cleaving the target region, or a polynucleotide encoding the sequence-specific nucleic acid cleaving molecule, and
(ii) two or more donor DNAs for selective markers, each of which has an upstream homology arm having a nucleotide sequence homologously recombinable with an upstream nucleotide sequence of the target region and a downstream homology arm having a nucleotide sequence homologously recombinable with a downstream nucleotide sequence of the target region, and comprises a nucleotide sequence of a selective marker gene between the upstream homology arm and the downstream homology arm, the two or more donor DNAs for selective markers respectively having selective marker genes distinguishable from each other, wherein the number of types of the donor DNAs for selective markers is equal to or more than the number of the alleles that are subject to genome engineering. In this aspect, the selective marker gene may be unique to each type of donor DNA for the selective marker. The kit may be used in the method of the present invention. The kit may be used in the method for preparing a cell in which two or more alleles in the chromosomal genome are engineered.

(i) and (ii) contained in the kit of the present embodiment are the same as (i) and (ii) described in the section [Genome engineering method]. Use of the kit of the present embodiment enables the genome engineering method to be easily performed.

In one embodiment, the present invention provides a cell in which two or more alleles in the chromosomal genome are engineered, the cell having different (distinguishable) selective marker genes in the two or more alleles, respectively. In an aspect, the cell can be a cell of a single-celled organism. In an aspect, the cell can be an isolated cell. In an aspect, the cell can be a cell selected from the group consisting of pluripotent or multipotent cells and pluripotent stem cells (embryonic stem cells and induced pluripotent stem cells, etc.). In an aspect, the cell can be a tissue stem cell. In an aspect, the cell can be a somatic cell. In an aspect, the cell can be a germ-line cell (e.g., a germ cell). In an aspect, the cell can be a cell line. In an aspect, the cell can be an immortalized cell. In an aspect, the cell can be a cancer cell. In an aspect, the cell can be a non-cancer cell. In an aspect, the cell can be a cell of a patient with a disease. In an aspect, the cell can be a cell of a healthy individual. In an aspect, the cell can be a cell selected from the group consisting of animal cells (e.g., human cells), for example, insect cells (e.g., silkworm cells), HEK293 cells, HEK293T cells, Expi293F(TM) cells, FreeStyle(TM) 293F cells, Chinese hamster ovary cells (CHO cells), CHO-S cells, CHO-K1 cells, and ExpiCHO cells, and cells derived from these cells. In a preferred aspect, in the cell, all the alleles in target regions in the chromosomal genome are engineered, and the regions thus engineered respectively have different (distinguishable) selective marker genes.

In one embodiment, the present invention provides a method for culturing a cell in which two or more alleles in the chromosomal genome are engineered, the cell having different (distinguishable) selective marker genes in the two or more alleles, respectively. When the selective marker genes are drug resistance marker genes, the culture can be culture in the presence of respective drugs for the drug resistance marker genes. The culture can be performed under conditions suitable for the maintenance or proliferation of the cell.

In one embodiment, the present invention provides a non-human organism having the chromosomal genome in which two or more alleles are engineered, the non-human organism having different (distinguishable) selective marker genes in the two or more alleles, respectively. In an aspect, the cell can be a cell of a single-celled organism. In an aspect, the non-human organism can be an organism selected from yeasts (e.g., fission yeasts and budding yeasts, for example, yeasts of the genus *Saccharomyces* such as *Saccharomyces cerevisiae, Saccharomyces carlsbergensis, Saccharomyces fragilis,* and *Saccharomyces rouxii,* the genus *Candida* such as *Candida utilis* and *Candida tropicalis,* the genus *Pichia,* the genus *Kluyveromyces,* the genus *Yarrowia,* the genus *Hansenula,* and the genus *Endomyces.* In an aspect, the non-human organism can be a filamentous bacterium (e.g., *Aspergillus, Trichoderma, Humicola, Acremonium, Fusarium,* and *Penicillium* species). In an aspect, the non-human organism can be a multi-celled organism. In an aspect, the non-human organism can be a non-human animal. In an aspect, the non-human organism can be a plant. In a preferred aspect, in the non-human organism, all the alleles in target regions in the chromosomal genome are engineered, and the regions thus engineered respectively have different (distinguishable) selective marker genes.

In the cell, one or more desired genes such as the genes necessary for cell survival or proliferation may be contained or gathered in another region of the chromosomal genome. Another region can be, for example, a safe harbor region (e.g., an AAVS1 region). Another region can be, for example, the region (ii) with a deletion.

### Examples

### Example 1: Search results for homologous sequences

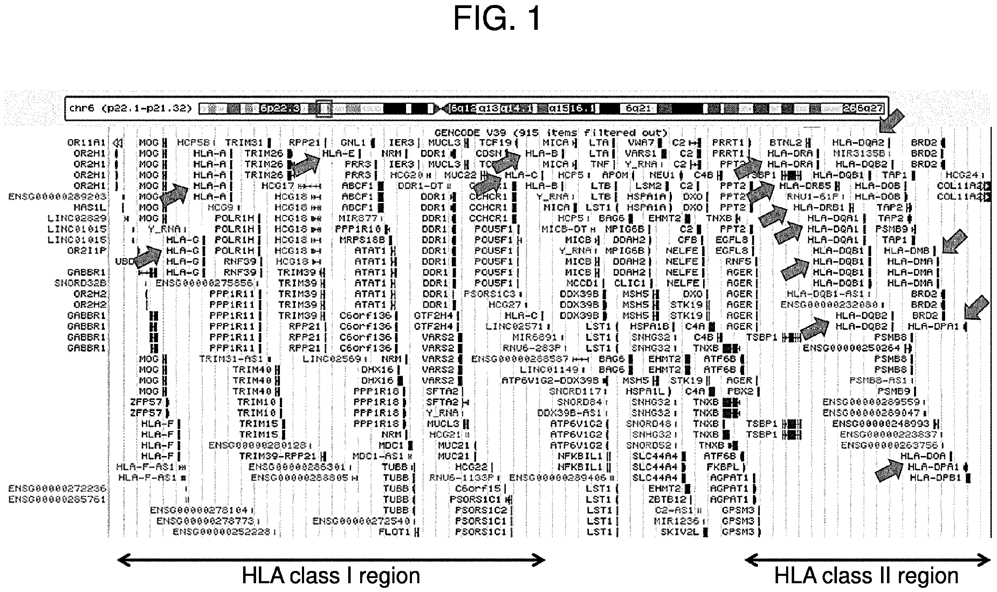

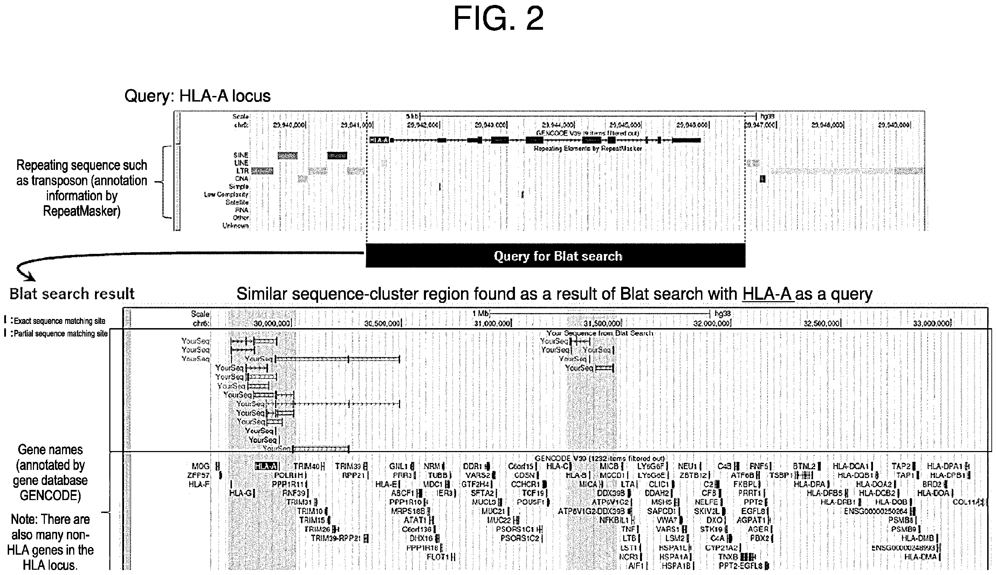

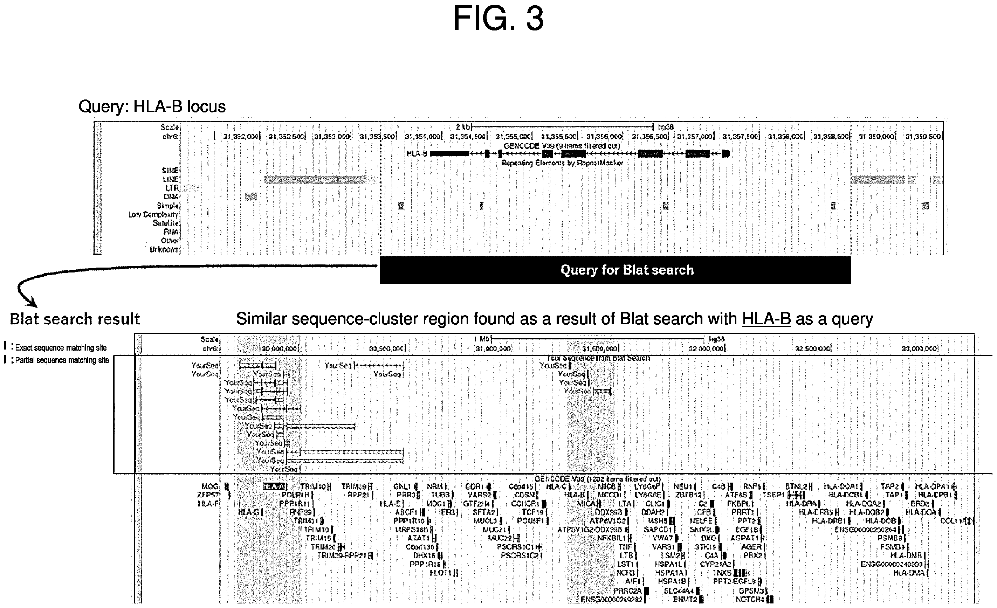

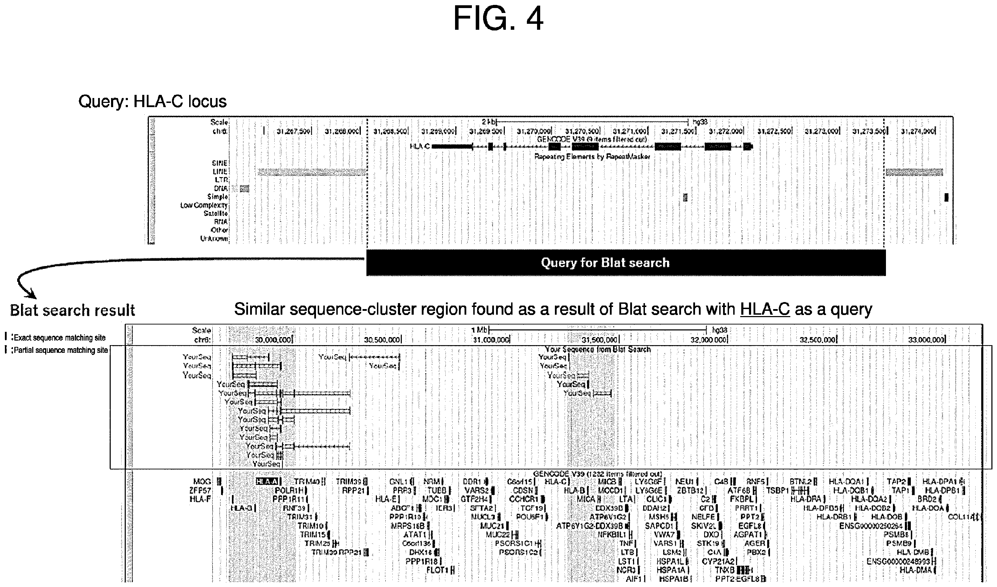

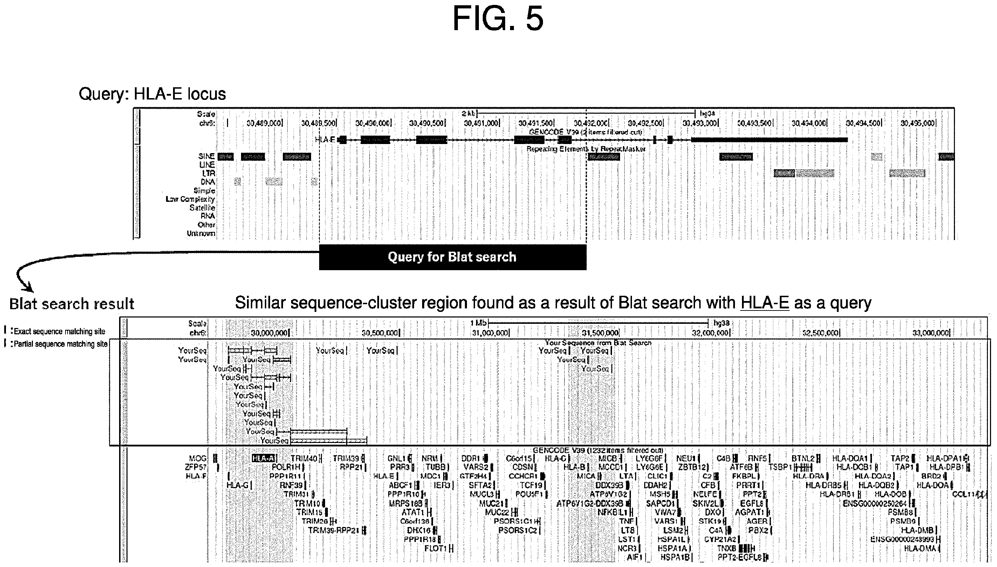

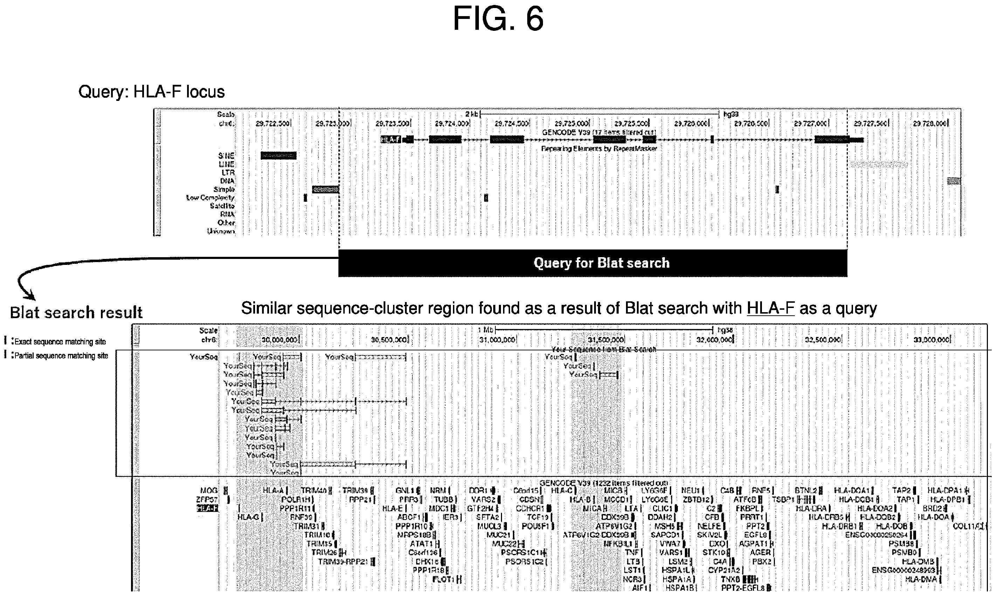

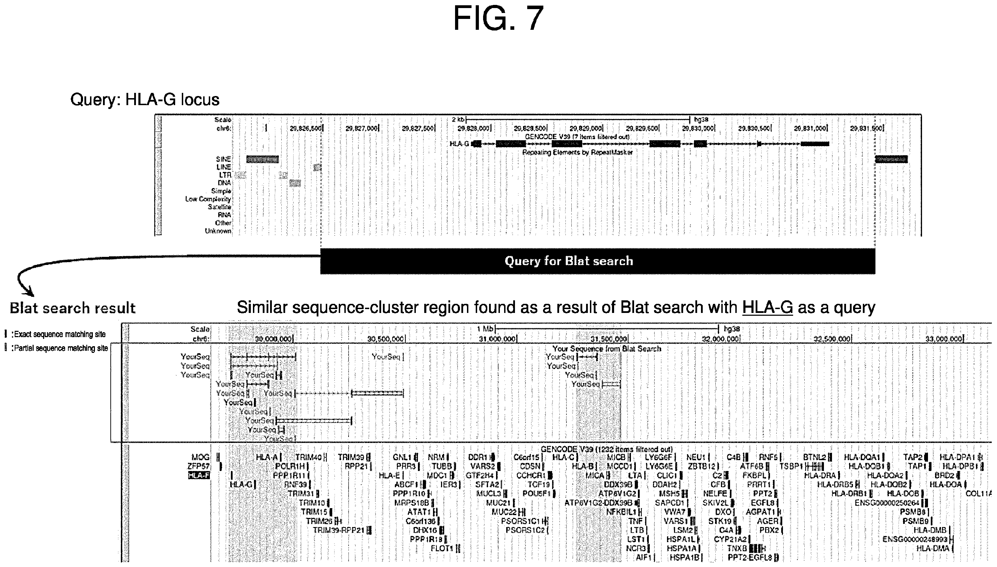

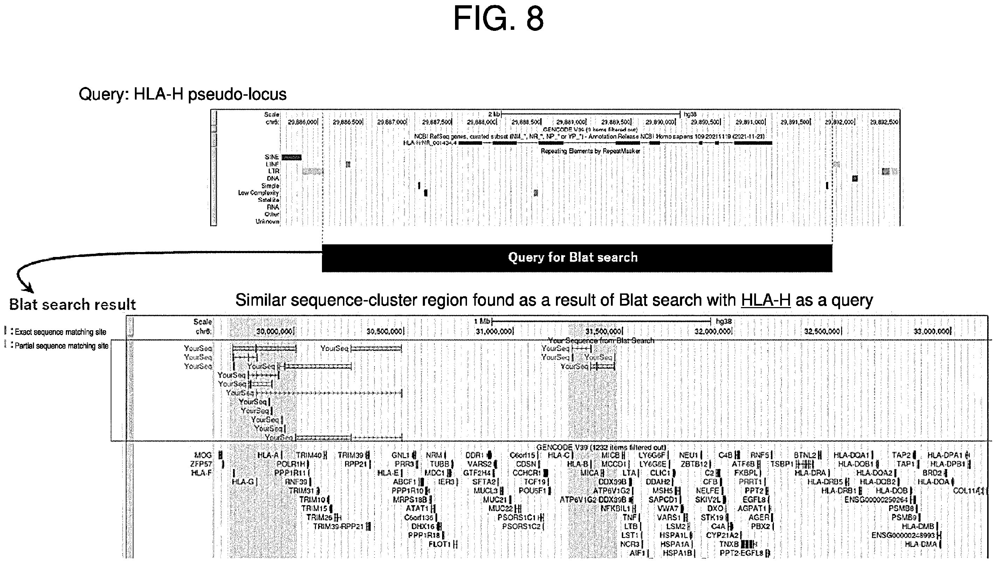

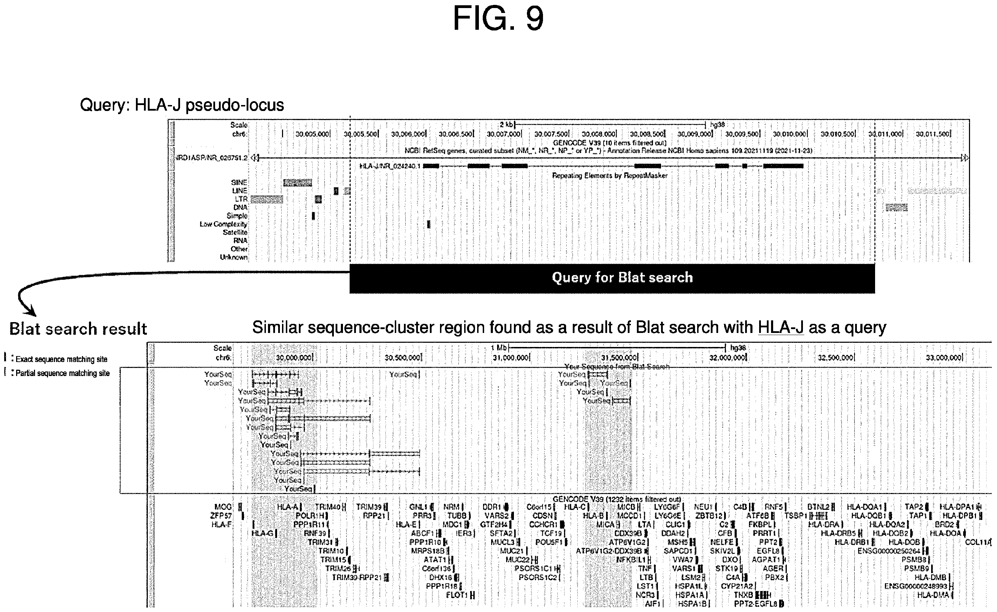

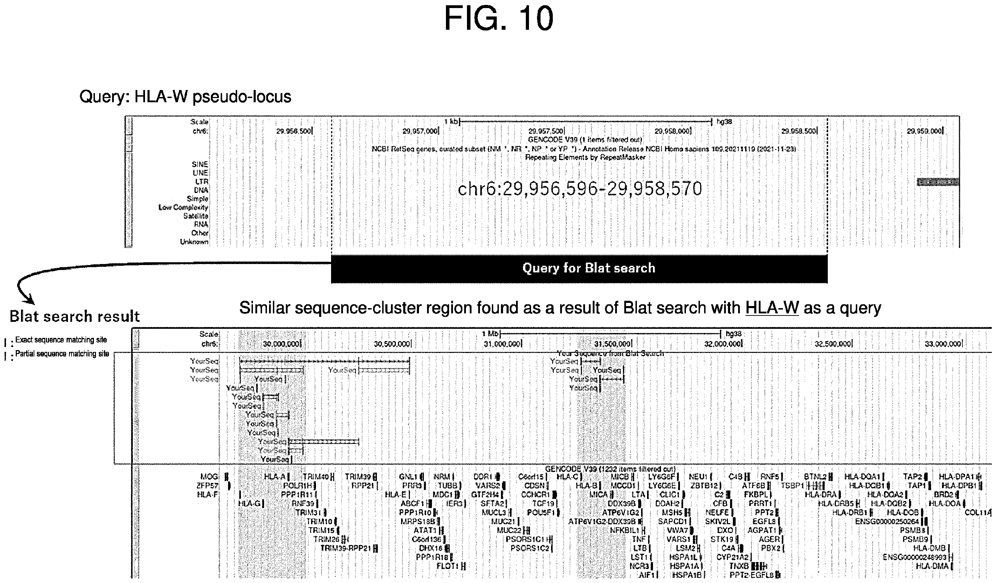

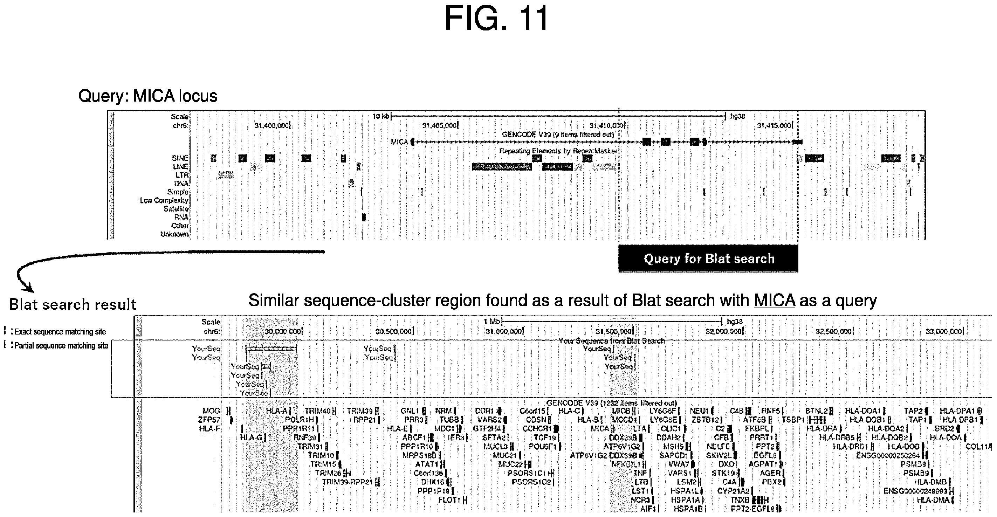

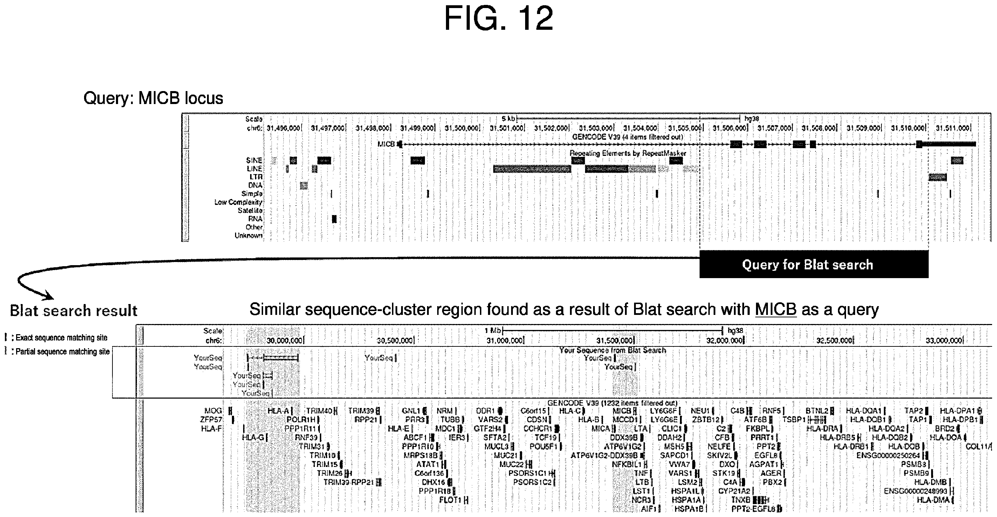

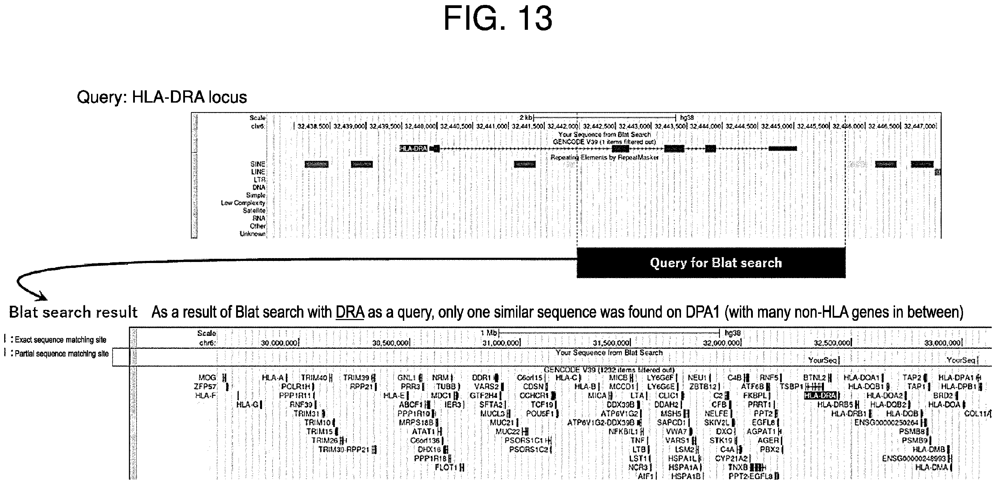

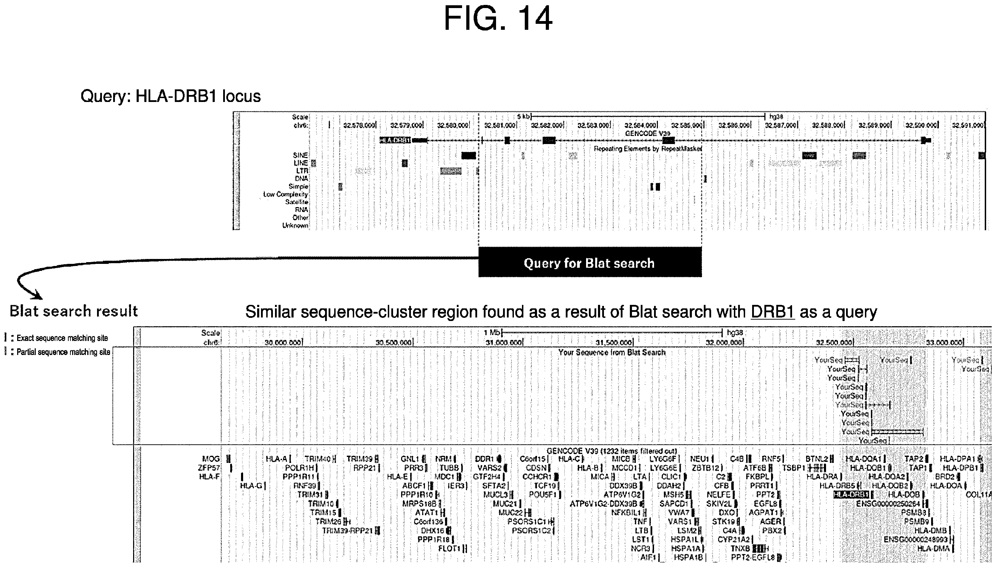

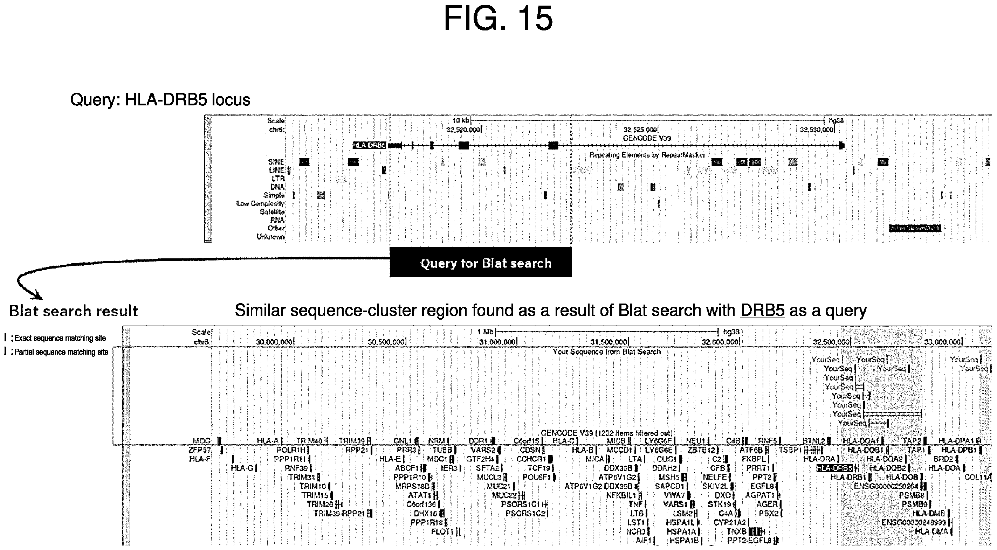

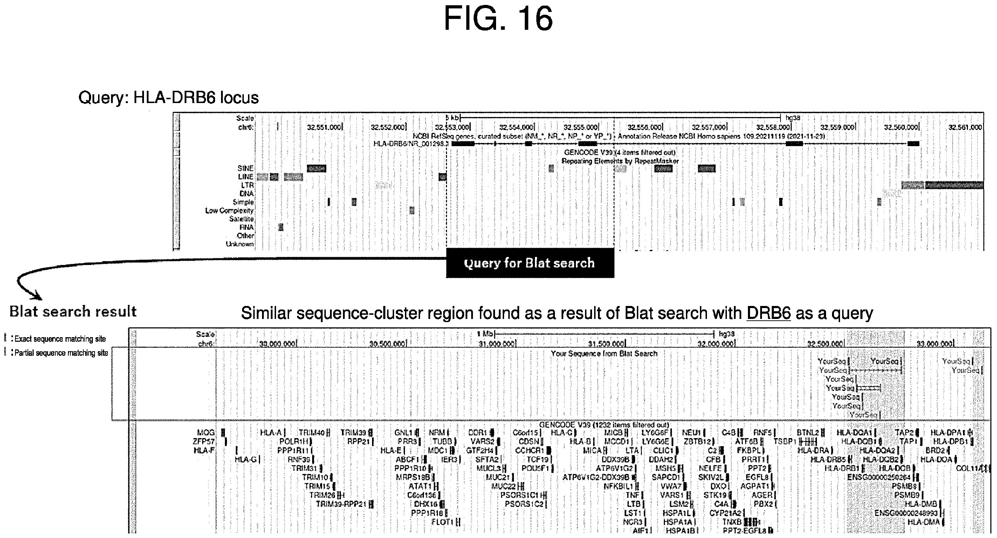

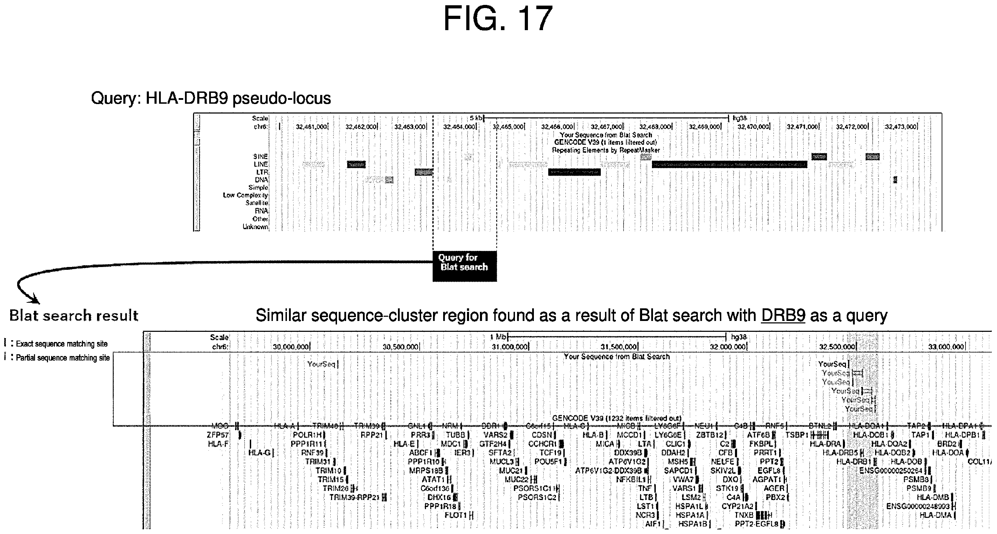

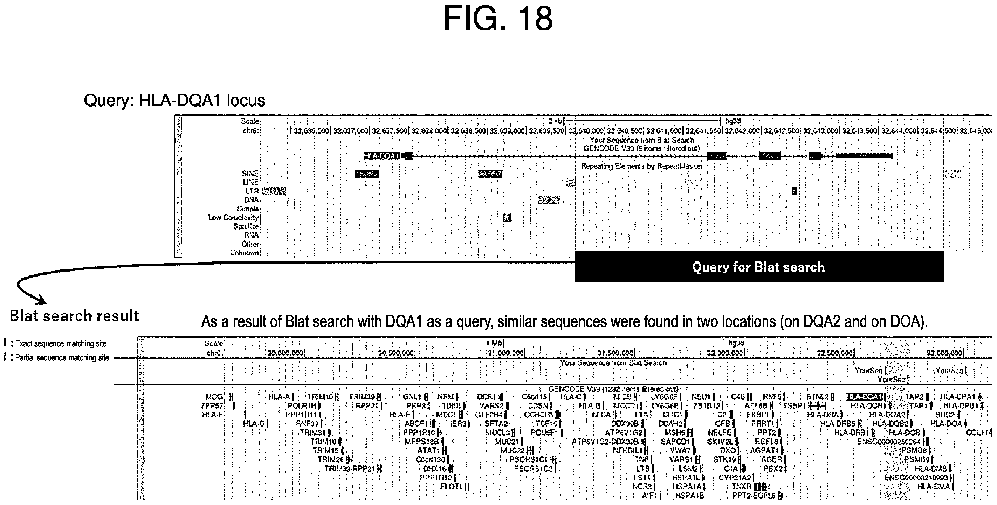

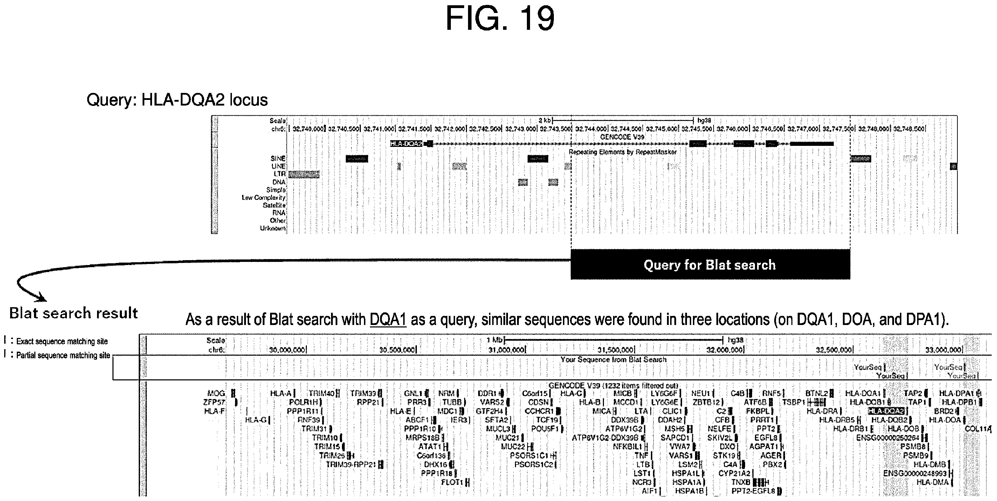

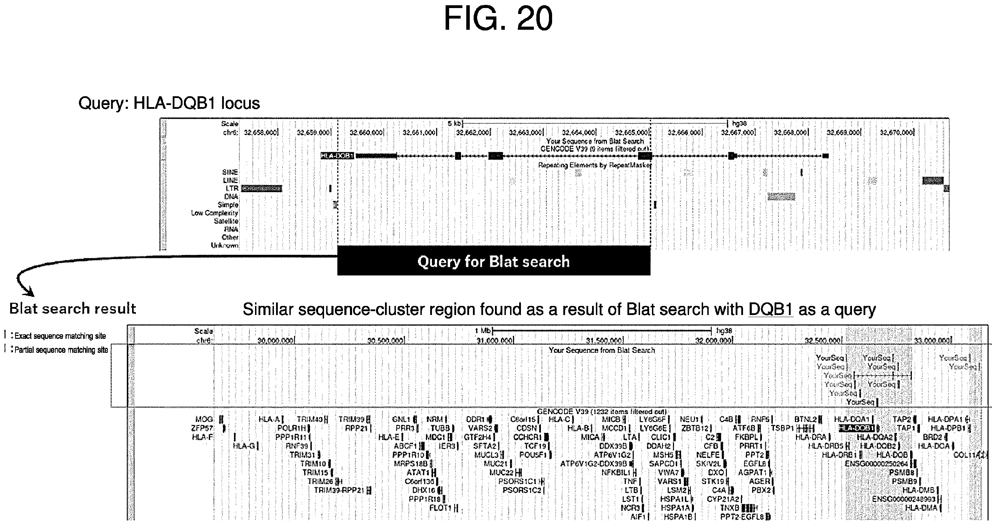

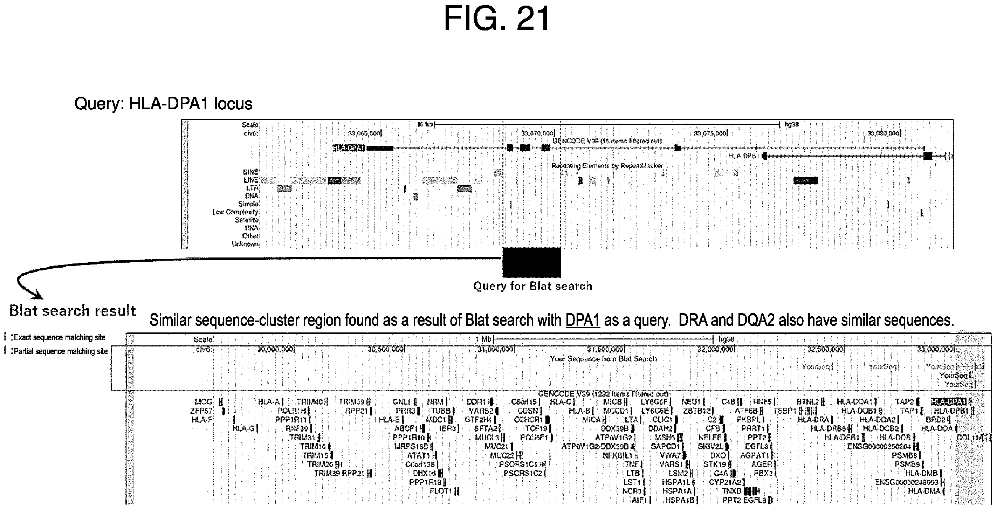

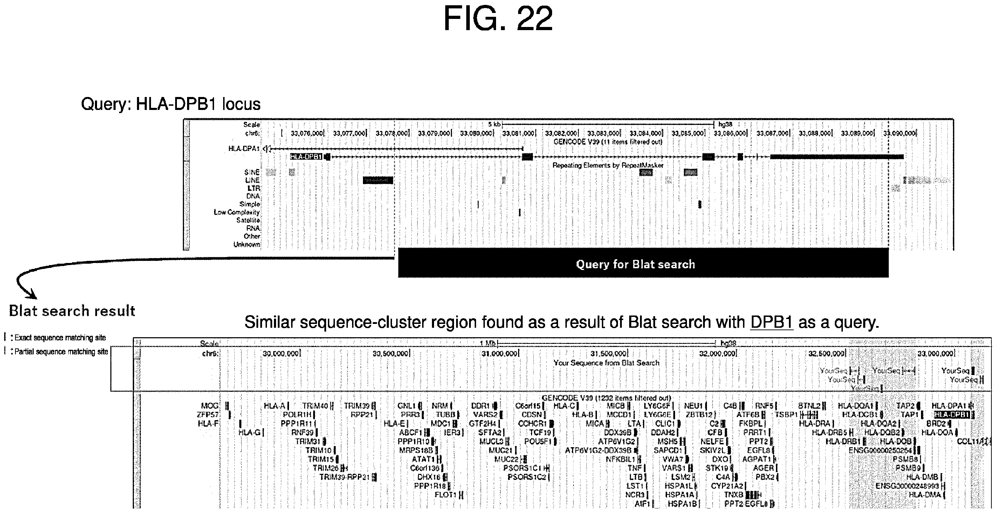

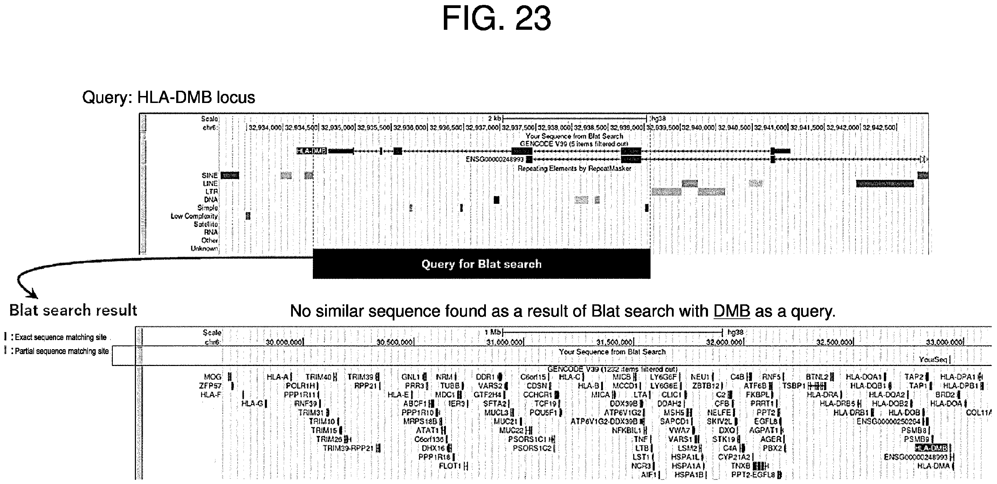

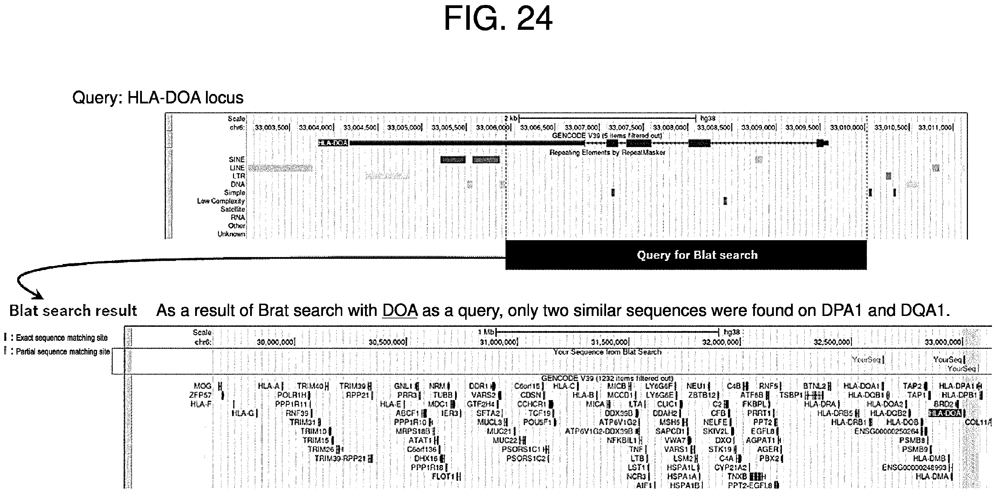

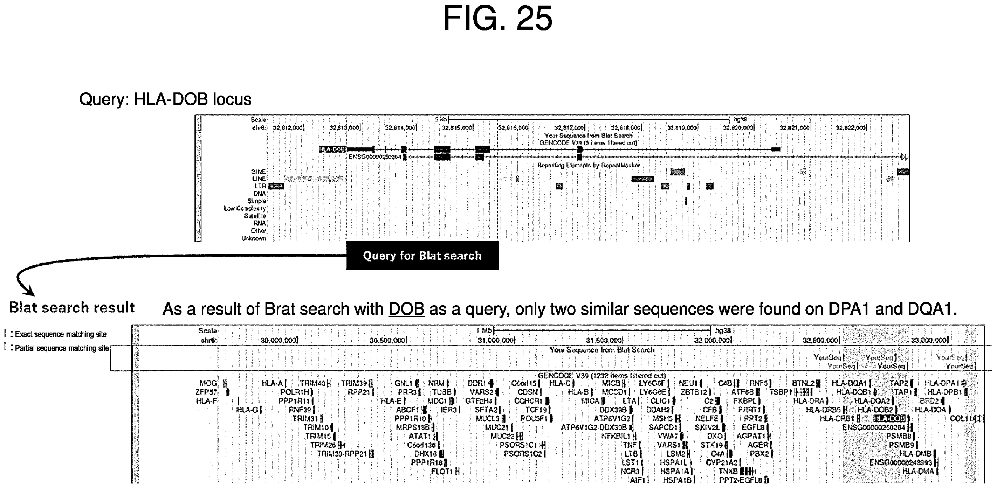

A Blat search was conducted on the human genomic sequence (hg38 genomic sequence) with each HLA as a query. HLA-A, B, C, E, F, G, H, J, and W; MICA and MICB; and HLA-DRA, DRB1, DRB5, DRB6, DRB9, DQA1, DQA2, DQB1, DPA1, DPB1, DMB, DOA, and DOB were each queried as HLA. The results were each as shown in Figures 1 to 25.

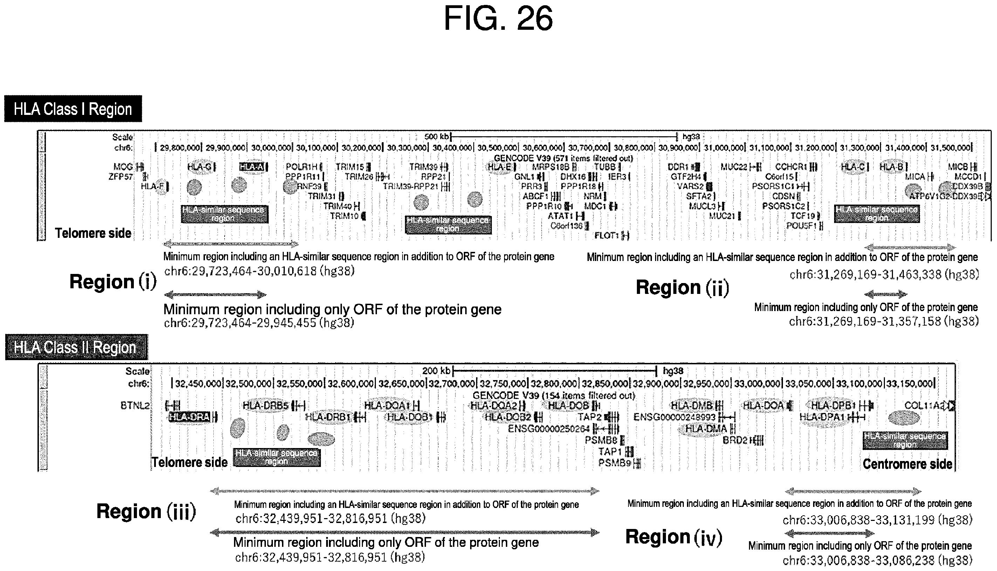

As shown in Figures 1 to 25, a plurality of similar sequences were accumulated in (i) chr6:29,711,000-30,020,000 region, (ii) chr6:31,176,000-31,534,000 region, (iii) chr6:32,445,000-32,821,000 region, and (iv) chr6:33,020,000-33,147,000 region. The results were summarized in Figure 26. The regions (i) to (iv) in Figure 26 each correspond to the regions described above. More specifically, the plurality of similar sequences were located in (i) a chromosomal region corresponding to chr6:29,723,464-29,945,455 chromosomal region of the hg38 genomic sequence, (ii) a chromosomal region corresponding to chr6:31,269,169-31,357,158 chromosomal region of the hg38 genomic sequence, (iii) a chromosomal region corresponding to chr6:32,439,951-32,816,951 chromosomal region of the hg38 genomic sequence, and (iv) a chromosomal region corresponding to chr6:33,006,838-33,086,238 chromosomal region of the hg38 genomic sequence.

One of the reasons for the difficulty of sequencing HLA loci is the accumulation of a plurality of similar sequences. Therefore, even in an attempt to selectively disrupt one gene in any of the regions of (i) to (iv), genome editing methods including CRISPR/Cas9 may result in engineering to other similar sequences in addition to the targeted gene. It is also difficult to examine the effects on surrounding similar sequences because of the difficulty of sequencing the disrupted region due to the presence of overlapping similar sequences. Therefore, the present invention provides cells deficient in at least one, preferably two, more preferably three, and further preferably four of (i) to (iv). Such cells lack at least one HLA and are easy to sequence around the deleted region, thus suitable for revealing the success or failure of further editing by sequencing.

International Publication No. WO 2021/206054 discloses a technique for efficiently and simultaneously deleting a specific region of several hundred kb on two alleles in diploid cells (UKiS). In normal genome editing, one allele can be efficiently edited in a diploid cell, but the efficiency of simultaneously editing both alleles is extremely low. The UKiS technique provides a dramatic improvement in the efficiency of editing both alleles simultaneously and is suitable for knockout applications as in this case. In International Publication No. WO 2021/206054, a donor DNA having upstream and downstream homology arms adjacent to a region to be deleted and mutually distinguishable selective markers between the upstream and downstream homology arms is brought into contact with a genomic DNA having a cleavage in any or preferably both of a first genomic DNA portion to which the upstream homology arm hybridizes and a second genomic DNA portion to which the downstream homology arm hybridizes, whereby the genomic DNA region present between the first and second genomic DNA portions is deleted and replaced with a sequence (third sequence) between the upstream and downstream homology arms. In International Publication No. WO 2021/206054, the third sequence can also be removed from the genomic DNA, resulting in a genomic DNA in which the first genomic DNA portion and the second genomic DNA portion are seamlessly linked. Moreover, in International Publication No. WO 2021/206054, the same engineering can be achieved on both alleles at the same time. In this way, it is possible to delete the entire repeat-rich HLA region from the genomic DNA by using genome engineering techniques, such as those disclosed in International Publication No. WO 2021/206054.

According to this Example, the regions of (i) to (iv) were considered regions comprising sequences that are difficult to sequence. Therefore, deletion of at least one of the regions considered difficult to sequence facilitates sequencing of the regions with the deletion. In addition, deletion of repeats can also reduce the risk that the target of genome editing unexpectedly appears in other repeats to cause off-target engineering. Then, since the genomic DNA after engineering is easy to sequence, it is possible to confirm whether the gene has been engineered as intended.

Therefore, according to present invention, it can be understood that UKiS can in principle be used to create cells deficient in at least one, preferably two, more preferably three, and further preferably four of (i) to (iv). In order to apply the UKiS technique, a unique sequence in the genome is required. A deletion can be introduced in a sequence-specific manner by targeting the unique sequence. In the case of the deletion of a small region, exact homologous recombination is difficult because the sequence targeted for homologous recombination due to the repetitive sequence constitutes a repetitive sequence. In the case of the deletion of a large region, a unique sequence in the genome can be appropriately selected as a target sequence for homologous recombination.

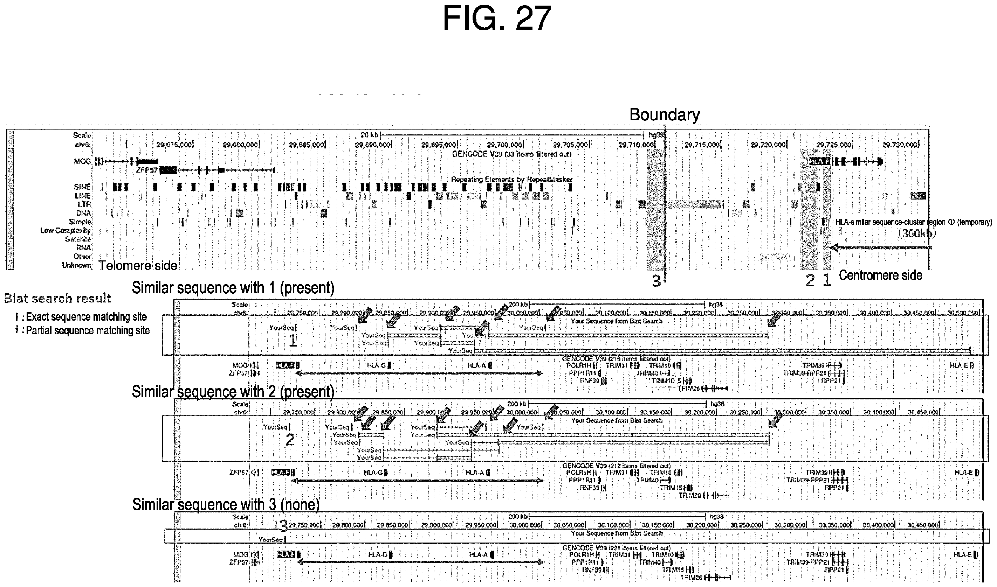

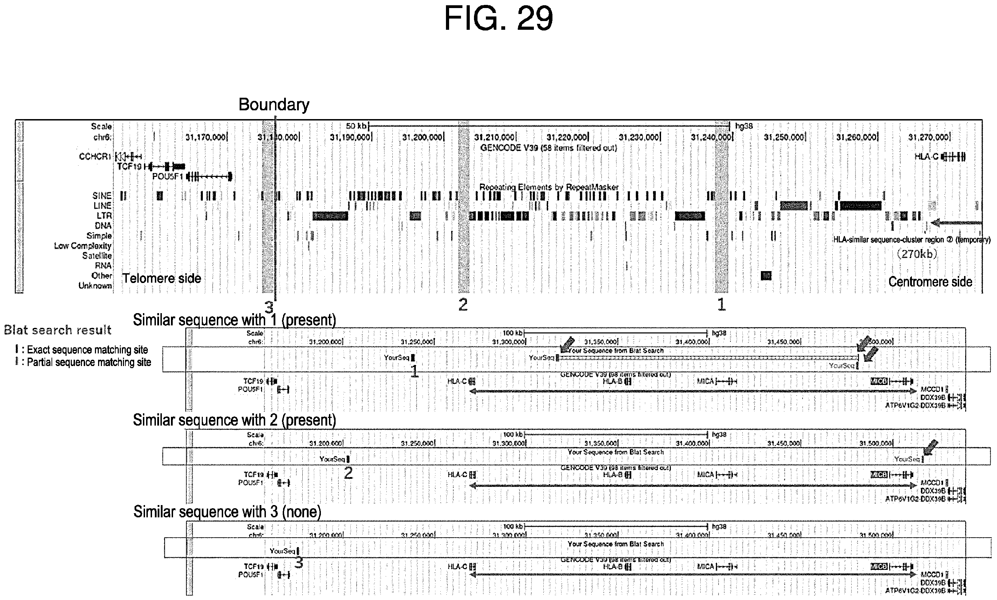

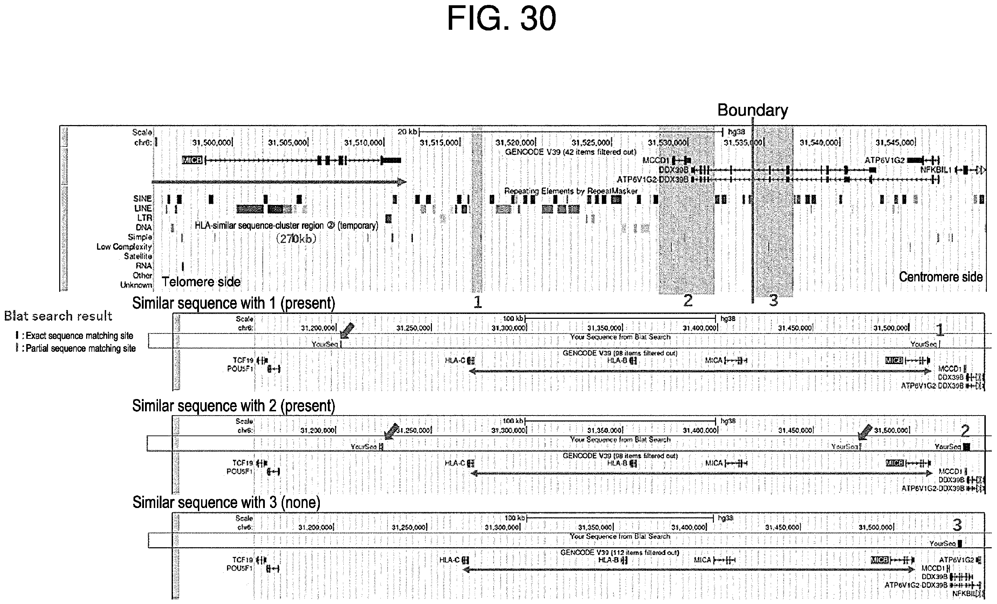

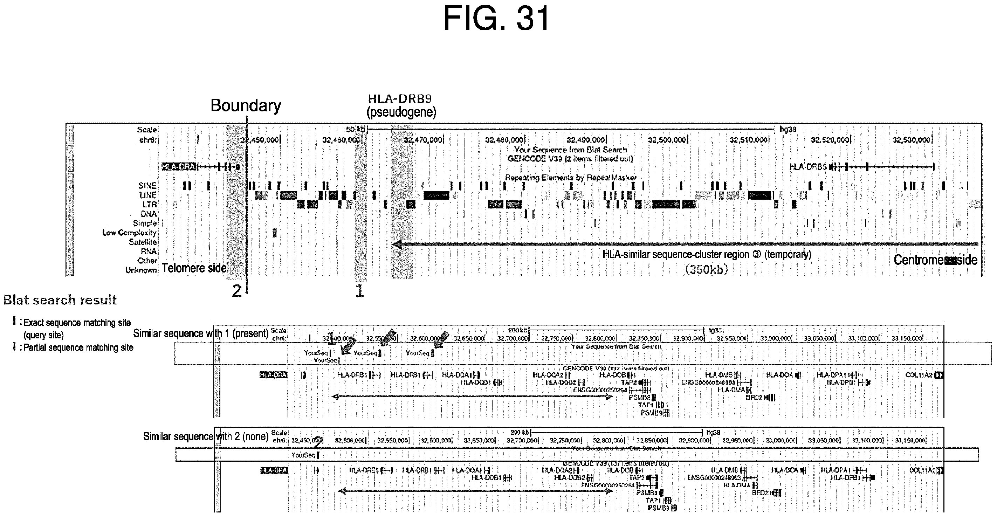

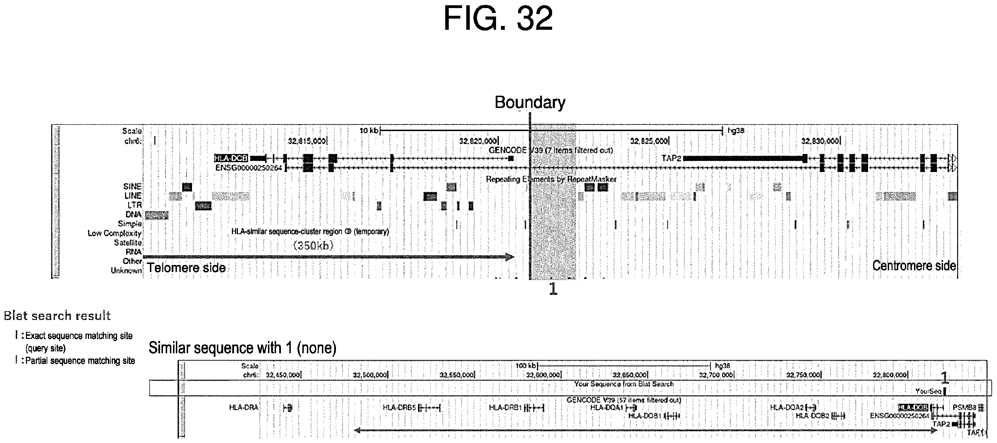

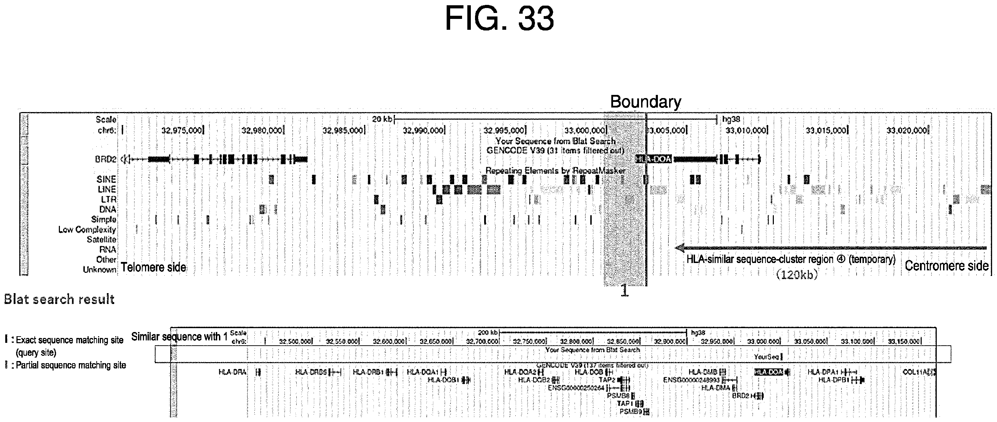

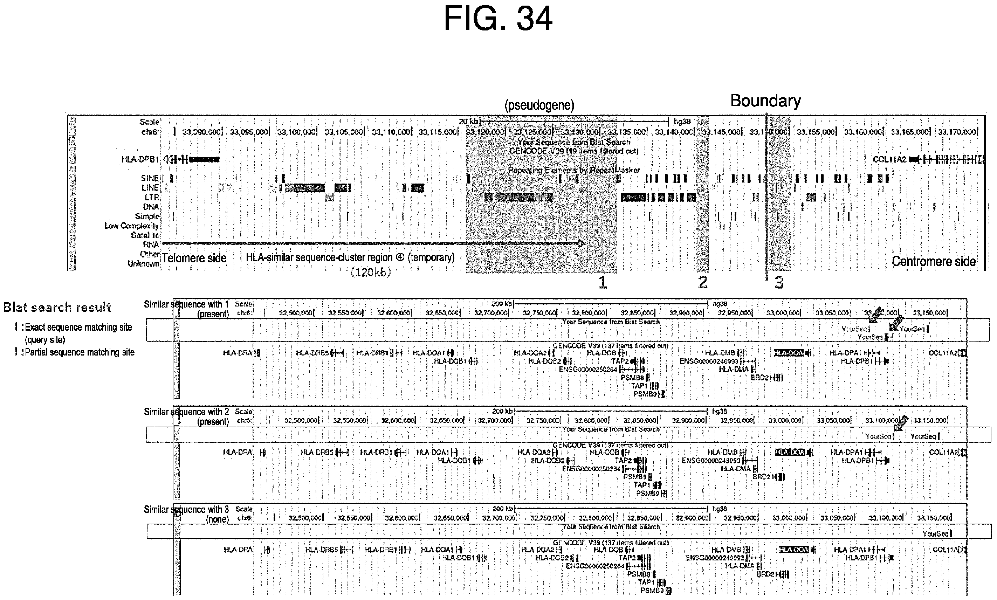

Figures 27 to 34 show the results of searches conducted to identify the telomeric and centromeric boundaries for (I) to (iv) above. According to Figure 27, region 1 and region 2 cannot be targeted because the regions have similar sequences, whereas region 3 comprises a unique sequence having no similar sequence and can be a candidate for a sequence that can be targeted.

### Example 2: Production of cells with HLA deficiency

In this Example, the technique of the present disclosure was applied to cells to induce HLA deficiency. The cells used were iPS cells capable of differentiating into various cells.

### (1) Introduction of UKiS marker

Cell preparation: iPS cells were seeded in 24-well plates pre-coated with iMatrix-511 diluted 150 times in DPBS at 1.25 × 10⁵ cells per well. The medium used was StemFit AK02N (10 µM Y-27632).

Marker insertion: 50 µL of OptiMEM, 300 ng of gRNA/Cas9 expression plasmid for cleaving marker insertion sequence, 100 ng of donor plasmid (GFP-Puro), 100 ng of donor plasmid (RFP-Blst), and 2 µL of Lipofectamine Stem transfection reagent were mixed and incubated at room temperature for 5 minutes to prepare a transfection solution which was then added to the cell-seeded 24-well plates. The donor plasmid (GFP-Puro) had genes encoding green fluorescent proteins (GFP) operably linked to an EF1 promoter and a drug resistance gene, puromycin resistance genes (Puro). The GFP and Puro were linked to each other via a T2A sequence. The donor plasmid (RFP-Blst) had genes encoding red fluorescent protein (RFP) operably linked to the EF1 promoter and a drug resistance gene, brastcidin resistance gene (Blst). The RFP and Blst were linked to each other via a T2A sequence. The details were as shown in Example 1 and Figure 1 of WO2021/206054. In the present specification, the marker carried on the donor plasmid is referred to as a UKiS marker, the cell into which the UKiS marker has been introduced is referred to as a UKiS marker-introduced cell, and the UKiS marker-introduced cell is also referred to as a UKiS 1^{st} stage cell.

Drug selection and expansion culture: The obtained cells were seeded in 6-well plates to perform drug selection of the cells in the presence of puromycin and brastcidin. StemFitR AK02N (containing 1 µM of puromycin and 10 µM of brastcidin) was used as the drug selection medium. As a result, surviving cells after drug selection are resistant to the two drugs, and specifically, have both puromycin resistance and brastcidin resistance genes. Such cells are theoretically generated when puromycin resistance genes are introduced to one allele of the gRNA-targeted region while brastcidin resistance genes are introduced to the other allele. Expansion culture of the surviving cells was performed to obtain cells with UKiS markers. The cells with UKiS markers were cloned as needed. The cells were cloned by recovering the surviving cells after drug selection one colony at a time and allowing the cells to grow.

### (2) Cleavage at two locations in the genome and flow cytometry

Cell preparation: iPS cells were seeded in 24-well plates pre-coated with iMatrix-511 diluted 150 times in DPBS at 1.25 × 10⁵ cells per well. The medium used was StemFit AK02N (10 µM Y-27632).

Introduction of gRNA/Cas9: Two gRNAs were prepared to target each end of the region to be deleted. A solution 1 (25 µL of OptiMEM and 1.5 µL of CRISPRMAX) and solution 2 (25 µL of OptiMEM and 62.5 ng of gRNA (left side), 62.5 ng of gRNA (right side), 750 µg of Cas9 protein, and 1.2 µL of Cas9 plus) were mixed and incubated at room temperature for 5 minutes to prepared a transfection solution which was then added to the cell-seeded 24-well plates. After 24 hours, the medium was changed.

Flow cytometry: After culturing, GFP-negative and RFP-negative cells were selected using a flow cytometer, and the cells were seeded one by one in 96-well plates to expand the cells. In this way, cells in which the region comprising the UKiS marker-introduced region is deleted in both alleles can be obtained.

### (3) Genotyping PCR

Genome purification: About 2 × 10⁵ mutation-introduced cells were recovered to purify the genome.

Primers for genotyping used were as shown in Table 1 below. PCR was carried out in a conventional manner.

**[Table 1]**

| Table 1: Primer for genotyping | | | |
|---|---|---|---|
| Correspondence figure | Subject to be amplified | Forward primer | Reverse primer |
| 36 | HLA-A | CCTCACCTTCCCCTCTTTTC | ACCTTCCCCTGTGACTTGTG |
| 36 | HLA-F | CTCACCTTCCCTTCCTTTCC | ACCTTCCCCTGTGATTTGTG |
| 36 | HLA-G | CCACCACCCTGTCTTTGACT | TGTCACCCCTTCCTTACCTG |
| 36 | LxUDC (0.1) junction | GAGGCAGAAAGGCACACATA | AGCCTCTCCTTCCCATTCTT |
| 37 | HLA-B | TCCATCAACCTCTCATAGCAAA | ACTTCTGGAAATTCCTTTTGG |
| 37 | HLA-C | GATGGCCCATGTGTGGAT | TTATCCCAGGTGCCTGTGTC |
| 37 | LxUDC (1.0) junction | TTCCCACTTACAGCCAGACC | TACGGGAGGTAGCCAGTGTT |
| 40 | Puro (L) | TGGGAAGTGGTCAGGAGATAA | GGCCTTCCATCTGTTGCT |
| 40 | Puro (R) | GACATCGGCAAGGTGTGG | GTGGTGGAGTGAACGGAGA |
| 40 | Blst (L) | TGGGAAGTGGTCAGGAGATAA | TGTAATCTTCTCTGTCGCTACTTC |
| 40 | Blst (R) | TGCACCAGATTGTTTTGTGT | GTGGTGGAGTGAACGGAGA |
| 41 | E-Del | CCACCTTAGCCTCCTCCTCT | AAAGCATATTTGGGCACTCTG |
| 42 | HLA-DRA | AGACAAGTTCACCCCACCAG | TCACCTCCATGTGCCTTACA |
| 42 | HLA-DQAI | ACAGAGAGAAGGGCACAGGA | CATCAGCAGAAGGGAGGAAG |
| 42 | HLA-DOB | AACATGCAAAGGGGATTCTG | TGGAAGTGGCTTTTGGTTTC |
| 42 | LxUDC (1. 0)_3 junction | TGGATTTCATGCCTCACAAA | TTCCTCTTGTCTCCCCAGAA |
| 43 | LxUDC (1. 0)_4 junction | CACCATCACCCTTCTTCCAC | TGCTTCCTCTTCCCAGCTAA |
| 44 | LxUDC (2.0) junction | CACCATCACCCTTCTTCCAC | TGCTTCCTCTTCCCAGCTAA |
| 43&44 | HLA-DMB | GCAGAGAGTGGGACCAAGAG | TGCTAATTCTGGGGAGATGG |
| 43&44 | HLA-DOA | TCCATCAGGGTCTTCTGGTC | TTTTTAACCGGCTCTGGATG |
| 43&44 | HLA-DPB2 | GAGAGCCCTTAGCTGGTGTG | GTGCAGTTTGTCCACCCTTT |
| 45 | Sig-E (L) | TGGGAAGTGGTCAGGAGATAA | GTCTGGGTAAGGGCCAGGGCC CCCGAGAGTAGCAGGAGGA |
| 45 | Sig-E (R) | ACGTAGGGTCCTTCATCCTG | GTGGTGGAGTGAACGGAGA |
| 47 | A&B (L) | TGGGAAGTGGTCAGGAGATAA | AGACAACTGCCAAGGACACC |
| 47 | A&B (R) | GTGGTGGAGTGAACGGAGA | CCCTGTGTGAGTCCAGAACA |
| 48 | C&E (L) | TGGGAAGTGGTCAGGAGATAA | CCCTGTGTGAGTCCAGAACA |
| 48 | C&E (R) | GTGGTGGAGTGAACGGAGA | CCCTGTGTGTGCCCAGAACA |

**[Table 2]**

| Table 2: gRNA used for genome cleavage | | | |
|---|---|---|---|
| Correspondence figure | For marker introduction | For genome cleavage (left side) | For genome cleavage (right side) |
| 36 | CTTCATCTCCGTCGGCTACG | ATGATGGAAAACTCGATGGG | TGGAATCCGGTATAGGCGCC |
| 37 | TGAGTGACCCGATAAAACTC | TCTTAAAAACTTCTATATCG | GTGCAACTCCCGAATAGGCT |
| 42 | TGGGCCTTATCATCCGTCAA | TGGGTCTGTCGAGCACAAGG | GGTCAAATGCCTTGTACTCG |
| 43, 44 | GGGAGGGCCGGTACCGTTGA | ATAGCTAACGTCACTATTGC | CGTTCTCAGCGCGGGCACTA |
| Correspondence figure | For UKiS 1^{st} stage | For UKiS 2^{nd} stage (GFP allele) | For UKiS 2^{nd} stage (RFP allele) |
| 40 | GCCGCTGCCGCTCTACGCTT | - | - |
| | CAGAAAGGGTAAGCGCACAG | - | - |
| 41, 46 | - | GGCGCAACGCGATCGCGTAA | - |
| | - | - | GACGCCAGTGCATTCGACGA |
| 45, 46, 47, 48 | - | GGCGCAACGCGATCGCGTAA | - |

### (4) Further engineering of cloned UKiS marker-introduced cell

UKiS marker-introduced cells represent a platform for a variety of further engineering. The UKiS marker-introduced cells can be further engineered to cleave a region in which the UKiS marker is inserted (UKiS marker-inserted region) and brought into contact with further donor DNA, thereby replacing the UKiS marker-introduced region with the sequence of the further donor DNA. For example, cells (i.e., UKiS marker-introduced cells) are prepared in which UKiS markers that are distinguishably different from each other are introduced to two alleles. One allele in the UKiS marker-inserted region can be engineered by selective cleavage of the one allele. The other allele can be engineered in the same manner.

Specifically, 50 µL of OptiMEM, 400 ng of gRNA/Cas9 expression plasmid for cleavage of UKiS marker-inserted regions (for GFP allele or RFP allele), 100 ng of donor plasmid for mutation introduction, and 2 µL of Lipofectamine Stem transfection reagent were mixed and incubated for 5 minutes at room temperature to prepare a transfection solution which was added to a cell-seeded 24-well plate. GFP-negative cells or RFP-negative cells were recovered by flow cytometry to expanded the cells.

The cells thus obtained indeed lacked four regions: chr6:29,706,837-29,956,199, chr6:31,225,738-31,376,781, chr6:32,421,257-32,820,167, and chr6:32,934,298-33,161,621.

### (5) Evaluation of HLA expression

The cells were dissociated into single cells with TrypLE. The cells were washed with PBS containing 0.5% BSA, and anti-HLA-A, anti-HLA-B, and anti-HLA-C antibodies (BioLegend 311413; 200× dilution) were applied to the cells. After washing, the cells were analyzed with a flow cytometer.

### (6) Evaluation of expression of pluripotent stem cell marker

The cells were fixed in PBS with 4% paraformaldehyde. The cells were washed with PBS, then PBST was added and allowed to stand for 30 minutes at room temperature. The cells were allowed to stand in PBST with 1% bovine serum albumin (BSA) for 1 hour at room temperature. Primary antibodies (anti-OCT4 antibody, anti-NANOG antibody, and anti-SSEA4 antibody) were reacted with the cells. The cells were washed and then reacted with secondary antibodies. The nuclei were then stained by DAPI. The stained cells were observed under a confocal microscope.

### Results

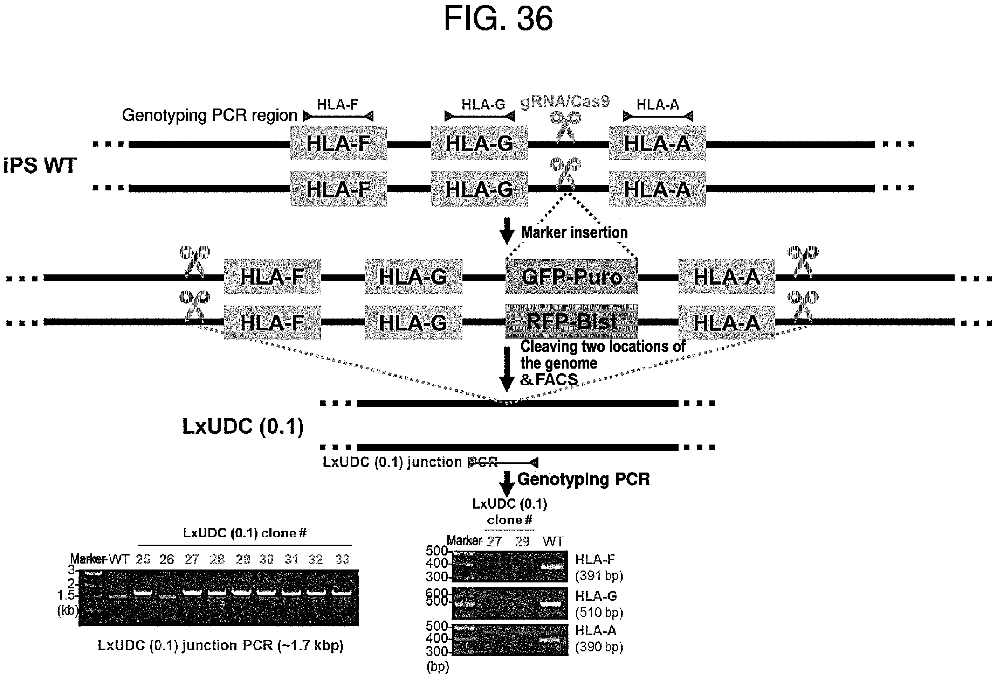

The region between HLA-G and HLA-A of the cell was targeted by gRNA and cleaved by Cas9, and the UKiS marker was integrated into the region (see Figure 36). The outside of a series of regions from HLA-F to HLA-A of the cell obtained by drug selection in the presence of Puro and Blst was targeted by gRNA and cleaved by Cas9, to obtain cells lacking the whole series of regions from HLA-F to HLA-A (clones 27 and 29) (see Figure 36).

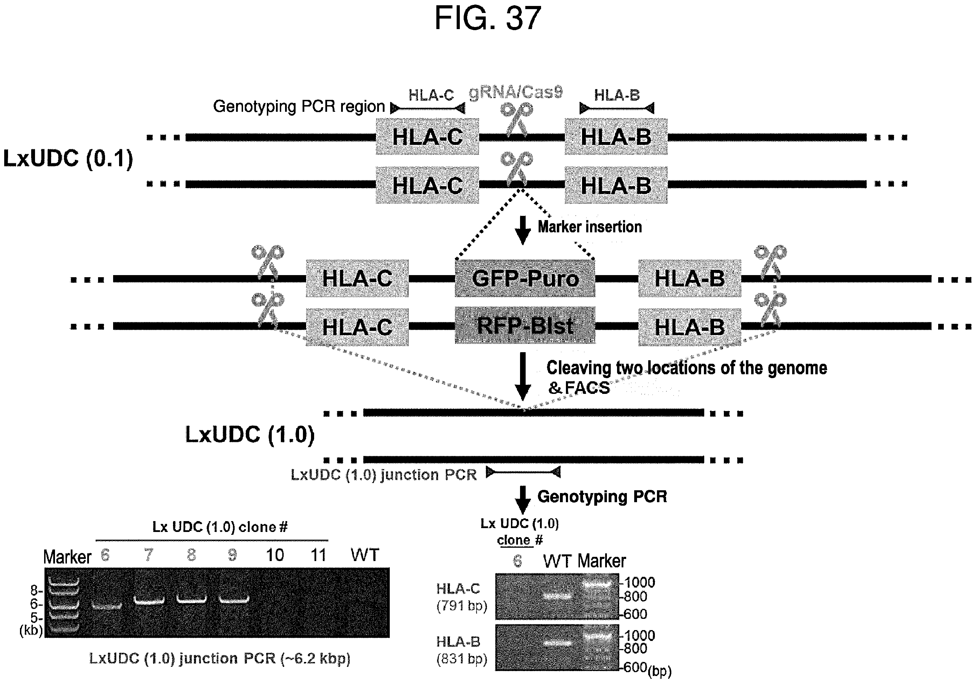

Next, the region between HLA-C and HLA-B of the clone 27 was targeted by gRNA and cleaved by Cas9, and the UKiS marker was integrated into the region (see Figure 37). The outside of the series of regions from HLA-F to HLA-A of the clone obtained by drug selection in the presence of Puro and Blst was targeted by gRNA and cleaved by Cas9 to obtain cells lacking the whole series of regions from HLA-F to HLA-A (clones 6) (see Figure 37).

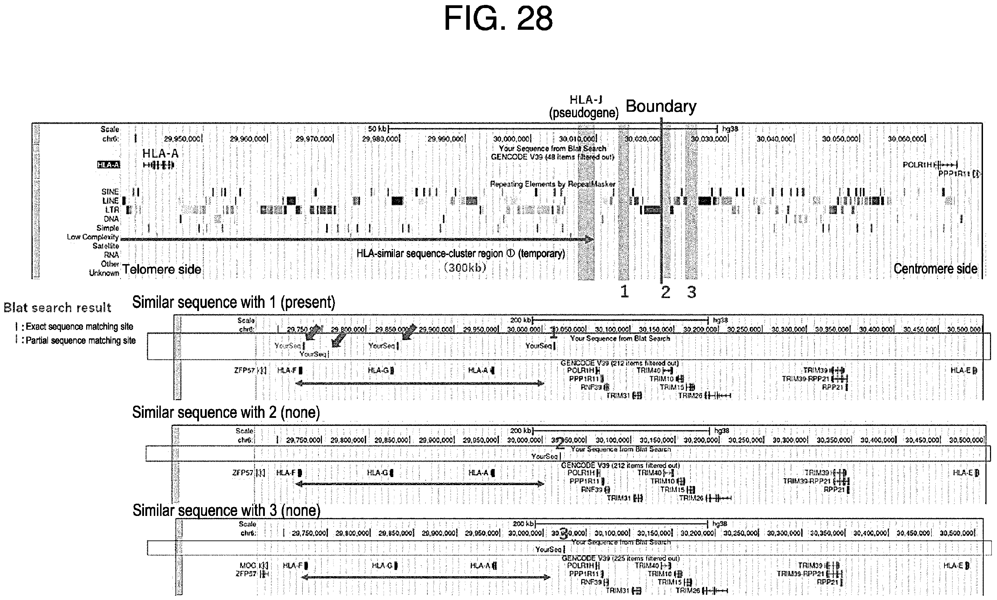

As for clone 6, the expression of HLA-A, HLA-B, and HLA-C was confirmed by flow cytometry. The results showed that, as shown in Figure 28, in the cells before engineering (iPS 771-3G WT), almost all cells were positive for at least one or more of HLA-A, HLA-B, and HLA-C, whereas in clone 6 obtained by engineering, the expression was completely disappeared.

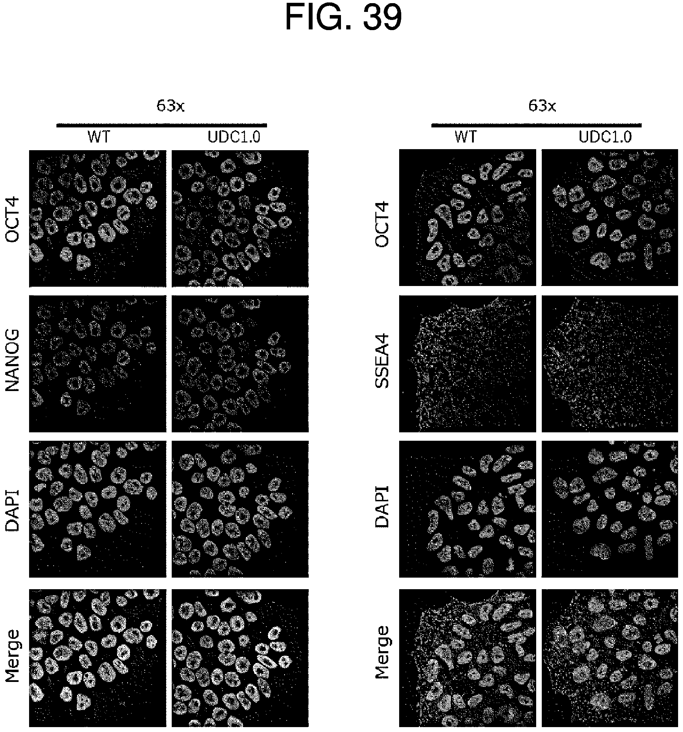

Next, the expression of pluripotent stem cell markers in the cells thus engineered (clone 6) was confirmed. As shown in Figure 39, the cells thus engineered (clone 6) exhibited expression of pluripotent stem cell markers equivalent to the iPS cells before engineering. Thus, it was revealed that a large deletion of the above HLA region did not show any significant effect on the cell proliferation or pluripotency.

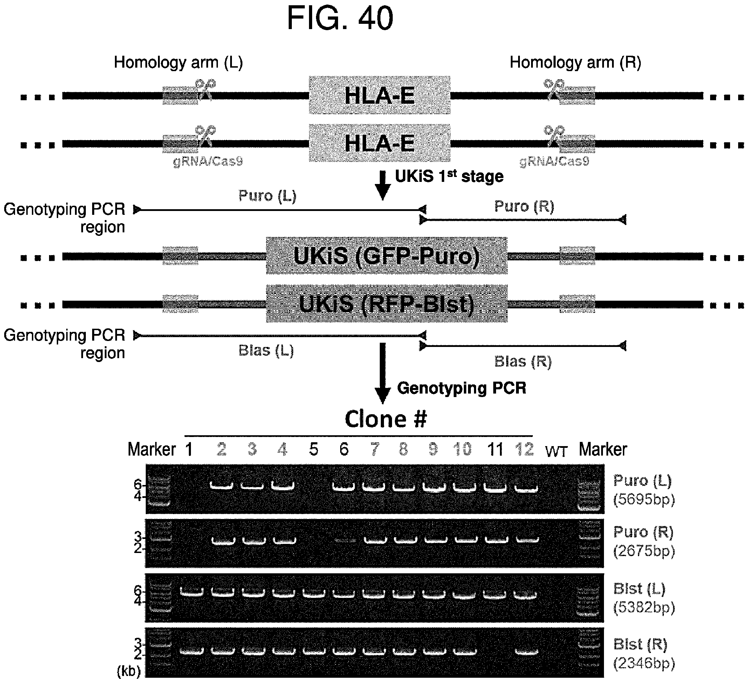

Furthermore, the region comprising HLA-E was targeted by gRNA and cleaved by Cas9 from clone 6, and the UKiS marker was integrated into the region (see Figure 40). As a result, eight clones having the UKiS marker in place of the region comprising HLA-E were obtained (see Figure 40).

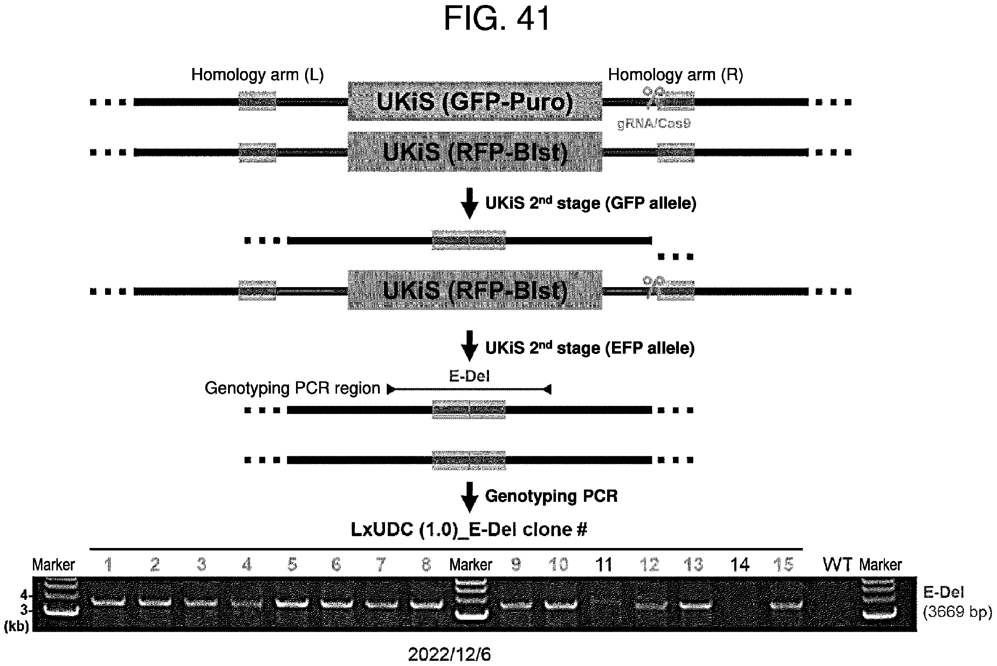

The UKiS marker was removed one by one from one of the obtained eight clones (see Figure 41). As a result, 13 clones in which the region comprising HLA-E was completely deleted were obtained (see Figure 41).

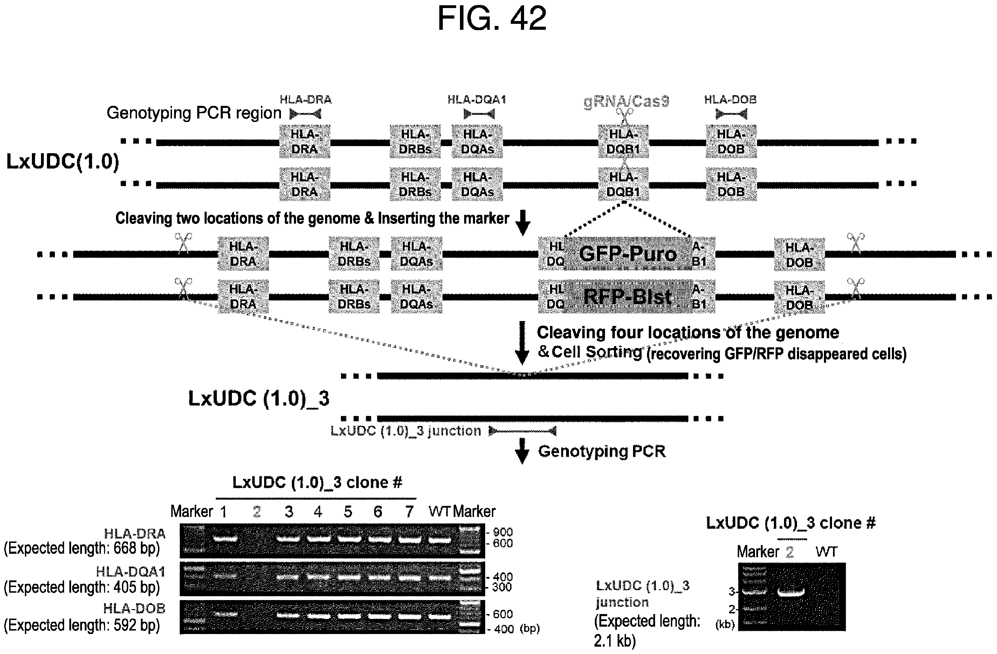

The HLA-class II region was further deleted from clone 6. Specifically, HLA-DQB1 of clone 6 was targeted by gRNA and cleaved by Cas9, and the UKiS marker was integrated into the region (see Figure 42). Then, the outside of a series of regions from HLA-DRA to HLA-DOB was targeted by gRNA and cleaved by Cas9 to obtain cells lacking the series of regions (clones 2) (see Figure 42).

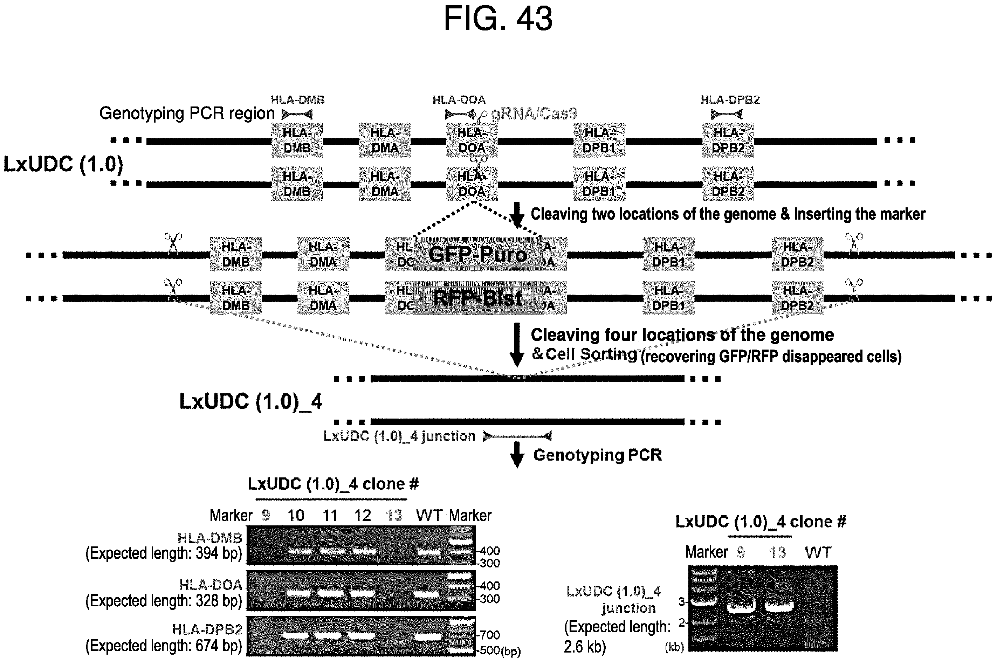

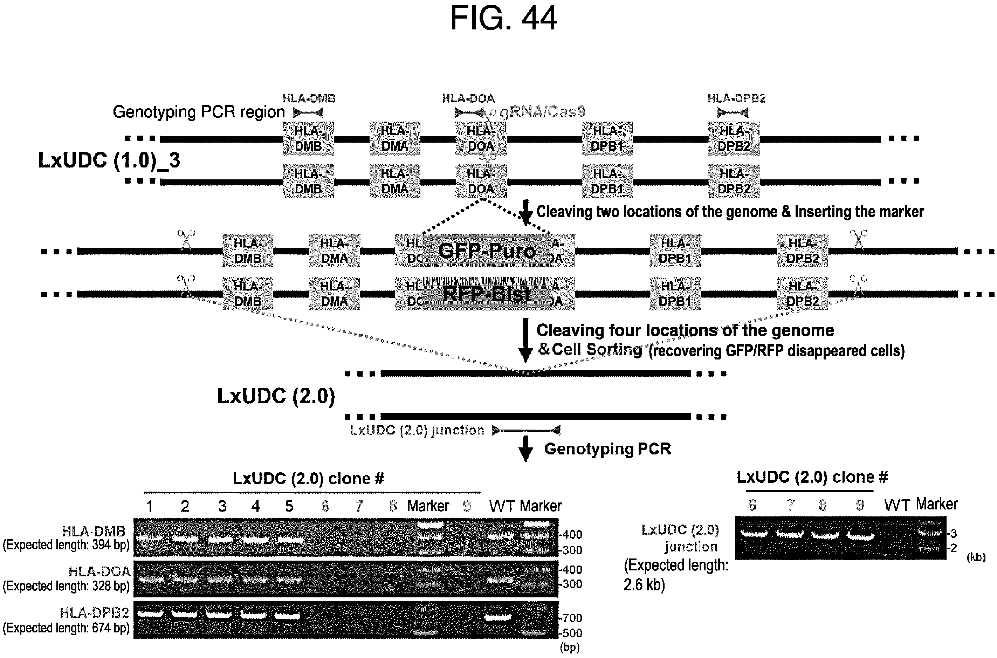

Another region of HLA-class II was further deleted from clone 6. Specifically, HLA-DOA of clone 6 was targeted by gRNA and cleaved by Cas9, and the UKiS marker was integrated into the region (see Figure 43). Then, the outside of a series of regions comprising HLA-DMB to HLA-DPB2 was targeted by gRNA and cleaved by Cas9, to obtain cells lacking the series of regions (clones 9 and 13) (Figure 43). Further, in the clones obtained in Figure 42, the HLA region was deleted according to the scheme of Figure 44. As a result, clones 6 to 9 further lacking the HLA region were obtained (Figure 44).

According to the invention of the present disclosure, it was thus revealed that HLA-cluster regions can be edited or deleted, and a plurality of HLA-cluster regions can also be deleted.

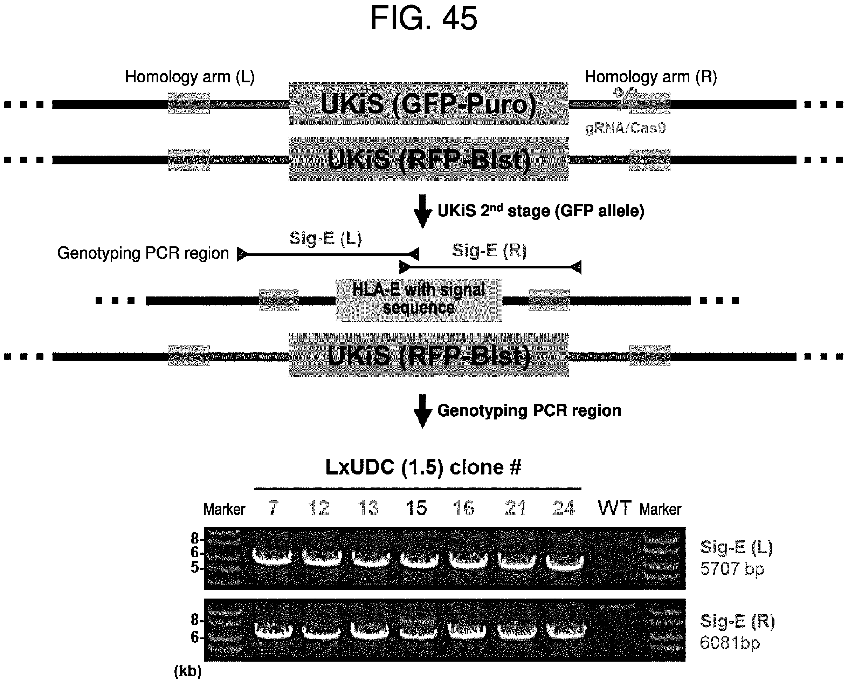

Furthermore, various foreign genes can be introduced to an arbitrary region (e.g., the locus of HLA-E). For example, Figure 45 shows a scheme for introducing a foreign gene to a cell obtained by deleting HLA-E from a cell (lacking HLA-A to C, F, and G), followed by introduction of a UKiS marker to the region instead. In Figure 45, HLA-E
(MAVMAPRTLLLLLSGALALTQTWAGSHSLKYFHTSVSRPGRGEPRFISVGYVDDTQ FVRFDNDAASPRMVPRAPWMEQEGSEYWDRETRSARDTAQIFRVNLRTLRGYYNQSE AGSHTLQWMHGCELGPDRRFLRGYEQFAYDGKDYLTLNEDLRSWTAVDTAAQISEQK SNDASEAEHQRAYLEDTCVEWLHKYLEKGKETLLHLEPPKTHVTHHPISDHEATLRC WALGFYPAEITLTWQQDGEGHTQDTELVETRPAGDGTFQKWAAVVVPSGEEQRYTCH VQHEGLPEPVTLRWKPASQPTIPIVGIIAGLVLLGSVVSGAVVAAVIWRKKSSGGKG GSYSKAEWSDSAQGSESHSL) artificially linked with a signal sequence was integrated into the above locus as a foreign gene. A sequence (AVMAPRTLLLLLSG) corresponding to the 2^{nd} to 15^{th} amino acids of HLA-A was used as the signal sequence. The results were as shown in Figure 45, and a foreign gene was successfully introduced in the 6/7 clones.

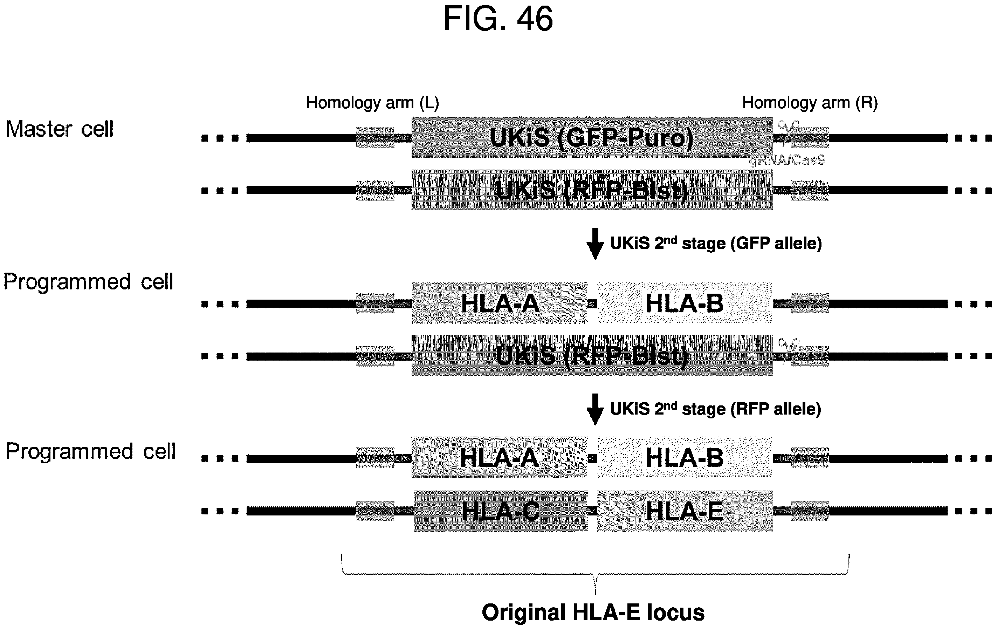

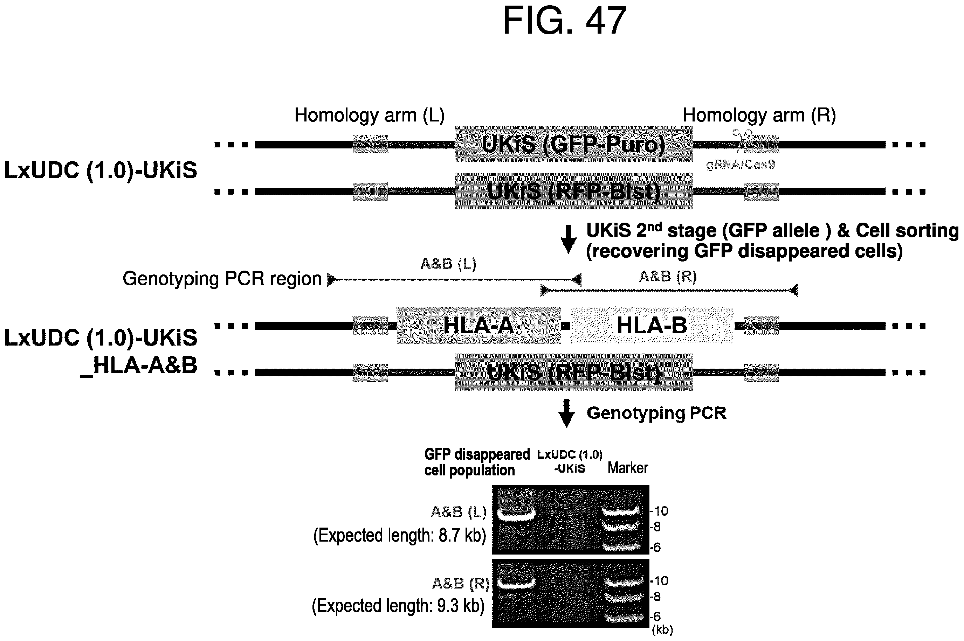

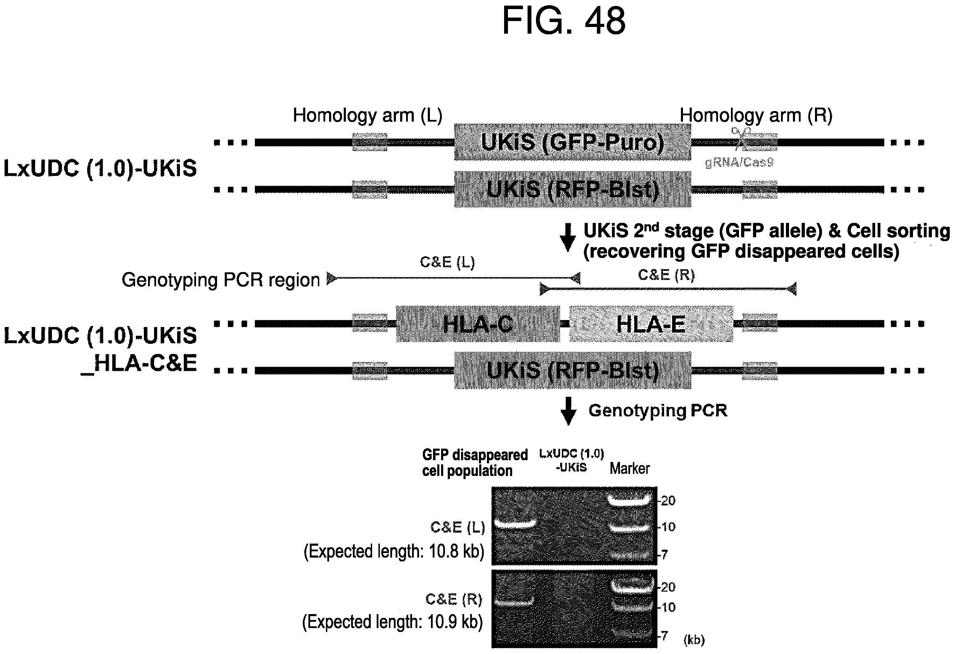

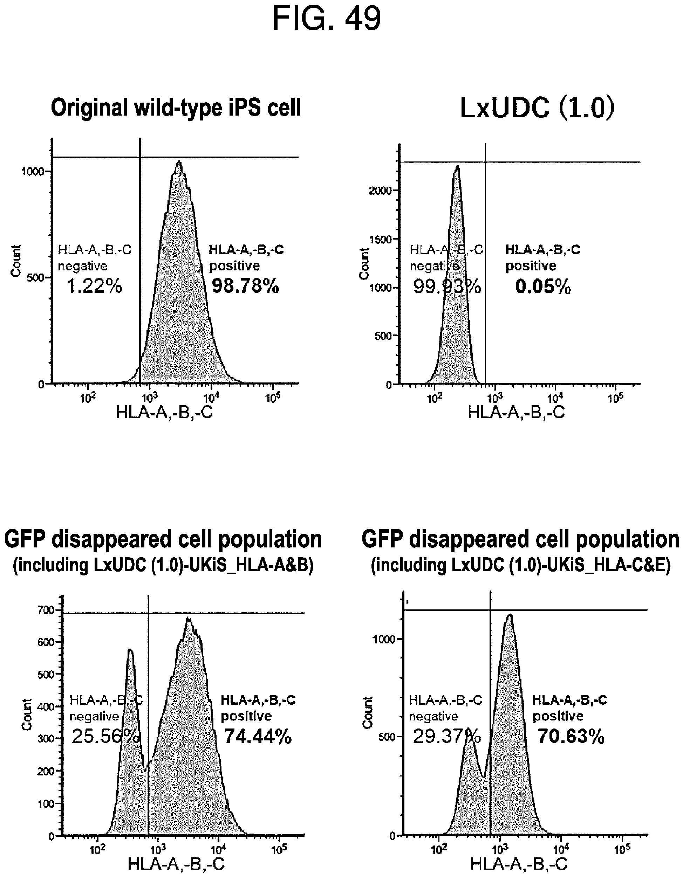

Furthermore, as foreign genes, HLA-A to C and E were introduced to an arbitrary region (e.g., the locus of HLA-E). Figure 46 shows, for example, a scheme for introducing a foreign gene to a cell obtained by deleting HLA-E from a cell (lacking HLA-A to C, F, and G), followed by introduction of a UKiS marker to the region instead. In Figure 46, HLA-A, HLA-B, HLA-C, and HLA-E were integrated into the locus as foreign genes. Each of these genes included the upstream of the protein coding region to the downstream of the stop codon, as well as all introns naturally contained in each gene. Specifically, the genes were as follows: HLA-A included 2.3 kbp upstream to 1.1 kbp downstream of the coding region, HLA-B included 2.1 kbp upstream to 1.1 kbp downstream of the coding region, HLA-C included 2.2 kbp upstream to 1.1 kbp downstream of the coding region, and HLA-E included 2.6 kbp upstream to 2.2 kbp downstream of the coding region. The results were each as shown in Figures 47 and 48, and the desired foreign gene was successfully integrated into the desired allele. The expression of the introduced foreign gene in the resulting cells was confirmed by flow cytometry. Antibodies binding to HLA-A, B and C (Biolegend Inc., serial number: 311413) were used as antibodies for cell detection. Although HLA expression was observed in the original iPS cells, in the engineered cells (LxUDC (1.0)), genes encoding these HLAs were deleted, and the cell surface expression of HLA actually disappeared (see Figure 49). By contrast, the cell surface expression of HLA was confirmed in cells with HLA-A and B integrated (see Figure 49). The cell surface expression of HLA was also confirmed in cells with HLA-C and E integrated (see Figure 49). According to the method of the present disclosure, a desired gene can be thus integrated into each monoallele. For example, as in this Example, the expression level and functionality of the introduced HLA can be evaluated by introducing the native HLA, including the control sequences and introns, to the cells lacking HLA. Thus, the method of the present disclosure is considered useful for expression analysis and functional evaluation of each of HLA-similar sequences and polymorphisms.

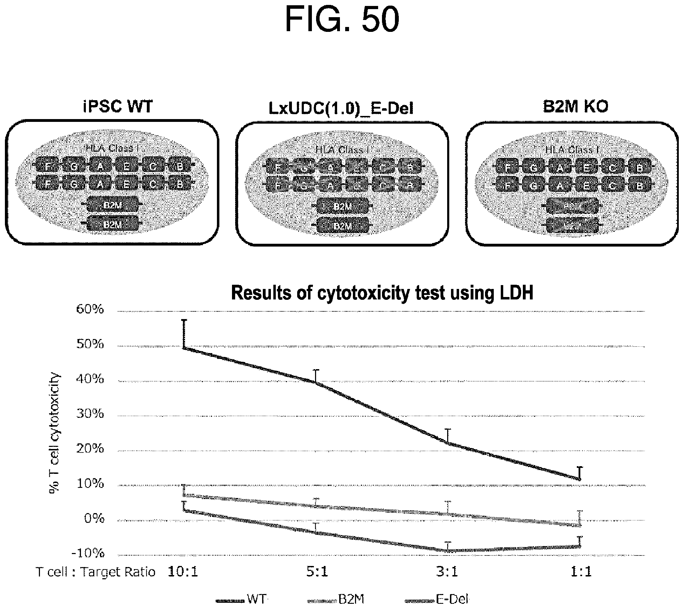

It was confirmed that the UDCs evaded cytotoxicity by T cells. iPS cells (wild type), LxUDC (1.0)_E-Del, or β2-microglobulin (B2M) knockout cells and T cells were mixed at a ratio of 1:1, 1:3, 1:5, or 1:10 to confirm T cell cytotoxicity. As a result, LxUDC (1.0) E-Del evaded cytotoxicity more strongly than the B2M knockout, as shown in Figure 50.

### Contents of Sequence Listing

Sequence Number (ID): 1 cttcatctcc gtcggctacg 20
Sequence Number (ID): 2 atgatggaaa actcgatggg 20
Sequence Number (ID): 3 tggaatccgg tataggcgcc 20
Sequence Number (ID): 4 tgagtgaccc gataaaactc 20
Sequence Number (ID): 5 tcttaaaaac ttctatatcg 20
Sequence Number (ID): 6 gtgcaactcc cgaataggct 20
Sequence Number (ID): 7 tgggccttat catccgtcaa 20
Sequence Number (ID): 8 tgggtctgtc gagcacaagg 20
Sequence Number (ID): 9 ggtcaaatgc cttgtactcg 20
Sequence Number (ID): 10 gggagggccg gtaccgttga 20
Sequence Number (ID): 11 atagctaacg tcactattgc 20
Sequence Number (ID): 12 cgttctcagc gcgggcacta 20
Sequence Number (ID): 13 gccgctgccg ctctacgctt 20
Sequence Number (ID): 14 cagaaagggt aagcgcacag 20
Sequence Number (ID): 15 ggcgcaacgc gatcgcgtaa 20
Sequence Number (ID): 16 gacgccagtg cattcgacga 20
Sequence Number (ID): 17 ggcgcaacgc gatcgcgtaa 20
Sequence Number (ID): 18 cctcaccttc ccctcttttc 20
Sequence Number (ID): 19 accttcccct gtgacttgtg 20
Sequence Number (ID): 20 ctcaccttcc cttcctttcc 20
Sequence Number (ID): 21 accttcccct gtgatttgtg 20
Sequence Number (ID): 22 ccaccaccct gtctttgact 20
Sequence Number (ID): 23 tgtcacccct tccttacctg 20
Sequence Number (ID): 24 gaggcagaaa ggcacacata 20
Sequence Number (ID): 25 agcctctcct tcccattctt 20
Sequence Number (ID): 26 tccatcaacc tctcatagca aa 22
Sequence Number (ID): 27 acttctggaa attccttttg g 21
Sequence Number (ID): 28 gatggcccat gtgtggat 18
Sequence Number (ID): 29 ttatcccagg tgcctgtgtc 20
Sequence Number (ID): 30 ttcccactta cagccagacc 20
Sequence Number (ID): 31 tacgggaggt agccagtgtt 20
Sequence Number (ID): 32 tgggaagtgg tcaggagata a 21
Sequence Number (ID): 33 ggccttccat ctgttgct 18
Sequence Number (ID): 34 gacatcggca aggtgtgg 18
Sequence Number (ID): 35 gtggtggagt gaacggaga 19
Sequence Number (ID): 36 tgggaagtgg tcaggagata a 21
Sequence Number (ID): 37 tgtaatcttc tctgtcgcta cttc 24
Sequence Number (ID): 38 tgcaccagat tgttttgtgt 20
Sequence Number (ID): 39 gtggtggagt gaacggaga 19
Sequence Number (ID): 40 ccaccttagc ctcctcctct 20
Sequence Number (ID): 41 aaagcatatt tgggcactct g 21
Sequence Number (ID): 42 agacaagttc accccaccag 20
Sequence Number (ID): 43 tcacctccat gtgccttaca 20
Sequence Number (ID): 44 acagagagaa gggcacagga 20
Sequence Number (ID): 45 catcagcaga agggaggaag 20
Sequence Number (ID): 46 aacatgcaaa ggggattctg 20
Sequence Number (ID): 47 tggaagtggc ttttggtttc 20
Sequence Number (ID): 48 tggatttcat gcctcacaaa 20
Sequence Number (ID): 49 ttcctcttgt ctccccagaa 20
Sequence Number (ID): 50 caccatcacc cttcttccac 20
Sequence Number (ID): 51 tgcttcctct tcccagctaa 20
Sequence Number (ID): 52 caccatcacc cttcttccac 20
Sequence Number (ID): 53 tgcttcctct tcccagctaa 20
Sequence Number (ID): 54 gcagagagtg ggaccaagag 20
Sequence Number (ID): 55 tgctaattct ggggagatgg 20
Sequence Number (ID): 56 tccatcaggg tcttctggtc 20
Sequence Number (ID): 57 tttttaaccg gctctggatg 20
Sequence Number (ID): 58 gagagccctt agctggtgtg 20
Sequence Number (ID): 59 gtgcagtttg tccacccttt 20
Sequence Number (ID): 60 tgggaagtgg tcaggagata a 21
Sequence Number (ID): 61 gtctgggtaa gggccagggc ccccgagagt agcaggagga 40
Sequence Number (ID): 62 acgtagggtc cttcatcctg 20
Sequence Number (ID): 63 gtggtggagt gaacggaga 19
Sequence Number (ID): 64 tgggaagtgg tcaggagata a 21
Sequence Number (ID): 65 agacaactgc caaggacacc 20
Sequence Number (ID): 66 gtggtggagt gaacggaga 19
Sequence Number (ID): 67 ccctgtgtga gtccagaaca 20
Sequence Number (ID): 68 tgggaagtgg tcaggagata a 21
Sequence Number (ID): 69 ccctgtgtga gtccagaaca 20
Sequence Number (ID): 70 gtggtggagt gaacggaga 19
Sequence Number (ID): 71 ccctgtgtga gtccagaaca 20
Sequence Number (ID): 72 
MAVMAPRTLL LLLSGALALT QTWAGSHSLK YFHTSVSRPG RGEPRFISVG
YVDDTQFVRF 60
WMHGCELGPD RRFLRGYEQF AYDGKDYLTL NEDLRSWTAV DTAAQISEQK
SNDASEAEHQ 180
WQQDGEGHTQ DTELVETRPA GDGTFQKWAA VVVPSGEEQR YTCHVQHEGL
PEPVTLRWKP 300
L 361
Sequence Number (ID): 73 AVMAPRTLLL LLSG 14

## Claims

1. A cell comprising genomic DNA with a deletion, wherein the deletion is of a region comprising a portion or the whole of an HLA-similar sequence-cluster region located in each of one or more chromosomal regions selected from the group consisting of chromosomal regions defined in the following (i) to (iv):
(i) a series of regions comprising HLA-F, HLA-G, and HLA-A,
(ii) a series of regions comprising HLA-C and HLA-B,
(iii) a series of regions comprising HLA-DRA, HLA-DRB5, and HLA-DRB1, and
(iv) a series of regions comprising HLA-DOA, HLA-DPA1, and HLA-DPB1,
wherein
the HLA-similar sequence-cluster region comprises all of HLA-encoding genes and HLA-similar sequences contained in each of the chromosomal regions, and
the portion comprises at least a stretch of region, the stretch of region comprising 50% or more of genes selected from the group consisting of the HLA-encoding genes and HLA-similar sequences contained in each of the chromosomal regions.

2. The cell according to claim 1, wherein any or preferably all of (i) to (iv) with the deletion defined in claim 1 in the genomic DNA are free of or only one among endogenous HLA and HLA-similar sequences.

3. The cell according to claim 1 or 2, wherein any or preferably all of (i) to (iv) with the deletion defined in claim 1 in the genomic DNA are free of endogenous HLA and HLA-similar sequences.

4. The cell according to any of claims 1 to 3, wherein the cell comprises
genomic DNA lacking the whole of the HLA-similar sequence-cluster region in (i) a series of regions comprising HLA-F, HLA-G, and HLA-A.

5. The cell according to any of claims 1 to 4, wherein the cell comprises
genomic DNA lacking the whole of the HLA-similar sequence-cluster region in (ii) a series of regions comprising HLA-C and HLA-B.

6. The cell according to any of claims 1 to 5, wherein the cell comprises
genomic DNA lacking the whole of the HLA-similar sequence-cluster region in (iii) a series of regions comprising HLA-DRA, HLA-DRB5, and HLA-DRB1 or a chromosomal region corresponding to chr6:32,445,000-32,821,000.

7. The cell according to any of claims 1 to 6, wherein the cell comprises
genomic DNA lacking the whole of the HLA-similar sequence-cluster region in (iv) a series of regions comprising HLA-DOA, HLA-DPA1, and HLA-DPB1.

8. The cell according to any of claims 1 to 7, wherein the deletion is of a region having the other end in a specific sequence (second sequence) in a chromosomal region corresponding to chr6:29,701,000-29,723,464 of the hg38 genomic sequence.

9. The cell according to any of claims 1 to 8, wherein the genomic DNA comprises an insertion of an endogenous or exogenous desired gene operably linked to a control sequence, the insertion being located in any of the regions (i) to (iv) or in a region other than the regions (i) to (iv).

10. The cell according to any of claims 1 to 9, wherein the deletion is of a region having one end in a specific sequence (third sequence) in a chromosomal region corresponding to chr6:31,166,000-31,269,169 of the hg38 genomic sequence and the other end in a specific sequence (fourth sequence) in a chromosomal region corresponding to chr6:31,357,158-31,544,000 of the hg38 genomic sequence.

11. The cell according to any of claims 1 to 10, wherein the deletion is of a region having one end in a specific sequence (fifth sequence) in a chromosomal region corresponding to chr6:2,416,000-33,445,000 of the hg38 genomic sequence and the other end in a specific sequence (sixth sequence) in a chromosomal region corresponding to chr6:32,439,951-32,831,000 of the hg38 genomic sequence.

12. The cell according to any of claims 1 to 11, wherein the deletion is of a region having one end in a specific sequence (seventh sequence) in a chromosomal region corresponding to chr6:32,924,000-33,006,838 of the hg38 genomic sequence and the other end in a specific sequence (eighth sequence) in a chromosomal region corresponding to chr6:33,086,238-33,165,000 of the hg38 genomic sequence.

13. The cell according to any of claims 1 to 12, wherein the regions (i) to (iv) with a deletion has no repetitive sequence within the chromosomal region.

14. The cell according to any of claims 1 to 13, wherein the cell comprises one or both of functional β2 microglobulin and functional CIITA.

15. The cell according to any of claims 1 to 14, wherein the cell does not substantially express HLA class I and/or HLA class II on a cell surface.

16. The cell according to any of claims 1 to 15, wherein the deletion is between 100 kb and 400 kb in size.

17. A composition comprising the cell according to any of claims 1 to 16.

18. A cell having a genome with a deletion of a region in all alleles of the genome, wherein the deletion comprises a region comprising a portion, or preferably the whole, of an MHC-similar sequence-cluster region of a locus encoding an MHC molecule.

19. The cell according to claim 18, wherein each MHC-similar sequence-cluster region comprises only a gene encoding no more than one MHC molecule.

20. The cell according to claim 18, wherein each MHC-similar sequence-cluster region comprises only a gene encoding no more than four MHC molecules.

21. The cell according to claim 18, wherein each MHC-similar sequence-cluster region does not comprise a gene encoding an MHC molecule.

22. The cell according to claim 18, wherein the deletion comprises a region comprising all of the MHC-similar sequence-cluster region of a locus encoding an MHC molecule.

23. The cell according to claim 22, further comprising a control sequence and a desired gene operably linked to the control sequence.

24. The cell according to claim 23, wherein the control sequence and the desired gene operably linked to the control sequence have a non-naturally occurring sequence as a whole.

25. The cell according to claim 23, wherein the control sequence and the desired gene operably linked to the control sequence have a naturally occurring sequence.
